(19) Europäisches Patentamt
European Patent Office
Office européen des brevets

(11) **EP 4 146 300 B1**

(12) **EUROPÄISCHE PATENTSCHRIFT**

(45) Veröffentlichungstag und Bekanntmachung des Hinweises auf die Patenterteilung:
**09.08.2023  Patentblatt 2023/32**

(21) Anmeldenummer: **22740329.2**

(22) Anmeldetag: **24.06.2022**

(51) Internationale Patentklassifikation (IPC):
**A61M 1/36** (2006.01)        **A61M 1/16** (2006.01)

(52) Gemeinsame Patentklassifikation (CPC):
**A61M 1/1609; A61M 1/1617; A61M 1/165; A61M 1/3658**

(86) Internationale Anmeldenummer:
**PCT/EP2022/067353**

(87) Internationale Veröffentlichungsnummer:
**WO 2022/269038 (29.12.2022 Gazette 2022/52)**

(54) **REZIRKULATIONSMESSUNG MITTELS ZWEI INTERIMSSCHALTUNGEN MIT KINETISCH UNTERSCHIEDLICHEN DIFFUSIONSZUSTÄNDEN**

MEASUREMENT OF RECIRCULATION BY MEANS OF TWO INTERIM CIRCUITS HAVING KINETICALLY DIFFERENT DIFFUSION STATES

MESURE DE RECIRCULATION AU MOYEN DE DEUX CIRCUITS INTÉRIMAIRES AYANT DES ÉTATS DE DIFFUSION DIFFÉRENTS DU POINT DE VUE CINÉTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.06.2021   DE 102021116343**

(43) Veröffentlichungstag der Anmeldung:
**15.03.2023  Patentblatt 2023/11**

(73) Patentinhaber: **B. Braun Avitum AG**
**34212 Melsungen (DE)**

(72) Erfinder: **JANIK, Waldemar**
**34212 Melsungen (DE)**

(74) Vertreter: **Winter, Brandl - Partnerschaft mbB**
**Alois-Steinecker-Straße 22**
**85354 Freising (DE)**

(56) Entgegenhaltungen:
**EP-A1- 3 431 118     WO-A1-2020/212332**

## Beschreibung

Die vorliegende Offenbarung betrifft eine extrakorporale Blutbehandlungsmaschine, insbesondere Dialysemaschine, als eine Vorrichtung zur Rezirkulationsmessung bei einer extrakorporalen Blutbehandlung, beispielsweise Hämodialyse, Hämofiltration und /oder Hämodiafiltration, sowie eine zur Nachrüstung an eine extrakorporale Blutbehandlungsmaschine ausgebildete Modulvorrichtung zur Rezirkulationsmessung. Ferner wird ein zugehöriges Verfahren zur Rezirkulationsmessung vorgeschlagen.

## Technischer Hintergrund

Bei einer extrakorporalen Blutbehandlung, beispielsweise einer Blutreinigung in Form einer Hämodialyse, Hämofiltration oder Hämodiafiltration, wird einem Dialysepatienten Blut über einen arteriellen Gefäßzugang entnommen, in einem Dialysator behandelt und dem Patienten anschließend über einen venösen Gefäßzugang zurückgegeben. Das Blut des Patienten wird also durch einen extrakorporalen Kreislauf geleitet. Bei chronisch kranken Patienten werden die extrakorporalen Blutbehandlungen so häufig vorgenommen, dass die Vene, über die das Blut nach der Behandlung zurückgegeben wird, auf Dauer sich entzünden und verkleben würde. Aus diesem Grund wird solchen Patienten operativ ein sogenannter Shunt als Zugang zum Blutgefäßsystem angelegt, welcher eine Querverbindung zwischen der Arterie und der Vene des Patienten darstellt und als dauerhafte Punktionsstelle verwendet wird. In Folge des Shunts verdickt sich die Gefäßwand der Vene, so dass diese sich leichter punktieren lässt und damit einen einfacheren Zugang für die Dialyse ermöglicht. In den meisten Fällen wird ein solcher Shunt in den Arm eines Patienten integriert.

Über die (arteriovenöse) Querverbindung zwischen Arterie und Vene kommt es jedoch auch zu einem Blutaustausch von venösem und arteriellem Blut, insbesondere wenn während der Blutbehandlung der Blutfluss an der Blutpumpe zu hoch eingestellt ist und infolgedessen das in den Shunt des Patienten zurückzufördernde Blut teilweise in den arteriellen Gefäßzugang des Patienten übertritt. Folglich verdünnt also das bereits gereinigte, venöse Blut das noch ungereinigte, arterielle Blut, so dass es zu einer Beeinträchtigung der Blutbehandlungseffizienz bzw. des Wirkungsgrads der Blutbehandlung kommt. Dem liegt unter Gesichtspunkten eines (transmembranen) Stofftransports bzw. der Diffusion zwischen einerseits einer Blut(-Membran)seite und andererseits einer Dialysat(-Membran)seite (bzw. Dialysierflüssigkeitsseite) (einer Dialysemembran) eines Dialysators ein nachteilig verringerter Konzentrationsgradient bzw. ein nachteilig verringertes treibendes Potential zugrunde. Dadurch wird die Behandlungszeit nachteilig verlängert.

Ein solcher Vorgang, bei dem das bereits gereinigte sowie in den Patienten zurückgeförderte Blut aus der Patientenvene über den Gefäßzugang, bspw. Shunt, in die Arterie einströmt und von dort erneut in den extrakorporalen Blutkreislauf gelangt, wird als Rezirkulation bzw. Rezirkulationsanteil, Rezirkulationswert (in %) bezeichnet und kann anhand unterschiedlicher Methoden qualitativ sowie quantitativ bestimmt werden. Sprich, die Rezirkulation bezeichnet das (Volumenstrom- bzw. Flussraten-) Verhältnis des im Shunt aus dem venösen Zugang in den arteriellen Zugang (quasi wiederholt) zurückgesaugten, schon gereinigten Blutes, bezogen auf den aktuellen (gesamten) Blutfluss. Dabei ist der (gesamte) Blutfluss (-Volumenstrom- bzw. die Blutflussrate) im extrakorporalen Blutschlauchsystem bekannt, da an der Maschine (als Förderate der Blutpumpe) einstellbar. Eine quantitative Rezirkulationsmessung wird unter anderem zur Überwachung des Shuntzustands und der Überprüfung der Blutbehandlungseinstellungen, beispielsweise der Förderrate der Blutpumpe, eingesetzt.

## Stand der Technik

Aus dem Stand der Technik sind mehrere unterschiedliche Methoden zur Bestimmung des Rezirkulationsanteils bekannt.

Eine Vielzahl häufig angewandter Verfahren zur Rezirkulationsbestimmung beruht auf dem Prinzip einer blutseitigen Bolusgabe. Beispielsweise kennt der Stand der Technik nach diesem Prinzip die Erzeugung eines definierten Temperaturbolus, welcher im venösen Blutzweig aufgegeben wird, mit anschließender Temperaturmessung im arteriellen Zweig, so dass basierend auf der aufgegebenen Temperaturdifferenz und der gemessenen Temperaturdifferenz mathematisch ein Rückschluss auf die Rezirkulationsquote gebildet werden kann. Ebenso ist es bekannt, anstelle der Temperatur einen anderen Indikator, beispielsweise eine bestimmte Stoffkonzentration oder die Leitfähigkeit, zu messen, wobei der Indikator zuvor in Form eines (Indikator-) Bolus aufgegeben wurde. Auch kann der Bolus dialysierflüssigkeitsseitig stromauf des Dialysators aufgegeben werden und dialysierflüssigkeitsseitig stromab des Dialysators eine entsprechende Messung durchgeführt werden, aus der die Rezirkulation bestimmbar ist.

Aus der Patentschrift DE 197 02 441 C1 ist beispielsweise eine Vorrichtung und ein Verfahren zur Bestimmung der Rezirkulation bei einer extrakorporalen Blutbehandlung unter Einsatz eines Shunts bekannt, wobei eine Indikatorgröße, beispielsweise ein Konzentrationsbolus, im Dialysierflüssigkeitskreislauf stromauf des Dialysators erzeugt und die Konzentration eine definierte Zeitspanne später im Dialysierflüssigkeitskreislauf stromab des Dialysators beobachtet wird, um einen Rückschluss auf die Rezirkulation zu ziehen.

Auch in EP 2 783 715 A1 ist ein Verfahren zur Rezirkulationsmessung offenbart, bei dem durch blutseitige Boluszugabe und dialysierflüssigkeitsseitigem spektralphotometrischen Messen eine Rezirkulation bestimmbar ist.

Als weiteres Beispiel beschreibt die Patentanmeldung US 5,588,959 A eine Vorrichtung und ein Verfahren für eine Rezirkulationsmessung mittels Temperatur. Dabei wird das Blut am venösen Schlauchabschnitt gekühlt und die Temperatur des Bluts im arteriellen Schlauchabschnitt gemessen.

Weiter offenbart die Patentanmeldung WO 96/08305 A1 ein Verfahren zur Rezirkulationsbestimmung bei extrakorporalen Blutbehandlungen, indem ein Indikator im venösen Blutzweig zugegeben wird und mittels eines Detektors am arteriellen Blutzweig ein dem Indikator zugeordneter Messwert erfasst und anhand der Verdünnungskurve eine Rezirkulationsrate berechnet wird.

Alternativ zu dem vorstehend zitierten Stand der Technik gemäß dem Prinzip der blutseitigen Bolusgabe offenbart die DE 10 2013 103 221 A1 eine Rezirkulationsmessung gemäß einer Transientenmethode nach Änderung des extrakorporalen Blutflusses. In die mathematische Bestimmung der Rezirkulation gemäß der Transientenmethode nach Änderung des extrakorporalen Blutflusses fließt ein Dämpfungsparameter hinsichtlich des Einschwingvorganges mit und ohne Rezirkulation ein, wozu mithilfe eines optischen Sensors am Dialysatausgang die variable Konzentration eines Stoffes am Dialysatausgang $c_{DO}$ erfasst wird. Dabei wird der Rezirkulationsbestimmung ein Berechnungsmodell mit der folgenden Gleichung bzw. einer zugehörigen Umformung (unter Heranziehen einer bekannten Gleichung aus den Grundlagen von Michaels [vgl. einführender Teil der die DE 10 2013 103 221 A1 zitierenden WO 2020/212332 A1]) zugrundegelegt:

$$R = \frac{1 - \dfrac{c_{DO} \cdot Q_D}{c_{sys} \cdot K_D}}{1 - \dfrac{c_{DO} \cdot Q_D}{c_{sys} \cdot K_D} + \dfrac{c_{DO} \cdot Q_D}{c_{sys} \cdot Q_B}}$$

Dabei gilt Folgendes:

| R | Rezirkulationswert bzw. Rezirkulationsrate (0... 1 bzw. 0...100%) | |
|---|---|---|
| $C_{DO}$ | Konzentration eines Stoffes am Dialysatausgang | Messwert (aktuell) bzw. messbar |
| $c_{sys}$ | systemische Blutanteilsstoff-Konzentration, welche nicht rezirkulationsbehaftet ist. (Konzentration im Patienten) | Messwert/Peakwert |
| $Q_D$ | Dialysierflüssigkeitsflussrate | Bekannt bzw. einstellbar |
| $Q_B$ | Blutflussrate im extrakorporalen Blutschlauchsystem | Bekannt bzw. einstellbar (vgl. DE 10 2013 103 221 A1, Abschnitt [0123]) |
| $K_D$ | theoretische Clearance des Dialysators | Funktion von ($K_{0A}$, $Q_B$, QD) → also bekannt |
| $K_{0A}$ | Dialysator-spezifischer Clearance-Koeffizient | bekannt (Lookup-Tabelle), d.h. spezifischer Clearance-Koeffizient, der abhängig ist vom aktuell eingesetzten Dialysator und verwendeter Dialysierflüssigkeit und der vorab (empirisch) ermittelbar ist |

Nachteilig hierbei ist, dass zur Bestimmung von $c_{sys}$ vor der Blutbehandlung zunächst eine Blutprobe des Patienten genommen und $c_{sys}$ (im analytischen Labor) gemessen werden muss. Anschließend kann die Rezirkulation R während der Blutbehandlung anhand der vorstehenden Gleichung ausgerechnet werden.

Der vorgenannte Stand der Technik hat jedoch immer den Nachteil, dass entweder ein erhöhter apparativer Aufwand, beispielsweise durch Vorsehen von Temperiereinrichtungen oder Einrichtungen für eine Stoff- bzw. Konzentrations-Boluszugabe, erforderlich ist oder vor der Behandlung eine separate Blutwertbestimmung durchgeführt werden muss. Des Weiteren ist die Bestimmung von $c_{sys}$ anhand einer Patientenblutprobe aufwändig und daher nicht zufriedenstellend.

Bereits die WO 2020/212 332 A1 der vorliegenden Anmelderin, die hiermit durch Verweis ausdrücklich zum Bestandteil der vorliegenden Offenlegungsschrift gemacht wird, überwindet die vorstehend genannten Nachteile und offenbart eine gattungsgemäße extrakorporale Blutbehandlungsmaschine. Insofern lehrt die Offenbarung der WO 2020/212 332 A1 eine Vorrichtung zur extrakorporalen Blutbehandlung, die dazu eingerichtet ist, die systemische Blutanteilstoff-Konzentration $c_{sys}$ (Konzentration eines Blutanteilsstoffs im Blut des Patientenkörpers) während der Blutbehandlung und insbesondere ausschließlich mit den an einer Vorrichtung zur extrakorporalen Blutbehandlung grundsätzlich vorgesehenen Einrichtungen zu bestimmen und die Rezirkulation R dann anhand der vorstehenden, bekannten Gleichung zu ermitteln.

Die als nächstliegender Stand der Technik ansehbare EP 3431118 A1 offenbart eine Vorrichtung und ein Verfahren zur extrakorporalen Blutbehandlung, insbesondere zur Hämodialyse, wobei Blut eines Patienten von einer Dialysierflüssigkeit in einem Dialysator umspült wird und wobei eine mit der Pfasma-Natrium-Konzentration des Blutes korrelierten Größe gemessen wird. Die Zusammensetzung der Dialysierflüssigkeit wird dann in Abhängigkeit der gemessenen Größe dergestalt eingestellt, dass die Plasma-Natrium-Konzentration des Blutes zumindest am Ende der Blutbehandlung den gleichen Wert aufweist, wie zu Beginn. Zur Messung der mit der Plasma-Natrium-Konzentration des Blutes korrelierten Größe kann bspw. ein Bypass-Betrieb eingerichtet werden, bei dem die Dialysierflüssigkeit am Dialysator vorbeigeleitet wird, sodass ein Restvolumen auf der Seite der verbrauchten Dialysierflüssigkeit die Konzentration der blutseitig gelösten Subtanzen zumindest teilweise annimmt.

## Kurze Beschreibung der Offenbarung

Somit ist es eine Aufgabe der Offenbarung, eine technische Lösung zur Bereitstellung eines zu der Offenbarung der EP 3431118 A1 alternativen Verfahrens, um eine Rezirkulation zu erfassen, zu finden.

Es ist ferner eine weitere Aufgabe der Offenbarung, eine zu der Offenbarung der WO 2020/212 332 A1 alternative technische Lösung zu finden, um die Nachteile aus dem Stand der Technik gemäß dem Prinzip der blutseitigen Bolusgabe oder gemäß der Transientenmethode zu überwinden oder zumindest zu mildern und insbesondere eine Vorrichtung zur Rezirkulationsmessung bei einer extrakorporalen Blutbehandlung, beispielsweise unter Verwendung eines Shunts, bereitzustellen, die ohne zusätzlichen apparativen Aufwand, beispielsweise einer Temperiereinrichtung, und/oder verfahrenstechnischen bzw. klinischen Aufwand, wie beispielweise einer separaten Blutprobenentnahme vor der Behandlung, durchführbar ist.

Gegenüber der Offenbarung der WO 2020/212 332 A1 besteht die zusätzliche Aufgabe, eine besonders kostengünstige und einfach durchzuführende Rezirkulationsmessung bereitzustellen, welche nicht von der systemischen Blutanteilstoff-Konzentration $c_{sys}$ und den weiteren in die Bestimmung eingehenden Maschinen- bzw. Therapieparametern abhängig ist.

Die Aufgabe der Offenbarung wird mit einer extrakorporalen Blutbehandlungsmaschine mit den Merkmalen des Anspruchs 1 gelöst. Eine erfindungsgemäße Modulvorrichtung weist die technischen Merkmale nach Anspruch 14 auf. Eine erfindungsgemäße Speichereinheit weist die technischen Merkmale nach Anspruch 15 auf.

Gemäß der Offenbarung hat eine extrakorporale Blutbehandlungsmaschine, insbesondere eine Dialysemaschine, einen Dialysator sowie zumindest eine Sensorvorrichtung, die auf einer Dialysierflüssigkeitsseite (bzw. in dem Dialysierflüssigkeitskreis) dem Dialysator nachgeschaltet und mit einer Steuer- und Recheneinheit elektrisch verbunden ist. Der Begriff des Dialysators betrifft eine Vorrichtung zur Blutreinigung, wie Hämodialyse, Hämofiltration und/oder Hämodiafiltration, insbesondere ein Dialysemodul, weiter bevorzugt ein Hohlfasermodul. Dabei ist offenbarungsgemäß die Steuer- und Recheneinheit zur Bestimmung eines Vorhandenseins einer Rezirkulation ohne blutseitige Bolusgabe dafür vorgesehen und ausgebildet (bzw. eingerichtet), das von der zumindest einen Sensorvorrichtung zur quantitativen Bestimmung zumindest eines vorzugsweise ausgewählten oder auswählbaren Blutanteilsstoffs in der verbrauchten Dialysierflüssigkeit gemessene zumindest eine Signal, hierin bezeichnet als $S_{DO}$, einer physikalischen und/oder chemischen Größe der verbrauchten Dialysierflüssigkeit in seinem zeitlichen Verlauf [d.h. als zeitlich veränderliches (Mess-)Signal bzw. als Messkurve, insbesondere als funktionale Abhängigkeit $S_{DO} = f(t)$ zu erfassen. Dabei kann die zumindest eine Sensorvorrichtung die Messkurve bzw. den zeitlichen Verlauf mittels kontinuierlicher und/oder diskontinuierlicher, bspw. getakteter, Messung erfassen.

Ferner ist dabei die Steuer- und Recheneinheit offenbarungsgemäß eingerichtet, die extrakorporale Blutbehandlungsmaschine in einem Grund-Modus zum kontinuierlichen Betrieb, in welchem die Dialysierflüssigkeitsströmung durch den Dialysator zugelassen ist, zu betreiben; sowie das der in dem Grund-Modus verbrauchten Dialysierflüssigkeit zugehörige Signal als ein Grund-Signal (bzw. als eine Basislinie, ein Bezugswertsignal, ein Normierungswert) zu messen.

Dabei bezeichnet der Begriff des Grund-Modus den kontinuierlichen Betrieb, in welchem die (kontinuierliche) Dialysierflüssigkeitsströmung durch den Dialysator zugelassen ist. Dabei kann insbesondere die Dialysierflüssigkeitsrate im Wesentlichen konstant sein, so dass die Durchströmung in dem Dialysator einem stationären Zustand entspricht. Insbesondere ist mit dem Begriff des Grund-Modus der für die Kernfunktion der Dialyse bzw. der extrakorporalen Blutbehandlung vorgesehene (normale) Dialyse-Betriebsmodus gemeint. Das Grund-Signal wird hierin als $S_{DO, \, pre}$ bezeichnet, wobei das Suffix "pre" gewählt ist, um einen einer Betriebsmodusänderung vorangehenden Zustand lediglich nominal und nicht-limitierend zu bezeichnen. Aufgrund der Eigenschaft eines Grund-Signals in dem Grund-Modus als dem (normalen) Dialyse-Betriebsmodus versteht der Fachmann, dass das Grund-Signal in einem nachfolgenden Zustand bzw. zu einem späteren Zeitpunkt (Suffix "post") gleichermaßen auftritt bzw. erfasst werden kann.

Ferner ist dabei die Steuer- und Recheneinheit offenbarungsgemäß eingerichtet, die extrakorporale Blutbehandlungsmaschine von dem Grund-Modus in einen Interims-Modus zum Interims-Betrieb, in welchem eine Dialysierflüssigkeitsmenge auf der Dialysierflüssigkeits-Membranseite im Dialysator eingesperrt wird, während der Blutstrom im extrakorporalen Blutkreis mit einer festgelegten Blutflussrate betrieben wird, wobei das Blut auf der Blut-Membranseite (8B) weiter strömt, für die jeweilige Dauer eines vorbestimmten und/oder vorbestimmbaren Interims-Zeitintervalls zu schalten. Insbesondere kann der Interims-Modus einen Bypass-Modus (bzw. eine Betriebsart in einer Bypass- bzw. Nebenschluss-Schaltung) betreffen.

Nachfolgend zu dem vorhergehenden vorstehenden Schaltungsschritt in den Interims-Modus, insb. Bypass-Modus, ist die Steuer- und Recheneinheit offenbarungsgemäß eingerichtet, in den Grund-Modus zu wechseln, um die zuvor eingesperrte Dialysierflüssigkeitsmenge als einen jeweiligen Dialysierflüssigkeitsbolus der zumindest einen Sensorvorrichtung zuzuführen. Dies dient offenbarungsgemäß dazu, um eine dem Dialysierflüssigkeitsbolus zugehörige Signaländerung (bzw. Signalabweichung) gegenüber dem (bzw. bezogen auf das, korrigiert um das) Grund-Signal als ein zugehöriges Bolus-Signal zu messen. Sprich, der Dialysierflüssigkeitsbolus wird durch das Zurückschalten in den Grund-Modus mit der wieder einsetzenden Dialysierflüssigkeitsrate an der zumindest einen Sensorvorrichtung vorbeigepumpt und passiert diese.

Je nach, weiter unten im Detail offenbarten, bevorzugten alternativen und/oder kumulativen Ausführungsformen kann dabei das zugehörige Bolus-Signal: i) als ein (maximales oder minimales oder betragsmäßiges) Peaksignal bzw. ein Extremum erfasst werden, hierin bezeichnet als $S_{DO,\,ext}$; und/oder ii) als eine integrale Gesamtfläche bw. ein gesamtes Flächenintegral erfasst werden, hierin bezeichnet als A; und/oder iii) als eine integrale Teilfläche bw. ein teilweises Flächenintegral erfasst werden, hierin bezeichnet als $A_{part}$.

Ferner ist dabei die Steuer- und Recheneinheit offenbarungsgemäß eingerichtet, den Interims-Modus für ein erstes Interims-Zeitintervall zur Gleichgewichtseinstellung des Stofftransports zu schalten. Dazu wird eine erste Dialysierflüssigkeitsmenge im Dialysator zumindest solange eingesperrt, nämlich für die Dauer des ersten Interims-Zeitintervalls, bis sich auf der Dialysierflüssigkeits-Membranseite und der Blut-Membranseite des Dialysators ein sich nicht mehr oder nur noch unwesentlich veränderndes Konzentrationsgleichgewicht des Blutanteilsstoffs einstellt.

Ferner ist dabei die Steuer- und Recheneinheit offenbarungsgemäß zur Ausbildung eines Messzyklus dafür vorgesehen und ausgebildet, den Interims-Modus für ein, gegenüber dem ersten Interims-Zeitintervall kürzeres, zweites Interims-Zeitintervall zu schalten. Dazu wird eine zweite Dialysierflüssigkeitsmenge im Dialysator nur solange eingesperrt, nämlich für die Dauer des zweiten Interims-Zeitintervalls, dass sich das Konzentrationsgleichgewicht des Blutanteilsstoffs noch nicht einstellt (bzw. eingestellt hat).

Ferner ist dabei die Steuer- und Recheneinheit offenbarungsgemäß eingerichtet, aus einer Abweichung eines zweiten Bolus-Signals in Folge des zweiten Interims-Zeitintervalls gegenüber einem ersten Bolus-Signal in Folge des ersten Interims-Zeitintervalls das Vorhandensein der Rezirkulation zu ermitteln.

Zusammenfassend stellt es einen wesentlichen Gedanken der vorliegenden Offenbarung dar, hintereinander (in beliebiger Reihenfolge) zumindest zwei (separate, unabhängige) Interims-Modi bzw.- Schaltungen, insbesondere Bypass-Modi (auch als Nebenschluss-Schaltungen bezeichnet), unterschiedlicher Dauer bzw. für unterschiedlich lange Interims-Zeitintervalle durchzuführen. Dabei markieren die unterschiedlich langen Interims-Zeitintervalle qualitativ signifikant unterschiedliche kinetische Zustände, nämlich zum einen einen Gleichgewichtszustand, zum anderen einen Ungleichgewichtszustand (bzw. einen Zustand vorab dem Erreichen des Gleichgewichtszustandes). Die beiden jeweiligen Signalausschläge, die am Dialysatausgang nach den zwei Interims-Modi bzw.- Schaltungen, insbesondere Bypass-Modi, bestimmt werden können, werden (zueinander) ins Verhältnis gesetzt. Aus diesem Verhältnis wird offenbarungsgemäß die Rezirkulation qualitativ (d.h. als solche) bestimmt bzw. das (insb. signifikante bzw. relevante) Vorhandensein der Rezirkulation erkannt. In, weiter unten im Detail offenbarten, bevorzugten alternativen und/oder kumulativen Ausführungsformen kann die Rezirkulation darüberhinausgehend quantitativ (d.h. als Rezirkulationswert, insbesondere als Rezirkulationsanteil in %) bestimmt bzw. errechnet werden.

Gemäß der vorliegenden Offenbarung ergeben sich mehrere Vorteile bzw. Verbesserungen gegenüber dem Stand der Technik. Insofern wird eine besonders einfache und schnelle, dennoch gleichzeitig sensitive Rezirkulationsmessung vorgeschlagen. Als automatische Rezirkulationsmessung erfolgt diese unscheinbar und für den Patienten nicht störend im Hintergrund. So erfordert die vorliegende technische Lösung zur Erkennung bzw. Bestimmung einer ggf. vorliegenden Rezirkulation im einfachsten Fall keinerlei Kenntnis weiterer Einflußgrößen bzw. Einstellparameter. Insbesondere ist keine Änderung und/oder Justage, Kalibrierung usw. sonstiger Einstellparameter eigens zum Zwecke der Rezirkulationsbestimmung erforderlich, wie z.B. der Dialysierflüssigkeits- und/oder der Blutflussrate. Aufgrunddessen eignet sich die vorliegende Offenbarung nicht nur besonders gut zur Entlastung des Klinikpersonals, sondern gerade auch für Dialyseanwendungen durch den Patienten alleine zuhause. Insbesondere bietet die Offenbarung eine technische Lösung, für welche keine Infusionslösungen notwendig sind.

Aufgrund des Umstandes, dass die Signale direkt in die Auswertung durch die Steuer- und Recheneinheit eingehen, handelt es sich um eine besonders robuste, nicht störungsanfällige Detektions- bzw. Messmethode. Nicht zu vernachlässigen ist ferner der Kostenvorteil hinsichtlich Bauteilen und Wartung. Insofern kann die Offenbarung mit in Dialysemaschinen etablierter Sensorik durchgeführt werden, so dass kein zusätzliches Equipment benötigt wird.

Zu diesem Zweck bedient sich die Offenbarung zumindest einer insbesondere die Leitfähigkeit messenden, kumulativ oder alternativ weiter bevorzugt einer optisch arbeitenden, Sensorvorrichtung am oder stromab zu einem dialysierflüssigkeitsseitigen Dialysatorausgang, welche im Allgemeinen zur Erfassung/Bestimmung von bestimmten Blutstoffanteilen wie urämischen Toxinen (z.B. Harnstoff) in der verbrauchten Dialysierflüssigkeit verwendet werden sowie einer vorzugsweise Dialysemaschineeigenen elektronischen Steuerung bzw. Steuer- und Recheneinheit.

Dabei ist die elektronische Steuerung offenbarungsgemäß dafür vorgesehen und angepasst, die Dialysemaschine zur Ausführung eines Messzyklus zumindest zweimal jeweils in einen Interims-Modus, insbesondere in einen Bypass-Modus bzw. in einen Nebenschluss-(Schaltungs-)Modus, zu schalten, in welchem Dialysierflüssigkeit im Dialysator einerseits für ein erstes Interims-Zeitintervall und andererseits für ein zweites Interims-Zeitintervall eingesperrt wird.

Hinsichtlich des Interims-Modus ist es unerheblich, in welcher Reihenfolge in das erste Interims-Zeitintervall und in das zweite Interims-Zeitintervall aus dem davor, dazwischen und/oder danach herrschenden Grund-Modus (temporär) umgeschaltet wird. Sprich, es spielt offenbarungsgemäß keine Rolle, ob - jeweilig ausgehend vom Grund-Modus und nach dem Zeitablauf zurückgehend in den Grund-Modus - zuerst in das erste Interims-Zeitintervall und später in das zweite Interims-Zeitintervall geschaltet wird; oder zuerst in das zweite Interims-Zeitintervall und später in das erste Interims-Zeitintervall. Insofern laufen die grenzflächenphysikalischen Vorgänge des Stofftransportes bzw. der (transmembranen) Diffusion des zumindest einen aus dem Blut in den jeweiligen Dialysierflüssigkeitsbolus (bzw. in das temporär stationär verweilende, d.h. eingesperrte Delta-Volumen der Dialysierflüssigkeits-Membranseite) übergehenden Blutanteilsstoffes sowie die Kinetik der grenzflächenphysikalischen Vorgänge unabhängig vom Weg ab. Sprich, die grenzflächenphysikalischen Vorgänge während des ersten Interims-Zeitintervalls hinsichtlich des ersten Dialysierflüssigkeitsbolus üben keinerlei Einfluss auf die Vorgänge während des zweiten Interims-Zeitintervalls hinsichtlich des zweiten Dialysierflüssigkeitsbolus aus; und umgekehrt.

Lediglich repräsentiert der erste Dialysierflüssigkeitsbolus, entsprechend dem (langen) ersten Interims-Zeitintervall, einen erreichten Gleichgewichtszustand. Dazu wird das (lange) erste Interims-Zeitintervall vorab so bestimmt bzw. gewählt, dass der erste Dialysierflüssigkeitsbolus (mindestens) solange eingesperrt wird, bis sich auf der Dialysierflüssigkeits-Membranseite und der Blut-Membranseite des Dialysators ein sich nicht mehr (oder nur unwesentlich) veränderndes Blutanteilsstoff-Konzentrationsgleichgewicht einstellt/eingestellt hat. D.h., während der Einsperrphase nimmt die Reinigung des Blutes im Dialysator ab und geht gegen Null, weshalb nach einer ausreichend langen Verweildauer schließlich ein Diffusionsgleichgewicht zwischen den Blut- und Dialysierflüssigkeits-Membranseiten herrscht. Diese im Dialysator temporär eingesperrte (Bilanz-) Menge bzw. ein (Bilanz-) Volumen an verbrauchter Dialysierflüssigkeit, als Dialysierflüssigkeitsbolus bezeichnet, hat demnach eine dem Patientenblut im Wesentlichen entsprechende Blutanteilsstoff-Konzentration.

In dem Zusammenhang des ersten Interims-Zeitintervalls sei angemerkt, dass die Kinetik des Stofftransportes bzw. der Diffusion und damit ein jeweiliger Zeitpunkt, zu welchem ein jeweiliges Diffusionsgleichgewicht erreicht ist, neben dem konkreten Blutanteilsstoff, beispielsweise einem bestimmten urämischen Toxin, ggf. auch vom Wirkungsgrad der Blutbehandlung abhängt, also von der tatsächlich vorliegenden Rezirkulation. Ist die Rezirkulation nur gering ausgeprägt oder gar nicht vorhanden, wird das Diffusionsgleichgewicht ggf. schneller erreicht, als wenn die Rezirkulation hoch ausfällt, da rezirkuliertes Blut zu einer Verdünnung des zu reinigenden, arteriellen Bluts führt und somit die Effizienz der Blutreinigung beeinträchtigt. Die Dauer bis zum Erreichen des Diffusionsgleichgewichts zwischen der Blut- und der Dialysierflüssigkeits-Membranseite ist maßgeblich aber abhängig von der eingestellten Blutflussrate und der Dialysatorgröße. Je größer die Blutflussrate und je kleiner die Dialysatorgröße, desto schneller wird ein diffusives Gleichgewicht erreicht.

Um nun sicherzustellen, dass die Dauer des Interims-Modus, insbesondere des Bypass-Modus, auch tatsächlich mindestens der Dauer, bis zu der sich das Diffusionsgleichgewicht eines bestimmten Blutanteilsstoffs im Dialysator eingestellt hat, entspricht, muss das erste Interims-Zeitintervall stets auf die Maximaldauer der Diffusionsgleichgewichtseinstellung ausgelegt werden. Diese wird der Steuer- und Recheneinheit vorab zur Verfügung gestellt, entweder durch Abruf der Information aus einer Datenbank / einem Datensatz oder durch Eingabe eines Benutzers, und kann abhängig vom Patienten, dessen Shuntzustand und/oder weiteren spezifischen Erfahrungswerten sein, wie beispielsweise anhand einer empirischen Ermittlung im Labor oder Auswertung von vergangenen Blutbehandlungen bestimmbar.

Demgegenüber, bzw. relativ zum ersten Interims-Zeitintervall, wird das (kurze) zweite Interims-Zeitintervall vorab so bestimmt bzw. (um einen absoluten und/oder anteiligen Verkürzungswert) verkürzt gewählt, dass sich der zweite Dialysierflüssigkeitsbolus (maximal) solange eingesperrt wird, dass sich auf der Dialysierflüssigkeits-Membranseite und der Blut-Membranseite des Dialysators gerade nicht bzw. keinesfalls ein sich nicht mehr (oder nur unwesentlich) veränderndes Blutanteilsstoff-Konzentrationsgleichgewicht einstellt/eingestellt hat. Sprich, der zweite Dialysierflüssigkeitsbolus repräsentiert, entsprechend dem (kurzen) zweiten Interims-Zeitintervall, einen kinetischen Zustand mit (ausreichendem) Abstand vorab einem erreichten Gleichgewichtszustand, d.h. einen Ungleichgewichtszustand. Mit anderen Worten wird eine noch weiter ablaufende Kinetik der grenzflächenphysikalischen Vorgänge, welche durch das Vorhandensein treibender Gradienten bzw. Konzentrationspotentiale bestimmt ist, zum Zeitpunkt des Endes des zweiten Interims-Zeitintervalls abgebrochen bzw. der herrschende Zustand für die nachfolgende Messung durch die zumindest eine Sensorvorrichtung stromab sozusagen eingefroren'.

Die für das erste (bzw. in Relation dazu: zweite) Interims-Zeitintervall zu erwartende Dauer ist weiter davon abhängig, was für ein Dialysator verwendet wird, beispielsweise vom Zustand (neu oder wiederverwendet) und/oder der (verfügbaren) Filterfläche und/oder des (dialysatseitigen) Füllvolumens, und mit welcher Blutflussrate das zu reinigende Blut durch den Dialysator geführt wird. Deshalb steht der Steuer- und Recheneinheit ein Datensatz zur Verfügung, aus dem sich die Soll-Dauer(n) für das erste (bzw. zweite) Interims-Zeitintervall und die einzustellende Soll-Blutflussrate unter Berücksichtigung der maximal möglichen Rezirkulation und der Eigenschaften des konkret verwendeten / angeschlossenen Dialysators ermitteln lassen. Es ist alternativ auch denkbar, die Soll-Dauer(n) für das erste (bzw. zweite) Interims-Zeitintervall auch in Abhängigkeit einer vorbestimmten Blutflussrate zu ermitteln.

Mit dem Umschalten aus dem Interims-Modus, insb. Bypass-Modus, (erneut zurück) in den Grund-Modus tritt wieder die Durchströmung des Dialysators ein, so dass die zuvor temporär eingesperrte (Bilanz-) Menge dann freigegeben ist, um wie ein Dialysierflüssigkeitsbolus der zumindest einen Sensorvorrichtung stromab dem Dialysierflüssigeitsablauf zugeführt zu werden. Sodann misst bzw. erfasst die zumindest eine Sensorvorrichtung eine mit dessen Blutanteilsstoff-Konzentration korrelierende chemische und/oder physikalische Größe als ein (dem jeweiligen Dialysierflüssigkeitsbolus zugehöriges) Bolus-Signal. Offenbarungsgemäß wird das Bolus-Signal als eine Änderung (bzw. eine Abweichung, ein Ausschlag) im Signal (bzw. Mess-, Sensorsignal) der zumindest einen Sensorvorrichtung (unmittelbar) nach der Freigabe des Dialysierflüssigkeitsbolus aus dem Dialysator bemerkbar bzw. erfasst. Dabei ist es unerheblich, ob das Bolus-Signal in positiver oder negativer Ordinatenachse ausschlägt.

An dieser Stelle sei darauf hingewiesen, dass unter dem Begriff "im Dialysator eingesperrt" konkret ein Einsperren der Dialysierflüssigkeit auf einer Dialysierflüssigkeits-Membranseite des Dialysators zu verstehen ist. Der Begriff "einsperren" ist darüber hinaus als nicht bzw. im Wesentlichen nicht durchströmend zu verstehen. Weiter umfasst der eingeschlossene Raum u.U. auch noch Teile der Dialysierflüssigkeitszu-/ablaufleitung. Wenn man beispielsweise davon ausgehen würde, dass z.B. durch Aktivierung eines Dialysator-Bypasses mit einem deutlich geringeren Strömungswiderstand (im Vergleich mit dem Dialysator) ein Strömen der Dialysierflüssigkeit durch den Dialysator unterbrochen bzw. im Wesentlichen unterbrochen wird, könnte auf die dicht abschließenden (Sperr-)Ventile für eine solche Bypass-Schaltung verzichtet werden. In diesem Fall wäre die Flüssigkeit nicht wirklich 'eingesperrt' sondern die Durchströmung des Dialysators mit Dialysierflüssigkeit wäre auf nahe Null reduziert.

Mit anderen Worten wird in dem Bypass-Modus als dem bevorzugten Interims-Modus die Dialysierflüssigkeits-Membranseite im Dialysator nicht mit frischer Dialysierflüssigkeit versorgt bzw. die Filtermembran dialysierflüssigkeitsseitig nicht mit frischer Dialysierflüssigkeit umspült. Vielmehr wird eine auf der Dialysierflüssigkeits-Membranseite vorhandene (erste bzw. zweite) Dialysierflüssigkeitsmenge dort gehalten, vorzugsweise unter Einsatz von (Sperr-) Ventilen, die in der Dialysierflüssigkeitszulaufleitung und der Dialysierflüssigkeitsablaufleitung angeordnet sind. Anstelle von (Sperr-)Ventilen sind selbstverständlich auch andere zur Fluidabsperrung geeignete Elemente, beispielsweise Pumpen, denkbar. Während nun die auf der Dialysierflüssigkeits-Membranseite befindliche Dialysierflüssigkeit dort gehalten wird, wird der Blutstrom im extrakorporalen Blutkreis mit einer festgelegten Blutflussrate betrieben. Die Blut-Membranseite des Dialysators wird also weiter mit arteriellem bzw. zu reinigendem Blut versorgt bzw. die Filtermembran blutseitig weiter mit arteriellem bzw. zu reinigendem Blut umspült. Folglich findet nun, wie bei Dialysebehandlungen üblich, aufgrund von Diffusion ein Übertritt von im Blut gelösten Stoffen (Blutanteilsstoffen) durch die Filtermembran in die Dialysierflüssigkeit statt, und zwar - bei fehlender Unterbrechung - solange, bis kein Konzentrationsgradient mehr zwischen dem auf der Blut-Membranseite vorhandenen Blut und der auf der Dialysierflüssigkeitsseite vorhandenen Dialysierflüssigkeit vorliegt, also in anderen Worten ein Diffusionsgleichgewicht erreicht ist. Anhand dieses kinetischen Zeitpunktes ist das erste Interims-Zeitintervall festlegbar. Da das Blut auf der Blut-Membranseite des Dialysators weiter strömt, während die Dialysierflüssigkeit auf der Dialysierflüssigkeits-Membranseite des Dialysators steht, reichern sich in der stehenden Dialysierflüssigkeit solange Blutanteilsstoffe weiter an, bis diese in der stehenden Dialysierflüssigkeit zumindest im Wesentlichen dieselbe Konzentration erreicht haben wie im weiter durch den Dialysator strömenden Blut. In anderen Worten entspricht zu dem Zeitpunkt, an dem das Diffusionsgleichgewicht eines Blutanteilsstoffs zwischen der stehenden Dialysierflüssigkeit und dem strömenden Blut (im Wesentlichen) erreicht ist, die Konzentration des in der stehenden Dialysierflüssigkeit gelösten Blutanteilsstoffs zumindest im Wesentlichen der Konzentration des im Blut des Patientenkörpers gelösten Blutanteilsstoffs. An dieser Stelle sei darauf hingewiesen, dass die die grenzflächenphysikalischen Vorgänge bestimmende Größe "Konzentration" auch durch einen mit diesem zusammenhängenden anderen Parameter repräsentiert sein kann, wie beispielsweise eine von einer Sensorvorrichtung messbare elektrische Leitfähigkeit und/oder eine Absorbanz.

Darüber hinaus ist es, hinsichtlich des Begriffs des Interims-Modus, weiter bzw. alternativ möglich, statt in den Bypass-Modus in die sogenannte sequenzielle Ultrafiltration zu schalten. In dieser ist ein Sperrventil stromab dem Dialysator weiterhin geöffnet, so dass eine konvektive Reinigung des Blutes mithilfe einer Ultrafiltrationsvorrichtung bzw. dem Dialysator während dieser Zeitdauer nach wie vor gegeben ist. Die Ultrafiltrationsrate kann hierfür im Vergleich zur Ultrafiltrationsrate im Grund-Modus bzw. Hauptschluss erhöht, herabgesetzt oder konstant gehalten werden. Sprich, der Begriff des Interims-Modus soll in Art eines Oberbegriffes den Begriff des Bypass-Modus bzw. Schaltungen in Form des Bypasses gleichermaßen wie den Begriff der sequenziellen Phase bei der Durchführung der sequenziellen Ultrafiltration umfassen. Da ansonsten die Offenbarung identisch ist, werden zur Vermeidung von Wiederholungen die bevorzugten Ausführungsformen im Folgenden nur anhand des Falls der Bypass-Schaltungen erläutert. Dabei sollen alle alternativen und/oder kumulativen technischen Merkmale gleichermaßen auch im Zusammenhang mit der sequenziellen Ultrafiltration offenbart sein.

Liegt eine Rezirkulation vor, so ist das in den Dialysator eintretende Blut während des ("kurzen") zweiten Interims-Zeitintervalls, d.h. während dieser Zeit vorab der Gleichgewichtseinstellung, von dem zurückfließenden venösen Blut beeinflusst und somit rezirkulationsbehaftet. Demzufolge ist die während des ("kurzen") zweiten Interims-Zeitintervalls eingeschlossene zweite Dialysierflüssigkeitsmenge der Dialysierflüssigkeits-Membranseite, bei (bzw. im Falle des) Vorhandenseins einer Rezirkulation, rezirkulationsbehaftet.

Dahingegen während des ("langen") ersten Interims-Zeitintervalls passt sich, wie vorstehend geschildert, die eingeschlossene erste Dialysierflüssigkeitsmenge der Dialysierflüssigkeits-Membranseite vollständig an das Blut an, welches ununterbrochen durch den Dialysator gefördert wird. Dadurch, dass das arterielle Blut in seiner Zusammensetzung nicht mehr von der Dialysierflüssigkeit beeinflusst wird, führt das zurückfließende venöse Blut selbst bei Vorhandensein einer Rezirkulation (sprich, in dem Fall einer vorliegenden Rezirkulation, trotz dieser) zu keiner weiteren Veränderung des arteriellen Blutes.

Aufgrunddessen ist nach einer ausreichend langen Zeit im Bypass-Modus bzw. spätestens mit Ende des ersten Interims-Zeitintervalls das Blut in der arteriellen Leitung nicht mehr rezirkulationsbehaftet.

Demzufolge ist die während des ("langen") ersten Interims-Zeitintervalls eingeschlossene bzw. eingesperrte erste Dialysierflüssigkeitsmenge der Dialysierflüssigkeits-Membranseite, selbst bei (bzw. im Falle des) Vorhandenseins einer Rezirkulation, nicht rezirkulationsbehaftet. Sprich, es spielt keine (bzw. eine vernachlässigbare) Rolle, ob eine Rezirkulation vorliegt oder eben nicht. Rezirkulation spielt hierbei nur insoweit eine Rolle, als dass sich die Zeitspanne bis zur Erreichung eines Diffusionsgleichgewichts verändert (verlängert).

Vorzugsweise, alternativ oder kumulativ, kann jeweils das erste Bolus-Signal und das zweite Bolus-Signal jeweils einem zugehörigen (bei positivem Signum: maximalen; bzw. bei negativem Signum: minimalen; bzw. betragsmäßigen) Peak (bzw. Extremum) im Signal der zumindest einen (insb. ersten und/oder zweiten) Sensorvorrichtung entsprechen. Dabei wird der Peak bzw. das Extremum von dieser unmittelbar nach der Freigabe des jeweiligen ersten bzw. zweiten Dialysierflüssigkeitsbolus aus dem Dialysator erfasst. Der (betragsmäßig) maximale Peak des ersten Bolus-Signals wird als $S_{DO,\,ext,\,L}$ bezeichnet; der (betragsmäßig) maximale Peak des zweiten Bolus-Signals wird als $S_{DO,\,ext,\,K}$ bezeichnet.

Dabei ist die Steuer- und Recheneinheit ferner dafür vorgesehen und ausgebildet, jeweilig eine zweite Differenz, bezeichnet als $D_{K,}$ aus dem maximalen Peak des zweiten Bolus-Signals $S_{DO,\,ext,\,K}$ und dem Grund-Signal $S_{DO,\,pre}$ sowie eine erste Differenz, bezeichnet als $D_{L}$ aus dem maximalen Peak des ersten Bolus-Signals $S_{DO,\,ext,\,L}$ und dem Grund- Signal $S_{DO,\,pre}$ zu berechnen. Sprich, die erste und die zweite Differenz bilden einen Delta-Wert bzw. einen um das Grund-Signal bereinigten bzw. verminderten Signalwert ab. Damit bilden die erste und die zweite Differenz die eigentliche Respons bzw. Signalantwort der zumindest einen Sensoreinheit auf die Passage des ersten bzw. zweiten Dialysierflüssigkeitsbolus ab.

Die jeweiligen Differenzen zwischen dem jeweiligen Peak bzw. Extremum und dem zugehörigen Wert vor dem Interims-Modus, insb. Bypass-Modus, beschreiben die folgenden zwei Gleichungen:

$$D_L = S_{DO,ext,L} - S_{DO,pre,L}$$

$$D_K = S_{DO,ext,K} - S_{DO,pre,K}$$

Der Wert $S_{DO,pre,L}$ entspricht dem eingependelten Wert, d.h. dem Grund-Signal, kurz vor Beginn oder Ende des ersten Interims-Zeitintervalls, insbesondere vor Beginn oder Ende der länger andauernden Bypass-Schaltung. Er kann auch dem eingependelten Wert kurz vor Beginn oder Ende des zweiten Interims-Zeitintervalls, insbesondere vor Beginn oder Ende der kürzer andauernden Bypass-Schaltung, bezeichnet als $S_{DO,pre,K}$, entsprechen oder umgekehrt.

Dabei ist die Steuer- und Recheneinheit ferner dafür vorgesehen und ausgebildet, einen Delta-Quotienten (als Verhältniswert) aus der zweiten Differenz $D_K$ bezogen auf die erste Differenz $D_L$ zu berechnen bzw. ermitteln. Ferner ist dabei die Steuer- und Recheneinheit dafür vorgesehen und ausgebildet, auf Grundlage des ermittelten Delta-Quotienten, bezeichnet als $V_D$, sowie eines vorbekannten Berechnungsmodells einen aktuellen Rezirkulationswert R (in %) zu bestimmen.

Das Verhältnis der zweiten Differenz zu der ersten Differenz bezeichnet der Delta-Quotient $V_D$:

$$V_D = \frac{D_K}{D_L}$$

Somit kann der aktuelle Rezirkulationswert R als Funktion dieses Verhältnisses bzw. des Delta-Quotienten $V_D$ bestimmt werden:

$$R = f(V_D)$$

Unter dem Begriff des Berechnungsmodells versteht der Fachmann insb. die Vielzahl der ihm bekannten numerisch-mathematischen Methoden, um einen mathematischen bzw. funktionalen Zusammenhang bzw. eine Korrelation zwischen Parametern abzubilden. Der Zusammenhang kann vorzugsweise als eine vorab bestimmte bzw. abgespeicherte Lookup-Tabelle, charakteristische Kennlinie, ein Kennfeld und/oder dergleichen vorliegen. Bspw. kann der Zusammenhang im Vorfeld mittels multipler Regression und/oder mittels eines künstlichen neuronalen Netzes und/oder eines sonstigen maschinellen Lernverfahrens ermittelt worden sein. Insbesondere kann es sich um ein, vorzugsweise multiparametriges bzw. multidimensionales, Kennfeld handeln, welches in Form von gefitteten Kennlinien bzw. Modell-Funktionsgleichungen abgebildet bzw. aufgeschlüsselt (auf einer Speichereinheit) vorliegt. Bspw. sind im Ingenieurwesen Fitting- und/oder Regressionsmethoden vielfach vorbeschrieben, um ein bspw. experimentell ermitteltes (graphisches) Kennfeld in eine formelhafte (numerische) Schreibweise, bspw. als Polynom, umzuformen. Dabei kann ein jeweils einzelner Parameter des Berechnungsmodells einer echten bzw. realen physikalischen und/oder chemischen Größe, bspw. einer Konzentration, oder einer (Natur-)konstanten oder einem Beiwert des Dialysators bzw. der Dialysemaschine, entsprechen; und/oder es kann ein jeweils einzelner Parameter des Berechnungsmodells gerade auch nicht einem im naturwissenschaftlichen Sinne realen Parameter entsprechen, sondern einen sogenannten Fitting-Parameter und/oder Compound-Parameter darstellen. Weiter ist vorbeschrieben, dass ein vorbestimmtes Kennfeld lediglich in einem Anwendungsintervall gültig ist.

Alternativ zur Auswertung der Signaldifferenzen können auch jeweilige Gesamtflächen unterhalb der beiden Signalausschläge $A_K$ und $A_L$ und/oder jeweilige Teilflächen unterhalb der beiden Signalausschläge $A_{part,\,K}$ und $A_{part,\,L}$ ins Verhältnis gebracht werden:

$$V_A = \frac{A_K}{A_L}$$

Gesamtflächenintegral-Quotient
Bzw.

$$V_{A,\,part} = \frac{A_{part,\,K}}{A_{part,\,L}}$$

Teilflächenintegral-Quotient
Auf diese Weise kann die Rezirkulation auch als Funktion der Flächenverhältnisse in Weise des Gesamtflächenintegral-Quotienten bzw. Teilflächenintegral-Quotienten bestimmt werden:

$$R = f(V_A)$$

$$R = f(V_{A,part})$$

Vorzugsweise, alternativ oder kumulativ, kann also das erste Bolus-Signal einem (ersten) gesamten Flächenintegral, bezeichnet als $A_L$, im Signal der zumindest einen Sensorvorrichtung unmittelbar nach der Freigabe des ersten Dialysierflüssigkeitsbolus aus dem Dialysator und das zweite Bolus-Signal einem (zweiten) gesamten Flächenintegral, bezeichnet als $A_K$, im Signal der zumindest einen Sensorvorrichtung unmittelbar nach der Freigabe des zweiten Dialysierflüssigkeitsbolus aus dem Dialysator entsprechen. Dabei ist das erste gesamte Flächenintegral $A_L$ und das zweite gesamte Flächenintegral $A_K$ jeweils um das Grund-Signal $S_{DO,\,pre}$ bereinigt. Wie oben bereits dargelegt, kann das Grund-Signal $S_{DO,\,pre}$ insbesondere, aber nicht limitierend, ein vorangehendes betreffen (wie durch das Suffix in der Bezeichnung angedeutet). Insofern kann das Grund-Signal gleichermaßen als ein nachfolgendes Grund-Signal vorgesehen bzw. erfasst sein. Auch ist es für alle bereinigten Bolus-Signale bzw. Quotienten denkbar, dasselbe Grund-Signal sowohl für das erste als auch für das zweite Bolus-Signal heranzuziehen.

Dabei ist die Steuer- und Recheneinheit ferner dafür vorgesehen und ausgebildet, einen Gesamtflächenintegral-Quotienten (als Verhältniswert) aus dem zweiten gesamten Flächenintegral $A_K$ bezogen auf das erste gesamte Flächenintegral $A_L$ zu berechnen bzw. ermitteln. Ferner ist dabei die Steuer- und Recheneinheit dafür vorgesehen und ausgebildet, auf Grundlage des ermittelten Gesamtflächenintegral-Quotienten, bezeichnet als $V_A$, sowie eines vorbekannten Berechnungsmodells einen aktuellen Rezirkulationswert R (in %) zu bestimmen.

Vorzugsweise, alternativ oder kumulativ, kann also das erste Bolus-Signal einem (ersten) teilweisen Flächenintegral, bezeichnet als $A_{part,\,L}$, im Signal der zumindest einen Sensorvorrichtung unmittelbar nach der Freigabe des ersten Dialysierflüssigkeitsbolus aus dem Dialysator und das zweite Bolus-Signal einem (zweiten) teilweisen Flächenintegral, bezeichnet als $A_{part,\,K}$, im Signal der zumindest einen Sensorvorrichtung unmittelbar nach der Freigabe des zweiten Dialysierflüssigkeitsbolus aus dem Dialysator entsprechen. Dabei ist das erste teilweise Flächenintegral $A_{part,\,L}$ und das zweite teilweise Flächenintegral $A_{part,\,K}$ jeweils um das Grund-Signal $S_{DO,\,pre}$ bereinigt. Wie oben bereits dargelegt, kann dies insbesondere, aber nicht limitierend, ein vorangehendes Grund-Signal $S_{DO,\,pre}$ betreffen.

Dabei ist die Steuer- und Recheneinheit ferner dafür vorgesehen und ausgebildet, einen Teilflächenintegral-Quotienten (als Verhältniswert) aus dem zweiten teilweisen Flächenintegral $A_{part,\,K}$ bezogen auf das erste teilweise Flächenintegral $A_{part,\,L}$ zu berechnen bzw. ermitteln. Ferner ist dabei die Steuer- und Recheneinheit dafür vorgesehen und ausgebildet, auf Grundlage des ermittelten Teilflächenintegral-Quotienten, bezeichnet als $V_{A,\,part}$, sowie eines vorbekannten Berechnungsmodells einen aktuellen Rezirkulationswert R (in %) zu bestimmen.

Insbesondere kann dabei das erste teilweise Flächenintegral $A_{part,\,L}$ bzw. das zweite teilweise Flächenintegral $A_{part,\,K}$ einen (um einen jeweiligen Anteilswert) anteiligen Ausschnitt bzw. eine anteilige Fläche des ersten gesamten Flächenintegrals $A_L$ bzw. des zweiten gesamten Flächenintegrals $A_K$ darstellen. Insb. kann es vorgesehen sein, dass der jeweilige Anteilswert für das erste teilweise Flächenintegral $A_{part,\,L}$ und das zweite teilweise Flächenintegral $A_{part,\,K}$ identisch gewählt ist. Bspw. kann der jeweilige Anteilswert kleinergleich 90%, weiter bevorzugt kleinergleich 80% und insb. bevorzugt kleinergleich 70% betragen. Insb. kann es bevorzugt sein, eine anteilige Fläche des ersten gesamten Flächenintegrals $A_L$ bzw. des zweiten gesamten Flächenintegrals $A_K$ auf eine anfängliche Teilfläche und/oder eine um den zugehörigen Peak zentrierte Teilfläche zu richten bzw. als eine solche vorzusehen, bspw. eine sich ausziehende Schleppe des ersten bzw. zweiten gesamten Flächenintegrals abzuschneiden bzw. für die Auswertung zu ignorieren. Dies bewirkt den Vorteil einer schnelleren und/oder präziseren Messung.

Vorzugsweise, alternativ oder kumulativ, kann das Berechnungsmodell zur Berechnung des aktuellen Rezirkulationswerts eine Dialysierflüssigkeitsflussrate des Dialysierflüssigkeitskreises, bezeichnet als $Q_D$, und/oder eine Blutflussrate, bezeichnet als $Q_B$, im extrakorporalen Blutschlauchsystem des Blutkreises und/oder eine theoretische Clearance des Dialysators berücksichtigen. Insbesondere kann das Berechnungsmodell weiter noch zumindest einen (weiteren Parameter) aus einer Anzahl weiterer Maschinen-, Dialysator- und/oder Einstellungsparameter (insb. betreffs der Blutbehandlungsmaschine) als Einflußgröße berücksichtigen. Insbesondere kann der zumindest eine weitere Parameter die Blutbehandlungsmaschine betreffen. Diese Parameter können bevorzugt der extrakorporalen Blutbehandlungsmaschine systembekannt sein und somit von der Steuer- und Recheneinheit herangezogen werden. Fallweise kann es bevorzugt sein oder (auch gerade) nicht bevorzugt sein, dass das Berechnungsmodell ausgeführt ist, den Einfluss des Dialysatortyps als vernachlässigbar zu betrachten bzw. (mathematisch) zu vernachlässigen. Dies kann eine weitere Vereinfachung der offenbarungsgemäßen Bestimmung in einem solchen Fall unterstützen, in welchem mittels Messungen verifiziert worden ist, dass eine Berücksichtigung des Dialysators zu keiner Verbesserung der Genauigkeit der Bestimmung des Vorhandenseins der Rezirkulation bzw. des aktuellen Rezirkulationswertes führt. Insgesamt trägt die optionale Berücksichtigung zumindest eines weiteren Parameters vorteilhaft zu einer weiteren Erhöhung der Genauigkeit und/oder der Reproduzierbarkeit der Bestimmung des Vorhandenseins der Rezirkulation bzw. des aktuellen Rezirkulationswertes bei.

So hat sich gezeigt, dass die Genauigkeit der Rezirkulationsbestimmung erhöht wird, wenn zusätzlich zu den vorstehend offenbarten Quotienten, d.h. dem Delta-Quotienten $V_D$, dem Gesamtflächenintegral-Quotienten $V_A$ und/oder dem Teilflächenintegral-Quotienten $V_{A,\,part}$ zumindest ein oder eine Vielzahl weiterer Parameter hinzugezogen wird und/oder sogar mehrere der Quotienten miteinander kombiniert werden.

Die Funktion zur Bestimmung der Rezirkulation kann also verallgemeinert folgendermaßen beschrieben werden:

$$R = f\left(V_D, V_A, V_{A,part}, S_{DO,ext,K}, S_{DO,ext,L}, S_{DO,pre,K}, S_{DO,pre,L}, Q_b, Q_d\right)$$

Dieser Zusammenhang kann als abgespeichertes Berechnungsmodell, insbesondere als eine Lookup-Tabelle oder als charakteristische(s) Kennlinie(nfeld), vorliegen.

Das Ergebnis der Rezirkulationsbestimmung kann vorzugsweise dabei dem Anwender, insb. dem medizinischen bzw. klinischen Personal und/oder dem Patienten, bspw. auf einer Anzeigevorrichtung der extrakorporalen Blutbehandlungsvorrichtung oder auf einer Anzeigevorrichtung, die mit der extrakorporalen Blutbehandlungsvorrichtung kabellos oder kabelgebunden in Kontakt steht, dargestellt werden. Die Rezirkulation kann sowohl qualitativ (Vorhandensein einer Rezirkulation als solche) als auch als quantitative Maßzahl bzw. als aktueller Rezirkulationswert (in %) ausgegeben werden. Eine qualitative Ausgabe bedeutet dabei insbesondere, dass angezeigt wird, ob eine medizinisch relevante Rezirkulation (bspw. Rezirkulationswert größergleich 15%) vorliegt oder nicht. Eine halbquantitative Ausgabe kann bspw. ein Ampelfarben-System betreffen.

Die jeweilige Dauer der Interims-Schaltungen, insb. die Dauer der Bypass-Schaltungen (bzw. der Nebenschluss-Schaltungen), insbesondere die optimale Dauer der längeren Bypass-Schaltung, d.h. das optimale erste Interims-Zeitintervall, ist abhängig von der eingestellten und/oder effektiven Blutflussrate durch den Dialysator sowie vom Dialysator(typ) selbst. So dauert es bis zur vollständigen Sättigung bzw. einer (asymptotisch einlaufenden) Gleichgewichtseinstellung in einem Fall mit einer geringen Blutflussrate und einem großen Dialysator länger als in einem anderen Fall mit einer höheren Blutflussrate und einem kleineren Dialysator. Zur Optimierung der Dauer der längeren Bypass-Schaltung, d.h. des ersten Interims-Zeitintervalls, können diese Informationen miteinbezogen werden.

Vorzugsweise, alternativ oder kumulativ, kann die Dauer des ersten Interims-Zeitintervalls aber auch so gewählt sein, dass alle als relevant vorab bestimmten bzw. vorbekannten Kombinationen von Blutflussraten und Dialysatorgrößen abgedeckt werden. So kann vorzugsweise das erste Interims-Zeitintervall zur Gleichgewichtseinstellung des Stofftransports zu einem pauschalen Wert gesetzt und/oder setzbar sein. Dabei kann insbesondere das erste Interims-Zeitintervall zu einer (pauschalen) Dauer zwischen 2 und 6 Minuten, weiter bevorzugt zwischen 4 und 5,5 Minuten, insb. von ca. 5 Minuten, gesetzt und/oder setzbar sein. Das dient dazu, eine mit Sicherheit zur Gleichgewichtseinstellung des Stofftransports ausreichende Dauer für das erste Interims-Zeitintervall pauschal vorzugeben, welche andererseits optimiert ist, den Messzyklus bzw. den Wechsel aus dem Grund-Modus nicht übermäßig zu verlängern. Ein pauschales, insbesondere erstes, Interims-Zeitintervall hat den Vorteil, dass für die extrakorporale Blutbehandlungsmaschine sowie das zugehörige Überwachungsverfahren nicht bekannt sein muss, welcher Dialysator verwendet wird.

Vorzugsweise, alternativ oder kumulativ, kann das zweite Interims-Zeitintervall vorab der Gleichgewichtseinstellung des Stofftransports zu einem pauschalen Wert gesetzt und/oder setzbar sein. Dabei kann das zweite Interims-Zeitintervall vorzugsweise kleinergleich 1 Minute, weiter bevorzugt 10 bis 20 Sekunden, insbesondere ca. 14 Sekunden betragen. Somit wird herstellerseitig eine besonders vorteilhafte, in experimentellen Versuchsserien bewährte Dauer für das zweite Interims-Zeitintervall empfohlen bzw. vorab vorgegeben. Dies bewirkt den Vorteil einer weitestgehenden Unabhängigkeit von anderen Einflußgrößen und somit einer sogenannten Robustheit gegen etwaige Störeinflüsse auf die Messgenauigkeit bzw. Signifikanz der Bestimmung des Vorhandenseins der Rezirkulation bzw. des aktuellen Rezirkulationswertes.

Vorzugsweise, alternativ oder kumulativ, kann der Verkürzungsfaktor, um den das zweite Interims-Zeitintervall bezogen (bzw. in Relation) auf das erste Interims-Zeitintervall verkürzt ist, größergleich 1:5, weiter bevorzugt größergleich 1:8 und insbesondere größergleich 1:20 betragen.

Vorzugsweise, alternativ oder kumulativ, kann die Steuer- und Recheneinheit weiter eingerichtet sein, den Messzyklus automatisch, insbesondere wiederholt und/oder nach einem vordefinierten Zeitregime, auszuführen. Vorzugsweise, alternativ oder kumulativ, kann die Steuer- und Recheneinheit weiter eingerichtet sein, aufgrund einer von einem Anwender über ein Eingabemittel ausgelösten Aufforderung den Messzyklus auszuführen. Dabei kann vorzugsweise das Eingabemittel kabellos oder kabelgebunden mit der Steuer- und Recheneinheit verbunden ausgeführt sein.

Vorzugsweise, alternativ oder kumulativ, kann die auf einer Dialysierflüssigkeitsseite dem Dialysator nachgeschaltete zumindest eine Sensorvorrichtung (die erste Sensorvorrichtung) und/oder gegebenenfalls eine weitere dem Dialysator nachgeschaltete Sensorvorrichtung eingerichtet sein, als das zumindest eine Signal insbesondere eine Leitfähigkeit der verbrauchten Dialysierflüssigkeit in seinem zeitlichen Verlauf zu erfassen, weiter bevorzugt als eine temperaturkompensierende Leitfähigkeitssonde ausgebildet zu sein. Vorzugsweise, alternativ oder kumulativ, kann die zumindest eine Sensorvorrichtung (die erste Sensorvorrichtung) und/oder gegebenenfalls eine weitere dem Dialysator nachgeschaltete Sensorvorrichtung eingerichtet sein, als das zumindest eine Signal eine Absorptionseigenschaft der verbrauchten Dialysierflüssigkeit in seinem zeitlichen Verlauf zu erfassen. Dabei betrifft die Absorptionseigenschaft (einer insbesondere, aber nicht limitierend, stromab des Dialysators angeordneten Sensorvorrichtung) insbesondere die Absorbanz und/oder den Transmissionsgrad. Vorzugsweise kann dabei die Absorptionseigenschaft bei einer Wellenlänge bzw. in einem Wellenlängenbereich zwischen 200 nm und 320 nm, weiter bevorzugt zwischen 250 nm und 310 nm und insbesondere bei 280 +/- 15 nm erfasst werden. Dazu kann die Sensorvorrichtung zum Beispiel Licht mit einer Wellenlänge im UV-Bereich emittieren. Vorzugsweise, alternativ oder kumulativ, kann die zumindest eine Sensorvorrichtung (die erste Sensorvorrichtung) und/oder gegebenenfalls eine weitere dem Dialysator nachgeschaltete Sensorvorrichtung in der Dialysierflüssigkeitsablaufleitung direkt an dem Dialysierflüssigkeitsablauf angeordnet sein, bspw.

direkt an diesen angeflanscht sein. Bei allen Ausführungsformen kann insbesondere (unabhängig) bevorzugt sein, dass sich die zweite Sensorvorrichtung, bzw. eine weitere Sensorvorrichtung stromab des Dialysators, auf eine andere physikalische und/oder chemische Größe richtet (bzw. zur Erfassung bzw. Messung einer solchen ausgebildet ist), als die zumindest eine Sensorvorrichtung (die erste Sensorvorrichtung) es ist. Die zumindest eine Sensorvorrichtung am oder stromab des Dialysierflüssigkeitsablaufs des Dialysators ist insbesondere dazu angepasst, durch die Filtermembran hindurchgetretene Blutanteilsstoffe, insbesondere urämische Toxine (bspw. Harnstoff, Kreatinin, Harnsäure, Kalium etc.), in der aus dem Dialysator ablaufenden, verbrauchten Dialysierflüssigkeit messtechnisch, insbesondere als Leitfähigkeitssonde, zu erfassen. Generell ist an dieser Stelle anzumerken, dass mit der Sensorvorrichtung eine physikalische und/oder chemische Größe gemessen wird, die zur Konzentration eines bestimmten Stoffes proportional sein kann. Tatsächliche bzw. unmittelbar gemessene Konzentrationswerte benötigt das vorgeschlagene (Überwachungs-)Verfahren zur Rezirkulationsbestimmung bzw. -Messung nicht.

Vorzugsweise, alternativ oder kumulativ, kann die Steuer- und Recheneinheit ferner dafür vorgesehen und ausgebildet sein, einen ersten aktuellen Rezirkulationswert auf Basis des ersten Signals der ersten Sensorvorrichtung und einen zweiten aktuellen Rezirkulationswert auf Basis des zweiten Signals der zweiten Sensorvorrichtung zu bestimmen. Dabei kann die Steuer- und Recheneinheit ferner dafür vorgesehen und ausgebildet sein, daraufhin einen Vergleich der ersten und zweiten aktuellen Rezirkulationswerte zu treffen, insbesonder eine gegebenenfalls vorliegende Abweichung (qualitativ / quantitativ) zu ermitteln bzw. zu bestimmen. Das bietet den Vorteil, dass unter Verwendung der zusätzlichen Information aus den zumindest zwei unterschiedlichen Sensorvorrichtungen bzw. Messsonden, zumal wenn auf Basis zweier unterschiedlicher Messprinzipien wie bspw. mittels Leitfähigkeit versus mittels einer optischen Sonde, für die Überwachung und den sicheren Betrieb nützliche Beurteilungen bzw. Entscheidungen erfolgen können.

Beispielsweise kann die Steuer- und Recheneinheit ferner dafür vorgesehen und ausgebildet sein, auf Grundlage des Vergleichs der ersten und zweiten aktuellen Rezirkulationswerte vorzugsweise einen mittleren aktuellen Rezirkulationswert (als Mittelwert) der ersten und zweiten aktuellen Rezirkulationswerte zu mitteln. Diese Mittelung kann vorteilhaft die Reproduzierbarkeit verbessern und der Unterdrückung von Messrauschen dienen.

Alternativ zu einer Mittelwertbildung, insbesondere im Falle einer auf Grundlage des Vergleichs der ersten und zweiten aktuellen Rezirkulationswerte bestimmbaren bzw. bestimmten (inbs. statistisch signifikanten) Abweichung, kann die Steuer- und Recheneinheit ferner dafür vorgesehen und ausgebildet sein, die (inbs. statistisch signifikante) Abweichung festzustellen. Weiter bevorzugt kann die Steuer- und Recheneinheit ferner dafür vorgesehen und ausgebildet sein, auf Grundlage der festgestellten (inbs. statistisch signifikanten) Abweichung der ersten und zweiten aktuellen Rezirkulationswerte voneinander einen (optionalen) weiteren Schritt auszuführen, insb. eine Steuerentscheidung automatisch zu treffen, vorzugsweise systemintern im Hintergrund oder unter Information an einen Anwender über ein Anzeigemittel, und/oder bei einem Anwender über eine interaktive Benutzerschnittstelle abzufragen.

Beispielsweise kann der weitere Schritt betreffen, das Ergebnis teilweise oder vollständig zu verwerfen (d.h. zumindest einen oder beide aus den ersten und zweiten aktuellen Rezirkulationswerten zu ignorieren). Insbesondere kann dies im Falle eines unzweifelhaften Kriteriums zum Verwerfen bzw. bei Vorliegen eines Artefaktes ausgeführt werden. Dabei kann insbesondere der Fall bzw. das Artefakt vorliegen, dass der erste und/oder zweite aktuelle Rezirkulationswert kleiner 0% oder größer 100% beträgt. Diese Werte ergeben physikalisch keinen Sinn, können als Artefakte aber aufgrund unzureichender Signalgüte in der betreffenden Sensorvorrichtung mathematisch möglich sein.

Beispielsweise, alternativ oder kumulativ, kann der weitere Schritt einen erneuten Messzyklus betreffen, wobei ein solcher automatisch initiierbar und/oder auf Abfrage über eine interaktive Benutzerschnittstelle an einen Anwender vorschlagbar bzw. auslösbar sein kann. Eine Abfrage über die Durchführung des erneuten Messzyklus bietet den Vorteil, dass der Anwender die Entscheidungshoheit darüber behält, ob er die Initiierung eines solchen zu dem fraglichen Zeitpunkt als notwendig bzw. nützlich erachtet.

Beispielsweise kann die (insb. statistisch signifikante) Abweichung pauschal größer 1 Sigma, weiter bevorzugt größer 2 Sigma und insbesondere größer 3 Sigma (Standardabweichung) betragen und/oder pauschal größer 10 Prozentpunkte (bezogen auf eine Differenz der beiden in % gemessenen ersten und zweiten Rezirkulationswerte), weiter bevorzugt größergleich 3 Prozentpunkte betragen.

In einer weiter alternativ oder kumulativ bevorzugten Ausführungsform kann die Steuer- und Recheneinheit ferner dafür vorgesehen und ausgebildet sein, auf Grundlage des Vergleichs der ersten und zweiten aktuellen Rezirkulationswerte eine Vorentscheidung hinsichtlich der Bedeutung bzw. Gewichtung der ermittelten Abweichung zu treffen und demzufolge den weiteren Schritt unterschiedlich auszuführen.

Dabei kann die Vorentscheidung auf Grundlage noch weiterer Daten, insbesondere von Zustandsdaten (bspw. Zeitlauf: Behandlungsbeginn versus Behandlungsende) zu der laufenden extrakorporalen Blutbehandlung, getroffen werden. Dies dient vorteilhaft der anwenderkonvenienten Berücksichtigung der messphysikalischen Rahmenbedingungen (bspw. zu Behandlungsbeginn bei hohen Konzentrationen der Protagonisten / Blutstoffe / Toxine ausreichende Signalgüte / optimales Messfenster; versus zum Behandlungsende nach bereits im Wesentlichen erfolgter Reinigung / Abtrennung der Toxine verminderte Signalgüte / ungünstiges Signal-/Rauschverhältnis).

Weiterhin kann die Steuer- und Recheneinheit ferner dafür vorgesehen und ausgebildet sein, die Vorentscheidung in Abhängigkeit von einem Relevanzbereich zu treffen, in welchen der entsprechende erste bzw. zweite aktuelle Rezirkulationswert (jeweilig) fällt bzw. fallen. Dabei kann der Relevanzbereich insbesondere zwischen 0% und 50%, weiter bevorzugt größergleich 15% Rezirkulationswert betreffen. Insbesondere kann die Steuer- und Recheneinheit ferner dafür vorgesehen und ausgebildet sein, bei Vorliegen des Relevanzbereiches die Messungen nicht zu verwerfen, weiter bevorzugt die im Relevanzbereich liegende Messung nicht zu verwerfen, sondern (als valide) heranzuziehen. Mit anderen Worten, kann die Steuer- und Recheneinheit selbst bei Feststellung einer Abweichung der beiden verglichenen Werte fallweise, d.h. in Abhängigkeit der Feststellung, ob jeweilig innerhalb oder außerhalb des Relevanzbereich liegend, entscheiden, ob der zumindest eine im Relevanzbereich liegende erste und/oder zweite aktuelle Rezirkulationswert herangezogen werden oder gerade nicht. Bspw. kann es bevorzugt sein, bei einer Messung für den ersten aktuellen Rezirkulationswert zu 15 % und bei einer Messung für den zweiten aktuellen Rezirkulationswert Wert zu 12 % diese Messungen nicht verwerfen, sondern das Erreichen einer relevanten Rezirkulation zu ermitteln. Das dient dem Vorteil, ohne Zeitverzug einen Anwender über das Ergebnis informieren bzw. (automatisch und/oder durch Eingreifen des Anwenders) Maßnahmen zur Reduktion der physikalisch stattfindenden Rezirkulation einleiten zu können.

Vorzugsweise kann weiterhin zumindest eine auf einer Dialysierflüssigkeitsseite dem Dialysator vorgeschaltete dritte Sensorvorrichtung vorgesehen sein, sprich, eine Sensorvorrichtung stromauf des Dialysators. Dabei kann die dritte Sensorvorrichtung eingerichtet sein, als das zumindest eine Signal insbesondere eine Leitfähigkeit der unverbrauchten Dialysierflüssigkeit in seinem zeitlichen Verlauf zu erfassen. Weiter bevorzugt ist dritte Sensorvorrichtung als eine temperaturkompensierende Leitfähigkeitssonde, insbesondere zur Messung einer Konzentration eines Kations, ausgebildet. Alternativ oder kumulativ kann die dritte Sensorvorrichtung ein optischer Sensor eingerichtet sein. Dazu kann die Sensorvorrichtung zum Beispiel Licht mit einer Wellenlänge im UV-Bereich emittieren. Vorzugsweise, alternativ oder kumulativ, kann die dritte Sensorvorrichtung und/oder gegebenenfalls eine weitere dem Dialysator vorgeschaltete Sensorvorrichtung in der Dialysierflüssigkeitszulaufleitung direkt an dem Dialysierflüssigkeitszulauf angeordnet sein, bspw. direkt an diesen angeflanscht sein. Bei allen Ausführungsformen kann insbesondere (unabhängig) bevorzugt sein, dass sich die dritte Sensorvorrichtung, bzw. eine weitere Sensorvorrichtung stromauf des Dialysators, auf eine andere physikalische und/oder chemische Größe richtet (bzw. zur Erfassung bzw. Messung einer solchen ausgebildet ist), als die dritte Sensorvorrichtung es ist. Insbesondere kann dabei bevorzugt sein, dass zumindest eine der dem Dialysator vorgeschalteten Sensorvorrichtungen, d.h. die dritte Sensorvorrichtung und/oder die weitere Sensorvorrichtung stromauf des Dialysators, stromauf der Bypassleitung angeordnet ist.

Vorzugsweise, alternativ oder kumulativ, kann die dem Dialysator vorgeschaltete dritte Sensorvorrichtung und die dem Dialysator nachgeschaltete erste Sensorvorrichtung auf Basis eines gleichen Messprinzips eingerichtet sein. Dabei ist die dem Dialysator nachgeschaltete zweite Sensorvorrichtung auf Basis eines demgegenüber anderen Messprinzip eingerichtet. Vorzugsweise, alternativ oder kumulativ, können die dritte Sensorvorrichtung und die erste Sensorvorrichtung eingerichtet sein, um eine gleiche physikalische und/oder chemische Größe, insbesondere eine Leitfähigkeit, als das jeweilig zugehörige Signal, d.h. das dritte Signal der dritten Sensorvorrichtung sowie das erste Signal der ersten Sensorvorrichtung, zu erfassen. Dabei ist die zweite Sensorvorrichtung eingerichtet, um eine demgegenüber, d.h. gegenüber dem dritten Signal sowie dem ersten Signal, andere physikalische und/oder chemische Größe als das zugehörige zweite Signal (der zweiten Sensorvorrichtung) zu erfassen.

Vorzugsweise, alternativ oder kumulativ, kann die Steuer- und Recheneinheit ferner dafür vorgesehen und ausgebildet sein, im Falle einer, für den Grund-Modus im Wesentlichen bestimmbaren, Übereinstimmung des dritten Signals und des ersten Signals das zweite Signal der zweiten Sensorvorrichtung priorisierend für den Messzyklus heranzuziehen, um das Vorhandenseins einer Rezirkulation zu ermitteln, insbesondere den aktuellen Rezirkulationswert zu bestimmen.

Vorzugsweise, alternativ oder kumulativ, kann eine konkrete Ausführungsform der extrakorporalen Blutbehandlungsmaschine folgende Merkmale aufweisen: Dabei kann der Dialysator einen Dialysierflüssigkeitszulauf für frische Dialysierflüssigkeit, einen Dialysierflüssigkeitsablauf für verbrauchte Dialysierflüssigkeit und eine Filtermembran aufweisen. Dabei trennt die Filtermembran die eine Dialysierflüssigkeits-Membranseite, auf der der Dialysator über eine Dialysierflüssigkeitszulaufleitung und eine Dialysierflüssigkeitsablaufleitung an einen Dialysierflüssigkeitskreis angeschlossen ist, von einer Blut-Membranseite, auf der der Dialysator an einen extrakorporalen Blutkreis angeschlossen oder anschließbar ist. Dabei kann eine Bypassleitung vorgesehen sein, mittels derer die Dialysierflüssigkeitsseite (bzw. der Dialysierflüssigkeitskreis) wahlweise in einem Bypass-Modus als dem Interims-Modul zum Interims-Betrieb für ein jeweiliges Interims-Zeitintervall, insbesondere für das erste Interims-Zeitintervall sowie für das zweite Interims-Zeitintervall, überbrückbar wird, um im Dialysator befindliche Dialysierflüssigkeit temporär einzusperren. Dazu kann an der Dialysierflüssigkeitszulaufleitung und der Dialysierflüssigkeitsablaufleitung wenigstens jeweils ein mit der Steuer- und Recheneinheit elektrisch verbundenes Sperrventil zwischen der Bypassleitung und dem Dialysator angeordnet sein. Sprich, die jeweiligen (von der Steuer- und Recheneinheit ansteuerbaren bzw. automatischen) Sperrventile dienen einer Bypass- bzw. Nebenschluss-Schaltung.

Dabei kann die Steuer- und Recheneinheit zur Steuerung der extrakorporalen Blutbehandlungsmaschine mit einer Speichereinheit ausgebildet oder ausrüstbar sein. Alternativ oder kumulativ kann die Steuer- und Recheneinheit über eine Schnittstelle mit einer hinsichtlich der Steuer- und Recheneinheit externen Speichereinheit elektrisch (bzw. signaltechnisch) verbunden oder verbindbar sein.

Dabei kann die extrakorporale Blutbehandlungsmaschine weiter (folgende Merkmale) aufweisen: Ein (von der extrakorporalen Blutbehandlungsmaschine umfasster) Datensatz, der auf der Speichereinheit abgelegt oder ablegbar ist, kann eine Anzahl von für unterschiedliche Parameter der Blutbehandlungsmaschine geeignete Blutflussraten im extrakorporalen Blutkreis, bezeichnet als $Q_B$, und zugehörige, vorzugsweise analytisch vorab bestimmte und/oder pauschal gesetzte, erste und zweite Interims-Zeitintervalle angeben. Dabei entspricht ein jeweiliges erstes Interims-Zeitintervall derjenigen Zeitdauer, innerhalb welcher im Dialysator bei entsprechend eingestellter Blutflussrate $Q_B$ ein Konzentrationsausgleich des zumindest einen ausgewählten oder auswählbaren Blutanteilsstoffs zwischen Blut im extrakorporalen Blutkreis und im Dialysator eingesperrter Dialysierflüssigkeit ausschließlich infolge von Diffusion beendet ist, wobei insbesondere ein maximal möglicher Rezirkulationswert anzunehmen bzw. annehmbar ist. Zur Bestimmung des ersten Bolus-Signals bzw. des zweiten Bolus-Signals ist somit die Steuer- und Recheneinheit vorzugsweise eingerichtet, die extrakorporale Blutbehandlungsmaschine jeweils für die Dauer des im Datensatz angegebenen ersten Interims-Zeitintervalls bzw. des, gegenüber dem ersten Interims-Zeitintervall kürzeren, zweiten Interims-Zeitintervalls in den Bypass-Modus zu schalten. Dabei ist die Steuer- und Recheneinheit vorzugsweise ferner eingerichtet, den extrakorporalen Blutkreis, vorzugsweise gleichzeitig, bei der angegebenen Blutflussrate zu betreiben. Dabei ist die Steuer- und Recheneinheit vorzugsweise ferner eingerichtet, unmittelbar nach jeweiliger Beendigung des Bypass-Modus für den, jeweilig durch den Bypass-Modus entstandenen, ersten bzw. zweiten Dialysierflüssigkeitsbolus in der aus dem Dialysator ablaufenden, verbrauchten Dialysierflüssigkeit mithilfe der zumindest einen Sensorvorrichtung die dem jeweiligen ersten bzw. zweiten Dialysierflüssigkeitsbolus zugehörige Signaländerung gegenüber dem Grund-Signal als ein zugehöriges erstes Bolus-Signal bzw. zweites Bolus-Signal messtechnisch zu erfassen.

Optional kann dabei die extrakorporale Blutbehandlungsmaschine weiter das auf der Speichereinheit abgelegte Berechnungsmodell aufweisen. Anhand des Berechnungsmodells kann die Steuer- und Recheneinheit ferner eingerichtet sein, optional einen aktuellen Rezirkulationswert unter Berücksichtigung des ersten Bolus-Signals, des zweiten Bolus-Signals sowie des Grund-Signals zu berechnen. Die Berechnung kann dabei vorzugsweise vor dem Start oder zu Beginn eines Behandlungszyklus mittels der extrakorporalen Blutbehandlungsmaschine erfolgen. Die Quantifizierung der ggf. als vorhanden bestimmten Rezirkulation im Sinne eines aktuellen Rezirkulationswertes (in %) unterstützt die Präzision und damit den Nutzen für einen Anwender. Somit können Änderungen der Behandlungsparameter bzw. Einstellungen der extrakorporalen Blutbehandlungsmaschine, insbesondere der Blutflussrate, individuell für den jeweiligen Patienten(status) optimiert bzw. adjustiert werden. Insofern dies vorab oder mit Beginn des Behandlungszyklus erfolgt, kann somit die nachteilige Rezirkulation im Vorfeld, mindestens weitgehend, vermieden werden. Dies kann insbesondere iterativ mittels sukzessiv wiederholter Messzyklen erfolgen, insb. automatisch, insb. im Hintergrund der Behandlung. Auf diese Weise kann eine Optimierung hinsichtlich einer effizienten, möglichst kurzen Behandlungsdauer bei gleichzeitiger Vermeidung der nachteiligen Rezirkulation erreicht werden. Weiterhin ist es anhand eines (quantitativ) bestimmten aktuellen Rezirkulationswertes besser möglich, eine noch vertretbare, bspw. bei einer beginnenden Shunt-Verstopfung bzw. - Drosselung auftretende, Rezirkulation mit niedrigen Prozentwerten besser von einer (prohibitiv) hohen unterscheiden zu können. Dies unterstützt die Stellbarkeit einer zutreffenden Diagnose, was (eine) mögliche Ursache(n) der Rezirkulation betrifft. Auch können anhand des Vergleichs einer Vielzahl bzw. einer Historie von (quantitativ) bestimmten aktuellen Rezirkulationswerten Beobachtungen über mehrere Behandlungszyklen und/oder entsprechende Rückschlüsse und/oder Vorhersagen im Sinne einer Wartung, insb. betreffs Verbrauchsmittel des Blutkreises, getroffen werden.

Optional kann dabei die extrakorporale Blutbehandlungsmaschine weiter eine Einrichtung zur Identifizierung des Dialysators und/oder zur Eingabe der Dialysatoridentifikation oder individueller Dialysatorparameter vorsehen. Das bewirkt den Vorteil, dass dem Dialysator zugehörige Parameter, bspw. eine theoretische Clearance, für eine gegebenenfalls weitere Präzisierung des Berechnungsmodells von der Speicher- und Recheneinheit herangezogen werden können. Dies kann bei spezifischen Konstellationen bzw. Diagnosen die Signifikanz des automatisch berechneten Rezirkulationswertes noch weiter erhöhen.

Wie jedoch bereits einleitend dargestellt, löst die vorliegende Offenbarung gegenüber der WO 2020/212 332 A1 die spezifische und wichtige Aufgabe, eine besonders kostengünstige, einfach durchzuführende Rezirkulationsmessung bereitzustellen, welche nicht nur nicht von der systemischen Blutanteilstoff-Konzentration $c_{sys}$ abhängig ist, sondern auch unabhängig von der Kenntnis sonstiger, in besagtem und dem allgemeinen Stand der Technik in die Bestimmung eingehender, Maschinen- bzw. individueller Therapieparameter bzw. Dialysatorparameter, insbesondere sogar quantitativ präzise, Ergebnisse zu liefern in der Lage ist. So konnte in seitens der Anmelderin durchgeführten Experimenten bestätigt werden, dass in repräsentativen Anwendungsfällen die Kenntnis des Dialysators nicht zu einer weiteren Verbesserung der Genauigkeit bzw. Reproduzierbarkeit der offenbarungsgemäßen Rezirkulationsbestimmung führte. Wie vorstehend geschildert, liegen also besondere Vorteile der vorliegenden Offenbarung gerade darin, dass eine innovative Möglichkeit zur Messung bereitgestellt wird, die sich durch Zuverlässigkeit bzw. Reproduzierbarkeit wie gleichmäßiger Sensitivität für das gesamte Messspektrum auszeichnet, dabei gleichzeitig den technisch bzw. apparativ benötigten Aufwand weitgehend gering hält sowie die Möglichkeit des ungewollten Eintrags von vielfachen Messfehlerquellen minimiert.

Insofern kann es alternativ auch bevorzugt sein, die vorliegende Offenbarung in einer medizinischen bzw. klinischen Situation zu nutzen, in welcher (bzw. wobei) die extrakorporale Blutbehandlungsmaschine gerade keine (d.h. nicht eine bzw. nicht notwendigerweise eine) Einrichtung zur Identifizierung des Dialysators und/oder zur Eingabe der Dialysatoridentifikation oder individueller Dialysatorparameter vorsieht. Dies kann beispielsweise in Ländern mit einer weniger guten medizinischen Versorgung besonders vorteilhaft sein und stellt somit einen Beitrag zur weltweiten Verfügbarkeit moderner Blutbehandlungsgeräte mit einer unaufwendigen wie zuverlässigen Behandlungsüberwachung dar.

Vorzugsweise, alternativ oder kumulativ, kann die Speichereinheit in der extrakorporalen Blutbehandlungsmaschine fest integriert sein. Das dient einer kompakten Bauweise mit vereinfachter Montage in der Herstellung. Weiterhin schützt dies vor Manipulationen der herstellerseitigen Inhalte auf der Speichereinheit durch Dritte.

Vorzugsweise, alternativ oder kumulativ, kann die extrakorporale Blutbehandlungsmaschine weiter eine, kabellos oder kabelgebunden verbundene, Anzeigevorrichtung aufweisen, welche zur Anzeige des ermittelten Vorhandenseins einer Rezirkulation, insbesondere des bestimmten aktuellen Rezirkulationswertes, eingerichtet ist. Dabei ist insbesondere für die Verbindung eine Schnittstelle vorgesehen. Beispielsweise kann die Anzeigevorrichtung eine optische Anzeigevorrichtung wie einen, insb. interaktiven, Bildschirm und/oder eine akustische Anzeigevorrichtung, insb. einen interaktiven Sprachausgabecomputer, umfassen. Die Anzeigevorrichtung kann dabei vorzugsweise dislokal auf einem, vorzugsweise mobilen bzw. persönlichen, Gerät wie einem Mobiltelephon, Laptop oder dergleichen des Patienten und/oder eines aus der Ferne behandelnden Arztes vorgesehen sein und/oder als ein solches Gerät vorgesehen sein. Dies bewirkt den Vorteil der Information und/oder des Bio-Feedbacks an einen Anwender der offenbarten extrakorporalen Blutbehandlungsmaschine wie den Patienten und/oder den Arzt bzw. das klinische Personal. Somit können mit der Kenntnis des ggf. Vorhandenseins der Rezirkulation bzw. des aktuellen Rezirkulationswertes entsprechende Änderungen der Behandlungsparameter bzw. Einstellungen der extrakorporalen Blutbehandlungsmaschine, insbesondere der Blutflussrate, individuell für den jeweiligen Patienten(status) optimiert und/oder in Echtzeit aufgrund einer, bspw. erst im Laufe der Dialysebehandlung, aufgetretenen Rezirkulation unverzüglich angepasst werden.

Vorzugsweise, alternativ oder kumulativ, können die zumindest eine Sensorvorrichtung und die Steuer- und Recheneinheit in bzw. an einer separaten, zu der extrakorporalen Blutbehandlungsmaschine nachrüstbaren und/oder nachgerüsteten, Modulvorrichtung zur Rezirkulationsbestimmung ausgelagert vorgesehen sein. Dabei weist die (separate, insb. autonome) Modulvorrichtung eine Sensoreinheit mit der zumindest einen Sensorvorrichtung und einen Anschlussabschnitt oder -teil auf. Dabei ist der Anschlussabschnitt oder -teil zum vorzugsweise adaptiven Anschließen der Sensoreinheit an den Fluidkreis der extrakorporalen Blutbehandlungsmaschine vorgesehen bzw. eingerichtet, derart, dass die zumindest eine Sensorvorrichtung auf der Dialysierflüssigkeitsseite dem Dialysator nachgeschaltet und zum Erfassen des zumindest einen Signals der physikalischen und/oder chemischen Größe der verbrauchten Dialysierflüssigkeit in seinem zeitlichen Verlauf eingerichtet ist. Ferner ermittelt dabei eine als die Steuer- und Recheneinheit ausgebildete bzw. agierende, unabhängig von der extrakorporalen Blutbehandlungsmaschine arbeitende Elektronik- und Auswerteeinheit der Modulvorrichtung das Vorhandensein der Rezirkulation. Insbesondere kann dabei die Steuer- und Recheneinheit bzw. die Elektronik- und Auswerteeinheit der Modulvorrichtung den aktuellen Rezirkulationswert bestimmen. Ferner kann dabei die Modulvorrichtung optional eine, vorzugsweise kombinierte, Eingabe-/ Ausgabeschnittstelle, die dafür ausgebildet ist, einem Benutzer die von der Elektronik- und Auswerteeinheit ermittelte Rezirkulation, insbesondere den bestimmten aktuellen Rezirkulationswert, auszugeben, aufweisen. Ferner kann dabei die Modulvorrichtung optional eine unabhängig von der extrakorporalen Blutbehandlungsmaschine ausgebildete Energieversorgung aufweisen. Dabei ist es gleichermaßen denkbar, dass die Modulvorrichtung eingerichtet ist, um an eine Energieversorgung der Blutbehandlungsmaschine (mit)angeschlossen zu werden.

Ein weiterer, zweitgenannter, Aspekt der vorliegenden Offenbarung betrifft eine (separate) Modulvorrichtung für eine Bestimmung einer Rezirkulation bzw. für eine Überwachung einer Rezirkulation(srate) bei einer extrakorporalen Blutbehandlung zur Nachrüstung an eine extrakorporale Blutbehandlungsmaschine mit einem Dialysator. Zur Vermeidung von Wiederholungen wird hinsichtlich wesentlicher und bevorzugter technischer Merkmale der Modulvorrichtung auf die gleichen Merkmale, wie zu dem vorstehend erstgenannten Aspekt der vorliegenden Offenbarung offenbart, verwiesen. Diese Modulvorrichtung dient vorteilhaft bspw. dazu, eine (vorhandene) extrakorporale Blutbehandlungsmaschine, ggf. auch eines älteren Bautyps, mit einer zusätzlichen Funktionalität zu versehen.

Die Modulvorrichtung (für die extrakorporale Blutbehandlungsmaschine und/oder der extrakorporalen Blutbehandlungsmaschine) weist eine Steuer- und Recheneinheit und eine mit der Steuer- und Recheneinheit elektrisch verbundene Sensoreinheit auf. Dabei ist die Steuer- und Recheneinheit als eine unabhängig von der extrakorporalen Blutbehandlungsmaschine arbeitende Elektronik- und Auswerteeinheit ausgebildet. Dabei hat die Sensoreinheit zumindest eine Sensorvorrichtung und einen Anschlussabschnitt oder -teil zum vorzugsweise adaptiven Anschließen der Sensoreinheit an den Fluidkreis der extrakorporalen Blutbehandlungsmaschine, derart, dass die zumindest eine Sensorvorrichtung auf einer Dialysierflüssigkeitsseite dem Dialysator nachgeschaltet ist. Dabei ist die Sensorvorrichtung eingerichtet (und angeschlossen/anschließbar), um zur quantitativen Bestimmung zumindest eines vorzugsweise ausgewählten oder auswählbaren Blutanteilsstoffs in der verbrauchten Dialysierflüssigkeit zumindest ein Signal einer physikalischen und/oder chemischen Größe der verbrauchten Dialysierflüssigkeit zu messen.

Dabei ist die Steuer- und Recheneinheit zur Bestimmung eines Vorhandenseins einer Rezirkulation ohne blutseitige Bolusgabe dafür vorgesehen und ausgebildet:

- das von der zumindest einen Sensorvorrichtung gemessene zumindest eine Signal in seinem zeitlichen Verlauf zu erfassen;
- die extrakorporale Blutbehandlung in einem Grund-Modus zum kontinuierlichen Betrieb, in welchem die Dialysierflüssigkeitsströmung durch den Dialysator zugelassen ist, zu betreiben,
- das der in dem Grund-Modus verbrauchten Dialysierflüssigkeit zugehörige Signal als ein Grund-Signal zu messen;
- die extrakorporale Blutbehandlung von dem Grund-Modus in einen Interims-Modus zum Interims-Betrieb, in welchem eine Dialysierflüssigkeitsmenge auf der Dialysierflüssigkeits-Membranseite im Dialysator eingesperrt wird, während der Blutstrom im extrakorporalen Blutkreis mit einer festgelegten Blutflussrate betrieben wird, wobei das Blut auf der Blut-Membranseite weiter strömt, für die jeweilige Dauer eines vorbestimmten und/oder vorbestimmbaren Interims-Zeitintervalls zu schalten, insbesondere in einen Bypass-Modus als den Interims-Modus zu schalten; nachfolgend
- in den Grund-Modus zu wechseln, um die zuvor eingesperrte Dialysierflüssigkeitsmenge als einen jeweiligen Dialysierflüssigkeitsbolus der zumindest einen Sensorvorrichtung zuzuführen, um eine dem Dialysierflüssigkeitsbolus zugehörige Signaländerung gegenüber dem Grund-Signal als ein zugehöriges Bolus-Signal zu messen;
- den Interims-Modus für ein erstes Interims-Zeitintervall zur Gleichgewichtseinstellung des Stofftransports zu schalten, wobei eine erste Dialysierflüssigkeitsmenge im Dialysator zumindest solange eingesperrt wird, bis sich auf der Dialysierflüssigkeits-Membranseite und der Blut-Membranseite des Dialysators ein sich nicht mehr oder nur noch unwesentlich veränderndes Konzentrationsgleichgewicht des Blutanteilsstoffs einstellt.

Dabei ist die Steuer- und Recheneinheit ferner zur Ausbildung eines Messzyklus dafür vorgesehen und ausgebildet:

- den Interims-Modus für ein, gegenüber dem ersten Interims-Zeitintervall kürzeres, zweites Interims-Zeitintervall zu schalten, wobei eine zweite Dialysierflüssigkeitsmenge im Dialysator nur solange eingesperrt wird, dass sich das Konzentrationsgleichgewicht des Blutanteilsstoffs noch nicht einstellt,
- aus einer Abweichung eines zweiten Bolus-Signals in Folge des zweiten Interims-Zeitintervalls gegenüber einem ersten Bolus-Signal in Folge des ersten Interims-Zeitintervalls das Vorhandensein der Rezirkulation zu ermitteln.

Dies ermöglicht, die vorliegend offenbarte Rezirkulationsbestimmung aufgrund der Beheitmatung / Anordnung von Sensorik und Auswertung / Steuerung in der separaten (ggf. mobilen) Modulvorrichtung, d.h. außerhalb der Maschinengrenze der primären extrakorporalen Blutbehandlungsmaschine, unabhängig von letzterer und deren technischer Konfiguration wie einem Baujahr/-typ usw. darzustellen. Die Nachrüstbarkeit ermöglicht eine kostensparende, nachhaltige Aufwertung von älteren Blutbehandlungsmaschinen mit neuen Funktionen.

Insbesondere kann die Modulvorrichtung eine (optionale) Eingabe-/ Ausgabeschnittstelle zu einem Benutzer, die dafür ausgebildet ist, einem Benutzer die von der Steuer- und Recheneinheit bestimmte Rezirkulation auszugeben, aufweisen. Insbesondere kann die Modulvorrichtung eine (optionale) unabhängig von der extrakorporalen Blutbehandlungsmaschine ausgebildete Energieversorgung aufweisen.

Insbesondere kann die Modulvorrichtung (optionale) zumindest zwei, insb. drei Sperrventile zur Schaltung in einen Interims-Betrieb des Fluidkreises aufweisen. In einer über vorgenannte Kernausstattung der Modulvorrichtung weiter bevorzugten erweiterten Konfiguration hat die Modulvorrichtung weiter optional ein Bypass-Sperrventil in der Bypass-Leitung, d.h. zur Fluidverbindung der Dialysierflüssigkeitsquelle mit der Dialysierflüssigkeitssenke, sowie zumindest das dem Dialysator vorgeschaltete (erste) Sperrventil (d.h. Eingangsventil zu dem Dialysator) in der Dialysierflüssigkeitszulaufleitung, insb. beide Sperrventile (d.h. das erste Sperrventil stromauf/vor dem Dialysator und das zweite Sperrventil stromab/nach dem Dialysator), um in einen Interims-Betrieb, insb. den Bypass-Modus, des Fluidkreises zu schalten. Sprich, die Sperrventile der extrakorporalen Blutbehandlungsmaschine, die für die oben beschriebene Bypass-Schaltung benötigt werden, sind in einem solchen optionalen Ausführungsbeispiel der erweiterten Modulvorrichtung zur Nachrüstung zugeordnet bzw. an/in dieser vorgesehen, insb. zu denen der Blutbehandlungsmaschine dupliziert. Auch ältere Blubehandlungsmaschinen können somit ohne Umrüstung bzw. Softwareupdates, demnach uneingeschränkt, mit der offenbarten neuen Funktionalität ausgestattet werden. Ferner dient die Modulvorrichtung einer weiteren Reduktion der Komplexität bzw. Verbesserung der Interaktion Mensch-Maschine. Insb. entfällt vorteilhafterweise eine Notwendigkeit für einen Benutzer, den Bypass-Modus an der Blutbehandlungsmaschine zu starten.

Ein weiterer, drittgenannter, Aspekt der vorliegenden Offenbarung betrifft ein Verfahren zur Überwachung einer Rezirkulation(srate) bei einer extrakorporalen Blutbehandlung unter Verwendung einer extrakorporalen Blutbehandlungsmaschine nach dem vorstehend erstgenannten Aspekt der vorliegenden Offenbarung und/oder unter Verwendung einer Modulvorrichtung zur Rezirkulationsbestimmung nach dem vorstehend zweitgenannten Aspekt der vorliegenden Offenbarung. Zur Vermeidung von Wiederholungen wird hinsichtlich wesentlicher und bevorzugter technischer Merkmale auf die Offenbarung insb. zu dem erstgenannten Aspekt verwiesen. Dabei weist das Verfahren zur Überwachung die folgenden (Überwachungs-)Schritte auf: Ein optionaler Schritt betrifft ein Identifizieren des Dialysators. In einem anfänglichen Schritt erfolgt ein Vorbestimmen des ersten Interims-Zeitintervalls, innerhalb welchem sich ein Konzentrationsgleichgewicht eines vorzugsweise zuvor ausgewählten Blutanteilsstoffs, vorzugsweise Harnstoff, einstellt. Insb. kann das Vorbestimmen des ersten Interims-Zeitintervalls in Abhängigkeit einer eingestellten Blutflussrate $Q_B$ oder pauschal erfolgen. In einem weiteren anfänglichen Schritt erfolgt ein Vorbestimmen des zweiten Interims-Zeitintervalls, innerhalb welchem sich das Konzentrationsgleichgewicht des Blutanteilsstoffs noch nicht einstellt. Demzufolge betrifft das zweite Interims-Zeitintervalls eine gegenüber dem ersten Interims-Zeitintervall kürzere Zeitdauer.

Daraufhin erfolgen ein Schritt eines Ermittelns eines ersten Bolus-Signals in Folge des ersten Interims-Zeitintervalls sowie ein Schritt eines Ermittelns eines zweiten Bolus-Signals in Folge des zweiten Interims-Zeitintervalls in zueinander beliebiger Reihenfolge.

Der Schritt des Ermittelns des ersten Bolus-Signals weist folgende (insb. sequenziellen) Unterschritte auf: i) Betreiben der extrakorporalen Blutbehandlungsmaschine in dem Grund-Modus sowie Messen des, der in dem Grund-Modus verbrauchten Dialysierflüssigkeit zugehörigen, Signals als einem Grund-Signal; ii) Umschalten von dem Grund-Modus in den Interims-Modus, insbesondere in den Bypass-Modus, für die Dauer des ersten Interims-Zeitintervalls; sowie nachfolgend zu Unterschritt (ii) bzw. zu dessen Beendigung iii) (Zurück-) Wechseln in den Grund-Modus, um die zuvor eingesperrte Dialysierflüssigkeitsmenge als den ersten Dialysierflüssigkeitsbolus der zumindest einen Sensorvorrichtung zuzuführen, und darauffolgendes Messen des ersten Bolus-Signals. Dabei betrifft das erste Bolus-Signal eine dem ersten Dialysierflüssigkeitsbolus zugehörige Signaländerung (bzw. eine Respons) gegenüber (bzw. im Vergleich zu) dem Grund-Signal. Während die Unterschritte (ii) und (iii) in nachfolgender Sequenz erfolgen müssen bzw. von der dazu eingerichteten Steuer- und Recheneinheit (automatisch) ausgeführt werden, ist es möglich, den Unterschritt (i) zeitlich vor oder nach den Unterschritten (ii) mit (iii) in Kombination auszuführen.

Vorher oder nachher zum Schritt des Ermittelns des ersten Bolus-Signals erfolgt der Schritt des Ermittelns eines zweiten Bolus-Signals in Folge des zweiten Interims-Zeitintervalls mit zugehörigen Unterschritten. Dabei sind die zugehörigen Unterschritte analog zu denen des Schrittes des Ermittelns des ersten Bolus-Signals.

Auf Grundlage des ermittelten zweiten Bolus-Signals gegenüber (bzw. im Vergleich zu) dem ermittelten ersten Bolus-Signal erfolgt offenbarungswesentlich ein Schritt eines Ermittelns des Vorhandenseins der Rezirkulation aus (bzw. auf Basis der Feststellung) einer Abweichung des zweiten Bolus-Signals gegenüber dem ersten Bolus-Signal. Insbesondere kann dabei der Schritt des Ermittelns des Vorhandenseins der Rezirkulation in Weise (bzw. als) ein Berechnen eines aktuellen Rezirkulationswertes (R in %) anhand eines Berechnungsmodells gemäß einem Quotienten erfolgen, wobei der Quotient als das zweite Bolus-Signal bezogen auf das erste Bolus-Signal definiert ist. Dabei versteht der Fachmann, dass der Quotient auch in Form eines Kehrwertes oder sonstig abgewandelten Kennwertes der vorgenannten Definition verwendet werden kann, was er mathematisch entsprechend im Sinne der Auswertung zu berücksichtigen weiß. Die Rezirkulation bzw. der aktuelle Rezirkulationswert kann in einem optionalen Schritt eines Anzeigens einem Anwender angezeigt bzw. qualitativ, vorzugsweise (halb)quantitativ, ausgewiesen werden.

Vorteilhafterweise kann somit eine tatsächlich vorliegende Rezirkulation bestimmt werden, ohne dass vorab durch separate Blutprobenentnahme am Patienten die Konzentration eines Blutanteilsstoffs im Blut des Patientenkörpers bestimmt werden muss, und folglich kann ein vollautomatisiertes, zeitsparendes und sicheres Blutbehandlungsverfahren ermöglicht werden.

**Kurzbeschreibung der Figuren**

Die Offenbarung wird nachstehend anhand bevorzugter Ausführungsbeispiele unter Bezugnahme auf die beigefügte Zeichnung näher beschrieben. Es zeigen:

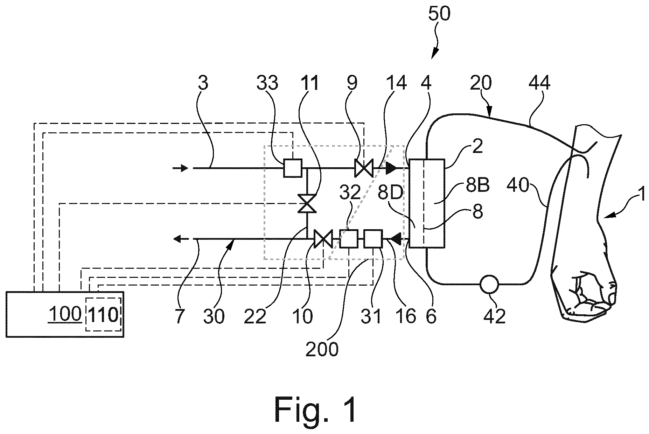

Fig. 1 ein bevorzugtes Ausführungsbeispiel einer Dialysemaschine als einer offenbarungsgemäßen extrakorporalen Blutbehandlungsmaschine unter Veranschaulichung einer (optionalen) Modulvorrichtung;

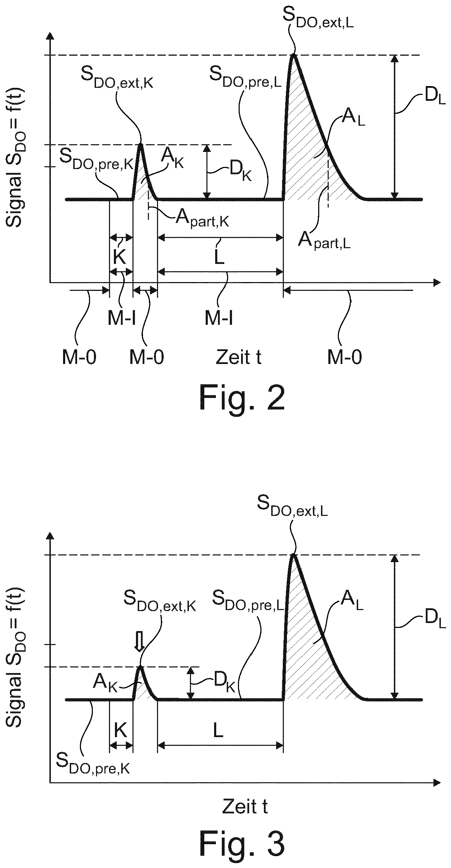

Fig. 2 ein erstes Ausführungsbeispiel eines offenbarungsgemäßen zeitlichen Verlaufs eines an einer Sensorvorrichtung einer offenbarungsgemäßen Dialysemaschine gemessenen Signals in zeitlicher Schaltungsreihenfolge eines kurzen Bypass-Modus gefolgt von einem langen Bypass-Modus, den Fall von 0 % Rezirkulation veranschaulichend, wobei die Sensorvorrichtung direkt hinter dem Dialysierflüssigkeitsablauf des Dialysators angeordnet ist;

Fig. 3 ein zweites Ausführungsbeispiel eines offenbarungsgemäßen zeitlichen Verlaufs des gemessenen Signals, den Fall von 25 % Rezirkulation veranschaulichend, wobei ansonsten vergleichbare Bedingungen gegenüber dem ersten Ausführungsbeispiel in Fig. 2 bestehen;

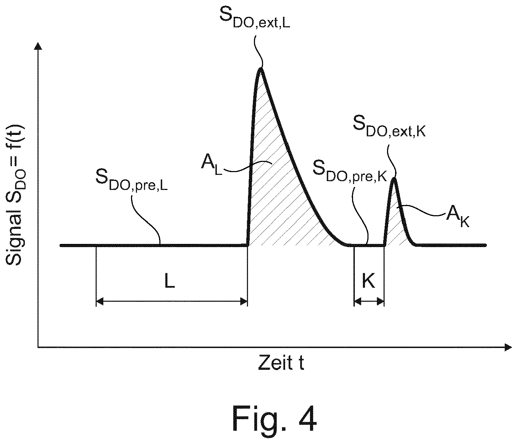

Fig. 4 ein drittes Ausführungsbeispiel eines offenbarungsgemäßen zeitlichen Verlaufs des an einer Sensorvorrichtung einer offenbarungsgemäßen Dialysemaschine gemessenen Signals in gegenüber dem ersten Ausführungsbeispiel in Fig. 2 bzw. gegenüber dem zweiten Ausführungsbeispiel in Fig. 3 geänderter zeitlicher Schaltungsreihenfolge eines langen Bypass-Modus gefolgt von einem kurzen Bypass-Modus, zur Veranschaulichung der grundsätzlichen Unabhängigkeit von der Schaltungsreihenfolge, insofern auf den jeweilig zugehörigen zeitlichen Verlaufsabschnitt des langen Bypass-Modus versus dem des kurzen Bypass-Modus bezogen.

Die Figuren sind lediglich schematischer Natur und dienen ausschließlich dem Verständnis der Offenbarung. Die gleichen Elemente sind mit denselben Bezugzeichen bezeichnet.

**Beschreibung bevorzugter Ausführungsbeispiele**

Nachstehend werden Ausführungsbeispiele der vorliegenden Offenbarung auf der Basis der zugehörigen Figuren beschrieben.

Fig. 1 zeigt schematisch ein bevorzugtes Ausführungsbeispiel einer offenbarungsgemäßen extrakorporalen Blutbehandlungsmaschine zur Bestimmung der Rezirkulation, ausgebildet als eine Dialysemaschine 50 mit einem Dialysator 2, der einen Dialysierflüssigkeitszulauf 4 und einen Dialysierflüssigkeitsablauf 6 aufweist. Ferner zeigt Fig. 1 eine (optionale) Modulvorrichtung 200, wie weiter unten ausgeführt.

Einem Patienten 1 wird über eine arterielle Blutleitung 40 mittels einer Blutpumpe 42 Blut entnommen und zum Dialysator 2 geleitet. Dort wird es gereinigt und anschließend über die venöse Leitung 44 als ein extrakorporaler Blutkreis 20 zurück zum Patienten 1 geleitet.

Der Dialysator 2 ist zum Zwecke der Blutreinigung weiter mit einer semipermeablen Filtermembran 8 ausgestattet, welche eine (dialysatseitige) Dialysierflüssigkeits-Membranseite 8D von einer (blutseitigen) Blut-Membranseite 8B trennt bzw. das Volumen des Dialysators in diese zwei Teilvolumina aufteilt. Dabei steht die Dialysierflüssigkeits-Membranseite 8D mit einer Dialysierflüssigkeitszulaufleitung 14 und einer Dialysierflüssigkeitsablaufleitung 16 eines Dialysierflüssigkeitskreises 30 in Fluidverbindung. Der Dialysierflüssigkeitskreis 30 stellt dabei einen Strömungsweg zwischen einer Dialysierflüssigkeitsquelle 3 hin zu einer Dialysierflüssigkeitssenke 7 dar. Die Blut-Membranseite 8B steht in Fluidverbindung mit dem extrakorporalen Blutkreis 20.

Bspw. kann der Dialysator 2 in Form eines (im Gegenstrom, Gleichstrom und/oder Kreuzstrom betreibbaren) Hohlfasermoduls ausgebildet sein, wobei die Filtermembran 8 in Form von, mit der Dialysierflüssigkeit (bzw. dem Dialysat) des Dialysierflüssigkeitskreis 30 umströmten, Hohlfaserbündeln ausgebildet ist. Dabei ist die Dialysierflüssigkeitszulaufleitung 14 an den Dialysierflüssigkeitszulauf 4 angeschlossen; und ist die Dialysierflüssigkeitsablaufleitung 16 an den Dialysierflüssigkeitsablauf 6 des Dialysators 2 angeschlossen.

In dem Dialysierflüssigkeitskreis 30 ist weiter eine Bypassleitung 22 vorgesehen, mit der der Dialysator 2 bzw. die Dialysierflüssigkeits-Membranseite 8D des Dialysators 2 überbrückt, also strömungstechnisch umgangen, werden kann.

Dies erfolgt (vgl. Figuren 2 bis 4) in einem (um)geschalteten Bypass-Modus M-I als Interims-Modus. Dabei wird in den Bypass-Modus M-I (um)geschaltet, um zeitlich einen Grund-Modus M-0 eines kontinuierlichen Dialysebetriebes [d.h. eines Dialysebetriebes, bei welchem die Dialysierflüssigkeit kontinuierlich über die bzw. entlang der Dialysierflüssigkeits-Membranseite 8D (hinweg) gefördert wird bzw. fließt] abzulösen bzw. um aus dem Grund-Modus M-0 in den Bypass-Modus M-I (temporär bzw. interimsweise) zu wechseln. Dazu ist in der Dialysierflüssigkeitszulaufleitung 14 und der Dialysierflüssigkeitsablaufleitung 16 jeweils ein Sperrventil 9, 10 vorgesehen, mit denen die Dialysierflüssigkeitszulaufleitung 14 und die Dialysierflüssigkeitsablaufleitung 16 geöffnet bzw. geschlossen werden können und somit entweder den Strömungsweg für frische Dialysierflüssigkeit bei geöffneten Sperrventilen 9, 10 durch die Dialysierflüssigkeitszulaufleitung 14, die Dialysierflüssigkeits-Membranseite 8D des Dialysators 2 und die Dialysierflüssigkeitsablaufleitung 16 freigeben, oder durch Verschließen zumindest des Sperrventils 9, idealerweise beider Sperrventile 9, 10, diesen Strömungsweg verschließen. Sprich, sind diese Sperrventile 9, 10 geschlossen, strömt die Dialysierflüssigkeit bei geöffnetem Bypass-Sperrventil 11 durch die Bypassleitung 22 am Dialysator 2 vorbei. Dieser Betriebszustand definiert den Bypass-Modus M-I (Fig. 2).

In der bevorzugten Ausführungsform der offenbarungsgemäßen Dialysemaschine 50 befindet sich in der Dialysierflüssigkeitsablaufleitung 16 bzw. stromab des Dialysierflüssigkeitsablaufs 6 (und bspw., aber nicht limitierend, stromauf des Sperrventils 10) eine erste Sensorvorrichtung 31 als die zumindest eine Sensorvorrichtung und weiterhin eine optionale zweite Sensorvorrichtung 32.

Ferner ist Fig. 1 zu entnehmen, dass in der Dialysierflüssigkeitszulaufleitung 14 bzw. stromauf des Dialysierflüssigkeitszulaufs 4 (und bspw., aber nicht limitierend, stromauf des Sperrventils 9) eine optionale dritte Sensorvorrichtung 33 vorgesehen sein kann.

Die jeweiligen Sensorvorrichtungen 31, 32, 33 messen jeweils eine chemische und/oder physikalische Größe des Dialysats. Bei der ersten Sensorvorrichtung 31 und der dritten Sensorvorrichtung 33 handelt es sich vorzugsweise um Sensorvorrichtungen zur Leitfähigkeitsbestimmung, insbesondere um temperaturkompensierende Leitfähigkeitssonden.

Die optionale zweite Sensorvorrichtung 32 ist beispielsweise als optische Sensorvorrichtung ausgebildet. Dazu nimmt die zweite Sensorvorrichtung 32 in der vorbeiströmenden Dialysierflüssigkeit mittels UV/VIS-Spektroskopie abhängig vom Absorptionsbereich des konkreten zu messenden Blutanteilsstoffs Messungen bei der von diesem Blutanteilsstoff absorbierbaren Wellenlänge vor, um vorzugsweise eine Konzentrationsbestimmung dieses Blutanteilsstoffs oder eine entsprechende Kenngrößenbestimmung zu ermöglichen. Konzentrations-/Kenngrößenbestimmungen durch Absorptionsmessung sind allgemein bekannt und werden daher an dieser Stelle nicht näher erläutert.

Neben den vorstehend konkretisierten Messverfahren sind auch alternative Messverfahren denkbar. Die jeweilige Sensorvorrichtung, insbesondere die erste, zweite und/oder dritte Sensorvorrichtung 31, 32 und 33, kann in Flussrichtung vor bzw. stromauf (wie in Fig. 1 beispielhaft abgebildet) oder hinter bzw. stromab der Bypassleitung 22 positioniert sein.

Die an der ersten Sensorvorrichtung 31 und an den optionalen zweiten und dritten Sensorvorrichtung 32, 33 generierten gemessenen Signalwerte werden an eine Steuer- und Recheneinheit 100 übertragen und von dieser ausgewertet.

Die Steuer- und Recheneinheit 100 steht zumindest mit den Sperrventilen 9, 10 und den Sensorvorrichtungen 31, 32, 33 in gegenseitigem Informationsaustausch, d.h. sie empfängt und sendet Informationen jeweilig von den Sperrventilen 9, 10 und den Sensorvorrichtungen 31, 32, 33 bzw. an die Sperrventile 9, 10 und die Sensorvorrichtungen 31, 32, 33. Insbesondere hinsichtlich der jeweiligen Sensorvorrichtung 31, 32, 33 betreffen die Informationen ein zeitlich veränderliches Signal $S_{DO}$ = f(t) [vgl. Ordinate der Figuren 2 bis 4], welchem jeweils die Messung einer chemischen und/oder physikalischen Größe des Dialysats zur Messung des zu messenden Blutanteilsstoffs zugrundeliegt.

Weiter ist die Steuer- und Recheneinheit 100 mit einer Speichereinheit 110 verbunden oder ausgestattet. Auf dieser Speichereinheit 110 ist zumindest ein Datensatz abgelegt bzw. ablegbar, mit dem die Dialysemaschine 50 abhängig von den vorgegebenen bzw. empfangenen Informationen gesteuert werden kann.

Die Dialysemaschine 50 verfügt weiter über ein weiteres Sperrventil in Form des Bypass-Sperrventils 11 in der Bypassleitung 22, das ebenfalls mit der Steuer- und Recheneinheit 100 in Informationsaustauschkontakt steht und von dieser gesteuert, also geöffnet und geschlossen werden kann. Das Bypass-Sperrventil 11 ist vorgesehen, um den Strömungsweg für frische Dialysierflüssigkeit über die Bypassleitung 22 freizugeben oder zu sperren. Die frische Dialysierflüssigkeit wird aus einer Dialysierflüssigkeitsbereitstellungseinheit als der Dialysierflüssigkeitsquelle 3 bezogen.

Stromab der Dialysierflüssigkeitsquelle 3 kann sich (optional für jegliche Ausführungsformen) eine Bilanzkammer (nicht abgebildet) befinden, die eingerichtet ist, in den Dialysierflüssigkeitskreis 30 einströmende frische Dialysierflüssigkeit und aus diesem ausströmende (verbrauchte) Dialysierflüssigkeit zu bilanzieren. Dabei kann vorzugsweise die (nicht abgebildete) Bilanzkammer strömungstechnisch so angeordnet sein, dass diese sich zwischen der Dialysierflüssigkeitsquelle 3 und einer Mündungsstelle der Dialysierflüssigkeitszulaufleitung 14 in die Bypassleitung 22 sowie zwischen der Dialysierflüssigkeitssenke 7 und einer Mündungsstelle der Bypassleitung 22 in die Dialysierflüssigkeitsablaufleitung 16 befindet.

Auf der Blut-Membranseite 8B des Dialysators 2 ist der extrakorporale Blutkreis 20 an den Dialysator 2 angeschlossen. Der Blutkreis 20 weist zumindest eine arterielle Blutleitung 40 auf, die einen arteriellen Patientenzugang mit einem blutseitigen Dialysatoreingang verbindet und an der eine Blutpumpe 42 angeordnet ist, und eine venöse Blutleitung 44, die einen blutseitigen Dialysatorausgang mit einem venösen Patientenzugang verbindet. Die Blutpumpe 42 befindet sich ebenfalls in (gegenseitigem) Informationsaustauschkontakt mit der Steuer- und Recheneinheit 100 und wird von dieser gesteuert.

Wie weiterhin Fig. 1 anhand der Einkastung mit grauen punktierten Linien schematisch veranschaulicht, kann die (optionale) Modulvorrichtung 200 separat vorgesehen sein. Dabei kann die (separate) Modulvorrichtung 200 nachgerüstet bzw. nachrüstbar an die Dialysemaschine 50 als die extrakorporale Blutbehandlungsmaschine sein. In einer Kernausstattung der optionalen Modulvorrichtung 200 (wie in Fig. 1 rechts von der grauen punktierten Schräglinie) weist diese eine als die Steuer- und Recheneinheit 100 ausgebildete, unabhängig von der Dialysemaschnie 50 arbeitende Elektronik- und Auswerteeinheit zur Rezirkulationsermittlung sowie eine mit der Steuer- und Recheneinheit 100 elektrisch verbundene Sensoreinheit mit der zumindest einen (ersten) Sensorvorrichtung 31 auf. Dabei hat die Sensoreinheit zusätzlich zu der zumindest einen (ersten) Sensorvorrichtung 31 einen Anschlussabschnitt oder -teil zum vorzugsweise adaptiven Anschließen der Sensoreinheit an den Dialysierflüssigkeitskreis 30 der Dialysemaschine 50 derart, dass die zumindest eine Sensorvorrichtung 31 (ggf. 32) auf der Dialysierflüssigkeitsseite dem Dialysator 2 nachgeschaltet und zum Erfassen des Signals $S_{DO}$ dient. Die Modulvorrichtung 200 ist über die Dialysierflüssigkeitszulaufleitung 14 stromauf der Dialysierflüssigkeits-Membranseite 8D mit dieser fluidverbunden bzw. fluidverbindbar. Die Modulvorrichtung 200 ist über die Dialysierflüssigkeitsablaufleitung 14 stromab der Dialysierflüssigkeits-Membranseite 8D mit dieser fluidverbunden bzw. fluidverbindbar.

In einer bevorzugten, über vorgenannte Kernausstattung der Modulvorrichtung 200 hinaus erweiterten Konfiguration (wie in Fig. 1 links von der grauen punktierten Schräglinie) hat die Modulvorrichtung 200 weiter optional ein Bypass-Sperrventil 11 in der Bypass-Leitung 22 zur Fluidverbindung der Dialysierflüssigkeitsquelle 3 mit der Dialysierflüssigkeitssenke 7 und zumindest das dem Dialysator 2 vorgeschaltete (erste) Sperrventil 9 (d.h. Eingangsventil zu dem Dialysator 2) in der Dialysierflüssigkeitszulaufleitung 14, insb. beide Sperrventile 9, 10 (d.h. das erste Sperrventil 9 stromauf/vor dem Dialysator 2 und das zweite Sperrventil 10 stromab/nach dem Dialysator 2), um in einen Interims-Betrieb, insb. den Bypass-Modus, des Fluidkreises zu schalten. Sprich, die Sperrventile der Dialysemaschine 50, die für oben beschriebene Bypass-Schaltung benötigt werden, gehören in einem solchen optionalen Ausführungsbeispiel zu der nachgerüsteten/nachrüstbaren Modulvorrichtung 200. Dabei handelt es sich zumindest um zwei Sperrventile 9, 11 (d.h. für den Interims-Modus), insb. um drei Sperrventile 9, 10, 11 (d.h. für den Bypass-Modus M-I). Ein drittes Sperrventil ist das Bypass-Sperrventil 11, das geöffnet die Dialysierflüssigkeit um den Dialysator 2 im Bypass-Modus bei geschlossenen Sperrventilen 9, 10 herumfließen lässt. Dadurch umgeht die Dialysierflüssigkeit im Bypass-Modus den Dialysator 2. Allgemein (d.h. unabhängig von einer optionalen Modulvorrichtung 200) sei angemerkt, dass das zweite Sperrventil 10 nicht zwingend geschlossen sein muss. Bspw. kann es fallweise bevorzugt sein, dass bei geöffnetem zweiten Sperrventil 10 weiterhin ultrafiltriert, d.h. Flüssigkeit aus dem Patientenblut entzogen werden kann. Je länger der Bypass-Modus dauert, desto vorteilhafter ist es, das zweite Sperrventil 10 nicht zu schließen. Bei einem kurzen Bypass-Modus und/oder niedrigen Ultrafiltrationsraten ist es für die Performance des Sättigungsverfahrens jedoch besser, wenn zusätzlich zu dem ersten Sperrventil 9 auch das zweite Sperrventil 10 für den Bypass-Modus geschlossen ist/wird.

Im Betrieb der Dialysemaschine 50 (Fig.1; unter Bezugnahme auf alle Ausführungsbeispiele der in den Figuren 2 bis 4 gezeigten zeitlichen Verläufe der gemessenen Signale) wird zunächst ein Patient 1 mit dem extrakorporalen Blutkreis 20 verbunden. Anschließend ruft die Steuer- und Recheneinheit 100 aus dem Datensatz, der auf der Speichereinheit 110 abgelegt ist, eine Soll-Blutflussrate $Q_B$ und jeweilige Soll-Zeitwerte für die jeweilige Dauer eines herstellerseitig vorbestimmten jeweiligen Interims-Zeitintervalls für den Bypass-Modus ab. Unter Bezugnahme auf die Figur 2 (sowie Figuren 3, 4) betreffen die vorbestimmten Interims-Zeitintervalle für den Bypass-Modus M-I ein erstes Interims-Zeitintervall L zur Gleichgewichtseinstellung des Stofftransports sowie ein demgegenüber verkürztes zweites Interims-Zeitintervall K hinsichtlich eines kinetischen Zeitpunktes vorab der Gleichgewichtseinstellung (gemäß dem ersten Interims-Zeitintervall L). Dabei können optional patientenabhängige minimale und maximale Obergrenzen für die Soll-Blutflussrate $Q_B$ im Datensatz Berücksichtigung finden. Der Zeitwert für das erste Interims-Zeitintervall L im Bypass-Modus M-I ist dabei vorzugsweise so gewählt/festgelegt, dass während des Bypass-Modus M-I auch unter dem schlechtesten Umstand, nämlich einer maximal möglichen oder maximal zu erwartenden Rezirkulation (z.B. Rezirkulationswert von 20% bis 30%), ein (nahezu) Diffusionsgleichgewicht für den, für die zumindest eine Sensorvorrichtung 31, 32 heranzuziehenden, Signal-relevanten, Blutanteilsstoff im Dialysator 2 erreicht wird. Hier gilt die Regel, dass je geringer die Blutflussrate $Q_B$ und je größer der Dialysator ist, es desto länger bis zum Erreichen des (nahezu) Diffusionsgleichgewichts dauert. Abhängig von dem konkreten für das Signal zu erfassenden Blutanteilsstoff, der optional vor der Behandlung aus mehreren möglichen Blutanteilsstoffen ausgewählt wird, kann das ausgegebene erste Interims-Zeitintervall L zusätzlich größer oder kleiner ausfallen.

Nachdem die Steuer- und Recheneinheit 100 das erste Interims-Zeitintervall L und das zweite Interims-Zeitintervall K als die Soll-Zeitwerte sowie die Soll-Blutflussrate $Q_B$ abgerufen hat bzw. diese manuell eingegeben wurden, stellt sie die Blutpumpe 42 auf die Soll-Blutflussrate $Q_B$ ein. Ist die Soll-Blutflussrate $Q_B$ erreicht und ist auch ein vorgegebener Wert für den Dialysierflüssigkeitsstrom durch den Dialysierflüssigkeitskreis 30 erreicht, kann ein stationärer Zustand des kontinuierlichen Betriebs angenommen werden bzw. ist ein solcher eingestellt. Der stationäre Zustand des kontinuierlichen Betriebs, insbesondere hinsichtlich der kontinuierlichen Durchströmung und kontinuierlich stattfindenden grenzflächenphysikalischen Stofftransport- bzw. Dialysevorgänge, definiert einen Grund-Modus M-0 (siehe Figur 2).

Nun kann in dem Grund-Modus M-0 zum kontinuierlichen Betrieb, in welchem die Dialysierflüssigkeitsströmung durch den Dialysator 2 zugelassen ist, das der verbrauchten Dialysierflüssigkeit zugehörige Signal von der ersten Sensorvorrichtung 31 als der zumindest einen Sensorvorrichtung und/oder von der zweiten Sensorvorrichtung 32 als ein (eingependeltes bzw. konstantes) Grund-Signal $S_{DO,\,pre}$ gemessen werden.

Jeweils nach dem (Erreichen des) Grund-Modus M-0 wird die Dialysemaschine 50 von der Steuer- und Recheneinheit 100 in den Bypass-Modus M-I geschaltet (siehe insb. Fig. 2, ferner Figuren 3, 4). Das bedeutet, dass für die jeweiligen vorbestimmten Interims-Zeitintervalle für den Bypass-Modus M-I, zum einen für das erste Interims-Zeitintervall L (Gleichgewicht) sowie zum anderen für das zweite Interims-Zeitintervall K (Ungleichgewicht; treibendes Konzentrationsgefälle nicht vernachlässigbar), und zwar in beliebiger Reihenfolge, die Sperrventile 9, 10 geschlossen sind, also die zwischen den Sperrventilen 9, 10 befindliche Dialysierflüssigkeit eingesperrt ist, und das Bypass-Sperrventil 11 in der Bypassleitung 22 geöffnet ist, so dass der Strömungsweg der frischen Dialysierflüssigkeit aus der Dialysierflüssigkeitsquelle 3 über die Bypassleitung 22 zu der Dialysierflüssigkeitssenke 7 führt.

Für die Dauer des Bypass-Modus wird nun die Blutpumpe 42 mit der eingestellten Blutflussrate betrieben, während die Dialysierflüssigkeit auf der Dialysierflüssigkeits-Membranseite 8D steht. Infolgedessen tritt aus dem die Blut-Membranseite 8B des Dialysators 2 durchströmenden Blut der, das Signal $S_{DO}$ gemäß der Messung der chemischen und/oder physikalischen Größe des Dialysats über seine Konzentration korrelativ beeinflussende bzw. bestimmende, Blutanteilsstoff über die Filtermembran 8 in die auf der Dialysierflüssigkeits-Membranseite 8D des Dialysators 2 stehende Dialysierflüssigkeit über.

In dem ersten Interims-Zeitintervall L reichert sich der, das Signal $S_{DO}$ über seine Konzentration korrelativ beeinflussende bzw. bestimmende, Blutanteilsstoff in der stehenden ersten Dialysierflüssigkeitsmenge solange an, bis ein Diffusionsgleichgewicht auf der Blut-Membranseite 8B und der Dialysierflüssigkeits-Membranseite 8D (im Wesentlichen) erreicht ist. Da das Blut im extrakorporalen Blutkreis 20 kontinuierlich weiter strömt, entspricht die Diffusionsgleichgewichtskonzentration des das Signal $S_{DO}$ bestimmenden Blutanteilsstoffs in der ersten Dialysierflüssigkeitsmenge (spätestens) zum Ende des ersten Interims-Zeitintervalls L im Wesentlichen der im Blut des extrakorporalen Blutkreises 20 vorliegenden Konzentration des, das Signal $S_{DO}$ bestimmenden, Blutanteilsstoffs. Letztere entspricht zu diesem Zeitpunkt wiederum der im Blut des Patientenkörpers vorliegenden Konzentration des, das Signal $S_{DO}$ bestimmenden, Blutanteilsstoffs. Folglich entspricht dann die Diffusionsgleichgewichtskonzentration des, das Signal $S_{DO}$ bestimmenden, Blutanteilsstoffs in der stehenden ersten Dialysierflüssigkeitsmenge der im Blut des Patientenkörpers vorliegenden Konzentration des, das Signal $S_{DO}$ bestimmenden, Blutanteilsstoffs.

Hingegen in dem, dazu (signifikant) kürzeren bzw. "kurzen", zweiten Interims-Zeitintervall K wird eine zweite Dialysierflüssigkeitsmenge im Dialysator 2 nur solange eingesperrt, dass sich das Konzentrationsgleichgewicht des, das Signal $S_{DO}$ bestimmenden, Blutanteilsstoffs noch nicht einstellt.

Beide Vorgänge sind unabhängig voneinander, verlaufen bei ansonsten konstanten Bedingungen jedoch entlang der identischen Stofftransport-, bzw. Diffusionskinetik. Lediglich werden die Vorgänge zu unterschiedlichen Zeitpunkten entsprechend dem jeweiligen vorbestimmten Interims-Zeitintervall zum Zwecke der Messung an der zumindest einen Sensorvorrichtung 31, 32 abgebrochen.

Auch während des zweiten Interims-Zeitintervalls K wird das Blut nach wie vor von der Blutpumpe 42 durch den Dialysator 2 gefördert. Aufgrund der geschlossenen Sperrventile 9 und 10 ist das Dialysatvolumen 8D eingeschlossen. Dabei nimmt es allmählich die Konzentration der im Blut gelösten und permeablen Stoffe, wie z.B. Harnsäure, Kreatinin, Natrium, Kalium, etc., an. Das zweite Interims-Zeitintervall K als Dauer der "kurzen" Bypass-Schaltung ist dabei so gewählt, dass ein vollständiger Ausgleich zwischen der Blut-Membranseite 8B und der Dialysierflüssigkeits-Membranseite 8D nicht stattfindet. In Laborversuchen hat sich gezeigt, dass für das zweite Interims-Zeitintervall K als Dauer der "kurzen" Bypass-Schaltung kleiner(gleich) 1 Minute ausreichend ist. Vorzugsweise beträgt die Dauer 10 bis 20 Sekunden, insbesondere (ca.) 14 Sekunden.

Liegt eine Rezirkulation vor, so ist das in den Dialysator 2 eintretende Blut während des ("kurzen") zweiten Interims-Zeitintervalls K, d.h. während dieser Zeit vorab der Gleichgewichtseinstellung, von dem zurückfließenden venösen Blut beeinflusst und somit rezirkulationsbehaftet.

Demzufolge ist es wesentlich für die vorliegende Offenbarung (unabhängig von sonstigen technischen Merkmalen), dass die während des ("kurzen") zweiten Interims-Zeitintervalls K eingeschlossene zweite Dialysierflüssigkeitsmenge der Dialysierflüssigkeits-Membranseite 8D, demzufolge auch der zweite Dialysierflüssigkeitsbolus, bei (bzw. im Falle des) Vorhandenseins einer Rezirkulation, rezirkulationsbehaftet ist.

Dahingegen während des ("langen") ersten Interims-Zeitintervalls L passt sich, wie vorstehend geschildert, die eingeschlossene erste Dialysierflüssigkeitsmenge der Dialysierflüssigkeits-Membranseite 8D vollständig an das Blut an, welches ununterbrochen durch den Dialysator 2 gefördert wird. Dadurch, dass das arterielle Blut in seiner Zusammensetzung nicht mehr von der Dialysierflüssigkeit beeinflusst wird, führt das zurückfließende venöse Blut selbst bei Vorhandensein einer Rezirkulation (sprich, in dem Fall einer vorliegenden Rezirkulation, trotz dieser) zu keiner weiteren Veränderung des arteriellen Blutes.

Aufgrunddessen ist nach einer ausreichend langen Zeit im Bypass-Modus bzw. spätestens mit Ende des ersten Interims-Zeitintervalls L das Blut in der arteriellen Leitung 40 nicht mehr rezirkulationsbehaftet.

Demzufolge ist es wesentlich für die vorliegende Offenbarung (unabhängig von sonstigen technischen Merkmalen), dass die während des ("langen") ersten Interims-Zeitintervalls L eingeschlossene bzw. eingesperrte erste Dialysierflüssigkeitsmenge der Dialysierflüssigkeits-Membranseite 8D, demzufolge auch der erste Dialysierflüssigkeitsbolus, selbst bei (bzw. im Falle des) Vorhandenseins einer Rezirkulation, nicht rezirkulationsbehaftet ist.

Wurde das jeweilige vorbestimmte Interims-Zeitintervall für den Bypass-Modus M-I, nämlich zum einen für das erste Interims-Zeitintervall L sowie zum anderen für das zweite Interims-Zeitintervall K, erreicht, hebt die Steuer- und Recheneinheit 100 den Bypass-Modus M-I auf. Folglich schließt sie das Bypass-Sperrventil 11 in der Bypassleitung 22 und öffnet zeitgleich die Ventile 9, 10 in der Dialysierflüssigkeitszulaufleitung 14 und der Dialysierflüssigkeitsablaufleitung 16, um wiederum in den Grund-Modus M-0 zurückzukehren bzw. (zurück) zu schalten. Nun in dem Grund-Modus M-0 fließt wieder frische Dialysierflüssigkeit von der Dialysierflüssigkeitsquelle 3 durch die Dialysierflüssigkeits-Membranseite 8D des Dialysators 2.

Die jeweils vorher, d.h. in dem jeweiligen ersten bzw. zweiten Interims-Zeitintervall, auf der Dialysierflüssigkeits-Membranseite 8D gestandene bzw. eingesperrte erste bzw. zweite Dialysierflüssigkeitsmenge (bzw. das zugehörige Delta-Volumen) fließt folglich in Form eines jeweiligen ersten bzw. zweiten Dialysierflüssigkeitsbolus durch die Dialysierflüssigkeitsablaufleitung 16 in Richtung der Dialysierflüssigkeitssenke 7, wobei sie die erste Sensoreinrichtung 31 und die zweite Sensoreinrichtung 32 passiert. Diese misst, vgl. Figuren 2 bis 4, in Folge der Anreicherung des, das Signal $S_{DO}$ über seine Konzentration korrelativ beeinflussenden bzw. bestimmenden, Blutanteilsstoffs einen jeweils zugehörigen Signalausschlag bzw. eine Signalrespons $S_{DO} = f(t)$, bezeichnet als zugehöriges Bolus-Signal.

Nach Beendigung des zweiten Interims-Zeitintervalls K als Dauer der "kurzen" Bypass-Schaltung bzw. Nebenschluss-Schaltung im Bypass-Modus M-I wird wieder in den Grund-Modus M-0 bzw. die Hauptschluss-Schaltung geschaltet, d.h. die Sperrventile 9 und 10 öffnen und das Bypass-Sperrventil 11 schließt. Frische Dialysierflüssigkeit strömt in den Dialysator 2 und verdrängt die zuvor zumindest teilweise angesättigte zweite Dialysierflüssigkeitsmenge in Form des zweiten Dialysierflüssigkeitsbolus. Passiert der zweite Dialysierflüssigkeitsbolus die erste Sensorvorrichtung 31 und/oder die zweite Sensorvorrichtung 32, so ist dort ein kurzzeitiger Signalausschlag als das zweite Bolus-Signal zu sehen.

Wie anhand der Figuren 2 bis 4 weiter nachzuvollziehen, wird das jeweilig dem ersten bzw. zweiten Interims-Zeitintervall L, K zugehörige Bolus-Signal dabei in Form eines Peaks bzw. Extremums $S_{DO, ext, L}$, $S_{DO, ext, K}$ (bspw. Leitfähigkeits- und/oder Lichtabsorptionspeak stellvertretend für die Konzentration des Blutanteilsstoffs) und/oder in Form einer darunter verlaufenden gesamten Flächenintegrals $A_L$, $A_K$ bzw. teilweisen Flächenintegrals $A_{L, part}$, $A_{K, part}$ (schraffiert hinterlegte Bereiche) verzeichnet bzw. erfasst.

Wie anhand der Figuren 2 bis 4 weiter nachzuvollziehen, ist der Wert am Peak bzw. im Extremum des Signals als $S_{DO,ext,K}$ bzw. $S_{DO, ext, L}$, bezeichnet. Wie oben dargelegt, entspricht der Wert für den ersten Dialysierflüssigkeitsbolus im Extremum $S_{DO,ext,L}$ dabei dem blutseitigen Wert ohne Rezirkulation (d.h. bei ca. 0 % Rezirkulation).

Hingegen wird der eingependelte Wert kurz vor Beginn oder Ende der jeweiligen ersten bzw. zweiten Bypass-Schaltung im Bypass-Modus M-I als $S_{DO,pre,L}$ bzw. $S_{DO,pre,K}$ bezeichnet.

Handelt es sich bei dem betrachteten Signal um die Absorbanz (bspw. zweite Sensorvorrichtung 32 in Fig. 1), so ist als Extremum immer mit einem Maximum zu rechnen. Ist es dagegen die Leitfähigkeit (bspw. erste Sensorvorrichtung 31 in Fig. 1) oder Natriumkonzentration (oder die Konzentration eines beliebigen anderen Blutanteilsstoffes, der sowohl im Blut als auch in der Dialysierflüssigkeit vorkommt), kann auch ein Minimum auftreten.

Für den etwaig möglichen Fall, dass kein signifikanter Ausschlag zustande kommt, insofern Richtung und Höhe des Ausschlags von dem Gradienten zwischen Dialysat-Membranseite 8D und Blut-Membranseite 8B abhängen, kann die Dialysemaschine vorzugsweise (alternativ oder kumulativ zu weiteren technischen Merkmalen) eingerichtet sein, den Gradienten vor Durchführung der Rezirkulationsbestimmung bzw. des Messzyklus kurzzeitig zu erhöhen, indem die Zusammensetzung und somit die Leitfähigkeit der Dialysierflüssigkeit verändert wird. Um eine(n) unerwünschte(n) Natriumentzug bzw. -Zufuhr durch die Anpassung der Dialysierflüssigkeit möglichst gering zu halten, kann die Leitfähigkeit der Dialysierflüssigkeit anschließend wieder auf den ursprünglichen Wert eingestellt werden.

Registriert die Steuer- und Recheneinheit 100 eine (statistisch signifikante) Abweichung des, in Folge des zweiten Interims-Zeitintervalls K auftretenden, zweiten Bolus-Signals $S_{DO, ext, K}$; $A_K$; $A_{part, K}$ gegenüber einem, in Folge des ersten Interims-Zeitintervalls L auftretenden, ersten Bolus-Signal $S_{DO, ext, L}$; $A_L$; $A_{part, L}$, ermittelt diese das Vorhandensein einer Rezirkulation.

Figuren 2 bis 4 zeigen (unter Bezugnahme auf die schematische Darstellung der Fig. 1) einen jeweiligen zeitlichen Verlauf des Signals $S_{DO} = f(t)$ der Messaufnahme (insb. Leitfähigkeit der Dialysierflüssigkeit des für das Signal zu erfassenden Blutanteilsstoffs) an der zumindest einen Sensorvorrichtung 31, 32 (insb. an der ersten Sensorvorrichtung 31) einer Dialysemaschine 50. Dabei ist die zumindest eine Sensorvorrichtung 31, 32 in Flussrichtung vor bzw. stromauf (wie in Fig. 1 beispielhaft abgebildet) oder hinter bzw. stromab dem Sperrventil 10 der Dialysierflüssigkeitsablaufleitung 16 angeordnet. Ferner ist dabei die zumindest eine Sensorvorrichtung 31, 32 in Flussrichtung vor bzw. stromauf (wie in Fig. 1 beispielhaft abgebildet) oder hinter bzw. stromab der Mündungsstelle der Bypassleitung 22 in die Dialysierflüssigkeitsablaufleitung 16 angeordnet. Der zeitliche Versatz des Peaks bei einer Verschiebung der Sensorvorrichtung 31, 32 in Richtung stromab ist der Einfachheit halber als vernachlässigbar unberücksichtigt. Jedoch kann es aufgrund des vorstehenden Gesichtspunktes bevorzugt sein, dass die zumindest eine Sensorvorrichtung 31, 32 (insb. die erste Sensorvorrichtung 31, vorzugsweise als temperaturkompensierende Leitfähigkeitssonde) direkt am Dialysierflüssigkeitsablauf 6 (stromab) angeordnet ist, insbesondere an diesem angeflanscht.

In den Diagrammen der Figuren 2 bis 4 bezeichnen die zu der Zeitachse parallelen Doppelpfeile L bzw. K das erste Interims-Zeitintervall L sowie das zweite Interims-Zeitintervall K als die jeweiligen Zeiträume, in denen der Bypass-Modus M-I (nur in Fig. 2 bezeichnet, in Figuren 3, 4 analog) aktiv ist, also an der zumindest einen Sensorvorrichtung 31, 32 keine von der Dialysierflüssigkeits-Membranseite 8D her strömende Dialysierflüssigkeit vorbeifließt.

Vor und/oder während des Bypass-Modus M-I bzw. L, K misst die zumindest eine Sensorvorrichtung 31, 32 in der Dialysierflüssigkeit folglich für den (spezifischen) Blutanteilsstoff ein (eingependeltes) konstantes Signal als das Grund-Signal $S_{DO, pre}$. Dabei ergibt sich die (weitgehende) Konstanz aus dem Umstand, dass die erste bzw. zweite Dialysierflüssigkeitsmenge (bzw. das zugehörige erste bzw. zweite Delta-Volumen), in welcher (bzw. in welchem) sich infolge von Diffusion der, das Signal $S_{DO}$ über seine Konzentration korrelativ beeinflussende bzw. bestimmende, Blutanteilsstoff anreichert, zwischen den Sperrventilen 9, 10 eingesperrt ist und frische Dialysierflüssigkeit von der Dialysierflüssigkeitsquelle 3 die Sensorvorrichtung nicht umströmt. Das erfasste Grund-Signal $S_{DO, pre}$ kann als korrelierend zu der Konzentration des, das Signal $S_{DO}$ über seine Konzentration korrelativ beeinflussenden bzw. bestimmenden, Blutanteilsstoffs am Dialysatorausgang unter den normalen, bekannten Behandlungs-/Betriebsbedingungen, d.h. im Grund-Modus M-0, angesehen werden.

Der Wert $S_{DO,pre,L}$ entspricht dem eingependelten Wert, d.h. dem Grund-Signal $S_{DO, pre}$, kurz vor Beginn oder Ende des ersten Interims-Zeitintervalls L, insbesondere vor Beginn oder Ende der länger andauernden Bypass-Schaltung. Er kann auch dem eingependelten Wert, d.h. dem Grund-Signal $S_{DO, pre}$, kurz vor Beginn oder Ende des zweiten Interims-Zeitintervalls K, insbesondere vor Beginn oder Ende der kürzer andauernden Bypass-Schaltung, bezeichnet als $S_{DO,pre,K}$, entsprechen oder umgekehrt.

Nach Beendigung des Bypass-Modus M-I bzw. L, K misst die zumindest eine Sensorvorrichtung 31, 32 einen deutlichen jeweiligen zweiten Peak im zeitlichen Verlauf des Signals $S_{DO} = f(t)$, da nach Öffnen des Sperrventils 10 die vorher jeweils eingesperrte erste bzw. zweite Dialysierflüssigkeitsmenge nun an der zumindest einen Sensorvorrichtung 31, 32 als der erste bzw. zweite Dialysierflüssigkeitsbolus vorbeiströmt.

Hat der erste bzw. zweite Dialysierflüssigkeitsbolus die Sensorvorrichtung 31, 32 vollständig passiert, entspricht das Signal wieder dem Grund-Signal $S_{DO, pre}$.

Die Steuer- und Recheneinheit (mit Bezugszeichen 100 in Fig. 1) berechnet zum einen eine zweite Differenz, bezeichnet als $D_K$ (Figuren 2, 3), aus dem maximalen Peak des zweiten Bolus-Signals $S_{DO, ext, K}$ und dem Grund-Signal $S_{DO, pre}$ sowie zum anderen eine erste Differenz, bezeichnet als $D_L$ (Figuren 2, 3), aus dem maximalen Peak des ersten Bolus-Signals $S_{DO, ext, L}$ und dem Grund-Signal $S_{DO, pre}$. Dies beschreiben die folgenden zwei Gleichungen:

$$D_L = S_{DO,ext,L} - S_{DO,pre,L}$$

$$D_K = S_{DO,ext,K} - S_{DO,pre,K}$$

Weichen die zweite Differenz $D_K$ und die erste Differenz $D_L$ voneinander ab, bestimmt die Steuer- und Recheneinheit (mit Bezugszeichen 100 in Fig. 1) demzufolge das Vorhandensein der Rezirkulation bzw. leitet daraus diese qualitative Schlussfolgerung ab bzw. detektiert die Rezirkulation als solche.

Bevorzugt kann die Steuer- und Recheneinheit 100 auf Grundlage des vorbekannten und von der Speichereinheit 110 (Fig. 1) abrufbaren Berechnungsmodells einen aktuellen Rezirkulationswert R (in %) quantitativ bestimmen. Dazu können, alternativ oder kumulativ, drei unterschiedliche Quotienten herangezogen werden:
Das Verhältnis der zweiten Differenz zu der ersten Differenz bezeichnet der Delta-Quotient $V_D$:

$$V_D = \frac{D_K}{D_L}$$

Somit kann der aktuelle Rezirkulationswert R als Funktion dieses Verhältnisses bzw. Delta-Quotienten $V_D$ bestimmt werden:

$$R = f(V_D)$$

Alternativ oder kumulativ zur Auswertung der Signaldifferenzen können auch die jeweiligen Gesamtflächen (schraffierte Bereiche) unterhalb der beiden Signalausschläge und $A_L$ und/oder jeweilige Teilflächen (bedeutet durch gestrichelte vertikale Trennungslinie) unterhalb der beiden Signalausschläge $A_{part, K}$ und $A_{part, L}$ ins Verhältnis gebracht werden:

$$V_A = \frac{A_K}{A_L}$$

Gesamtflächenintegral-Quotient
Bzw.

$$V_{A,part} = \frac{A_{part,K}}{A_{part,L}}$$

Teilflächenintegral-Quotient

Auf diese Weise kann die Rezirkulation auch als Funktion der Flächenverhältnisse in Weise des Gesamtflächenintegral-Quotienten bzw. Teilflächenintegral-Quotienten bestimmt werden:

$$R = f(V_A)$$

$$R = f(V_{A,part})$$

Wie der Vergleich der Figuren 2 und 3 veranschaulicht, hat sich gezeigt, dass die Höhe des Peaks bzw. die flächige Ausprägung des zweiten Bolus-Signals $S_{DO,ext,K}$ bezogen auf die Höhe des Peaks bzw. auf die flächige Ausprägung des ersten Bolus-Signals $S_{DO,ext,L}$ mit zunehmender Rezirkulation abnimmt.

Mit anderen Worten, nimmt ein jeweiliger der vorstehenden Quotienten bei Zunahme der Rezirkulation bzw. des aktuellen Rezirkulationswerts R ab. Sprich, dies bedeutet eine indirekte Korrelation.

Somit kann unter Einbeziehung zumindest eines der vorstehenden Quotienten, also des Delta-Quotienten $V_D$ und/oder des Gesamtflächenintegral-Quotienten $V_A$ und/oder des Teilflächenintegral-Quotienten $V_{A,part}$, ein aktueller Rezirkulationswert R anhand des (vorab bestimmten) Berechnungsmodells quantitativ bestimmt bzw. berechnet werden.

So zeigt Fig. 2 exemplarisch den Fall von 0% für den aktuellen Rezirkulationswert R; und zeigt Fig. 3 den Fall von 25% für den aktuellen Rezirkulationswert R. Aus dem Vergleich der ansonsten vergleichbaren Situationen bzw. Darstellungen ist zu erkennen, dass der Signalausschlag/Peak $S_{DO,ext,K}$ bzw. das Flächenintegral unterhalb des Signalausschlags nach Beendigung der "kurzen" Bypass-Schaltung bzw. des zweiten Interims-Zeitintervalls K in Fig. 3 im Vergleich zum entsprechenden Fall in Fig. 2 kleiner ausfällt bzw. verringert ist (in Figur 3 anhand des nach unten zeigenden Symbolpfeils oberhalb des zweiten Bolus-Signals $S_{DO,ext,K}$ angezeigt).

In der ansonsten zur Figur 2 analogen Figur 4 ist veranschaulicht, dass die Reihenfolge der jeweiligen Interims-Zeitintervalle, in denen der Bypass-Modus M-I (nur in Fig. 2 bezeichnet, in Figur 4 analog) aktiv ist, auch umgekehrt gewählt sein kann, so dass zuerst eine "lange" Bypass-Schaltung durchgeführt wird, gefolgt von einer "kurzen", entsprechend dem ersten Interims-Zeitintervall L gefolgt von dem zweiten Interims-Zeitintervall K. Dies kann insbesondere zu Beginn einer extrakorporalen Blutbehandlung vorteilhaft sein. Insofern ist beim Anlegen des Patienten der Bypass nämlich ohnehin aktiviert, so dass diese Zeitspanne bereits genutzt werden kann, um das dialysatseitige Volumen 8D vollständig bis zur Gleichgewichtseinstellung, d.h. in dem ersten Zeitintervall L, anzusättigen. Sobald das erste Bolus-Signal am Dialysatausgang nach Umschaltung in den Grund-Modus M-0 (bzw. in den Hauptschluss) bestimmt werden konnte und das Signal sich anschließend zu dem Grund-Signal $S_{DO,pre}$ eingependelt hat, kann die "kurze" Bypass-Schaltung über das zweite Zeitintervall K hinweg durchgeführt werden.

Wie bereits erwähnt, ist zur Durchführung des Verfahrens zur Überwachung der offenbarungsgemäßen Dialysemaschine 50 zumindest eine Sensorvorrichtung, insbesondere die erste Sensorvorrichtung 31 und/oder die zweite Sensorvorrichtung 32, vorgesehen. Falls beide Sensorvorrichtungen 31, 32 gleichzeitig eingesetzt werden sollten, kann ein Plausibilitätscheck der ermittelten Rezirkulation erfolgen, indem die Rezirkulation, die mittels der ersten Sensorvorrichtung 31 bestimmt, vorzugsweise als aktueller Rezirkulationswert R berechnet, wurde, und die Rezirkulation, die mittels der zweiten Sensorvorrichtung 32 bestimmt, vorzugsweise als aktueller Rezirkulationswert R berechnet, wurde, miteinander verglichen werden. Weichen beide aktuellen Rezirkulationswerte R, bspw. um mehr als 10 Prozentpunkte (Differenz) oder um mehr als 1 Sigma, voneinander ab, kann die ermittelte Rezirkulation verworfen und ein erneuter Messzyklus auf Basis des ersten und des zweiten Interims-Zeitintervalls L, K gestartet werden. Andererseits kann es auch bevorzugt sein, bei einer Messung für den ersten aktuellen Rezirkulationswert zu 15 % und bei einer Messung für den zweiten aktuellen Rezirkulationswert Wert zu 12 % diese Messungen nicht verwerfen, sondern das Erreichen einer relevanten Rezirkulation zu ermitteln. Bspw. kann dann eine Ampelfarbe "gelb" eine beginnend kritische Rezirkulation über eine (interaktive) Anwenderschnittstelle ausgewiesen werden.

Alternativ können diese beide aktuellen Rezirkulationswerte R der ersten und der zweiten Sensorvorrichtungen 31, 32 gemittelt werden.

Darüber hinaus ist es denkbar, die zugrundeliegende zumindest eine Sensorvorrichtung, d.h. die erste Sensorvorrichtung 31 oder die zweite Sensorvorrichtung 32, in Abhängigkeit des der chemischen und/oder physikalischen Größe zugehörigen Gradienten zwischen dem Dialysierflüssigkeitseingang (d.h. dem Dialysierflüssigkeitszulauf 4 und/oder der Dialysierflüssigkeitszulaufleitung 14) und dem Dialysatausgang (d.h. dem Dialysierflüssigkeitsablauf 6 und/oder der Dialysierflüssigkeitsablaufleitung 16) zu wählen. Messen bspw. die dritte Sensorvorrichtung 33 für den Dialysierflüssigkeitseingang und die erste Sensorvorrichtung 31 für den Dialysierflüssigkeitsausgang in etwa denselben Leitfähigkeitswert, so ist der zugehörige Gradient nahezu (bzw. quasi) null, so dass keine signifikanten Signalausschläge zu detektieren wären. Insbesondere in diesem Fall einer geminderten Sonde, jedoch nicht limitierend, kann es bevorzugt sein, eine Rezirkulationsbestimmung anhand der zweiten Sensorvorrichtung 32 (bspw. per optischer Messung) zu bevorzugen bzw. zu priorisieren.

Selbstverständlich ist es auch möglich, die jeweiligen Signale beider Sensorvorrichtungen, also der ersten Sensorvorrichtung 31 bzw. der der zweiten Sensorvorrichtung 32, von vorneherein einzeln in eine Gleichung bzw. in ein beide Signale berücksichtigendes Berechnungsmodell zur Rezirkulationsbestimmung einfließen zu lassen.

**Liste der Bezugszeichen**

| | |
|---|---|
| 1 | Patient |
| 2 | Dialysator |
| 3 | Dialysierflüssigkeitsquelle |
| 4 | Dialysierflüssigkeitszulauf |
| 6 | Dialysierflüssigkeitsablauf |
| 7 | Dialysierflüssigkeitssenke |
| 8 | Filtermembran |
| 8B | Blut-Membranseite |
| 8D | Dialysierflüssigkeits-Membranseite |
| 9 | Sperrventil |
| 10 | Sperrventil |
| 11 | Bypass-Sperrventil |
| 14 | Dialysierflüssigkeitszulaufleitung |
| 16 | Dialysierflüssigkeitsablaufleitung |
| 20 | Blutkreis |
| 22 | Bypassleitung |
| 30 | Dialysierflüssigkeitskreis |
| 31 | (erste) Sensorvorrichtung |
| 32 | zweite Sensorvorrichtung |
| 33 | dritte Sensorvorrichtung |
| 40 | arterielle Blutleitung |
| 42 | Blutpumpe |
| 44 | venöse Blutleitung |
| 50 | extrakorporale Blutbehandlungsmaschine |
| 100 | Steuer- und Recheneinheit |
| 110 | Speichereinheit |
| 200 | Modulvorrichtung |
| A | gesamtes Flächenintegral |
| $A_{part}$ | teilweises Flächenintegral |
| $D_L$ | erste Differenz |
| $D_K$ | zweite Differenz |
| K | zweites Interims-Zeitintervall |
| L | erstes Interims-Zeitintervall |
| $S_{DO}$ | Signal (Dialysierflüssigkeit, Out) |
| M-I | Interims-Modus (insb. Bypass-Modus) |
| M-0 | Grund-Modus (kontinuierlicher Dialysebetrieb) |

**Patentansprüche**

1. Extrakorporale Blutbehandlungsmaschine (50), insbesondere Dialysemaschine, mit einem Dialysator (2) sowie zumindest einer Sensorvorrichtung (31, 32), die auf einer Dialysierflüssigkeitsseite (30) dem Dialysator (2) nachgeschaltet und mit einer Steuer- und Recheneinheit (100) elektrisch verbunden ist,

wobei die Steuer- und Recheneinheit (100) zur Bestimmung eines Vorhandenseins einer Rezirkulation ohne blutseitige Bolusgabe dafür vorgesehen und ausgebildet ist:

- das von der zumindest einen Sensorvorrichtung (31, 32) zur quantitativen Bestimmung zumindest eines vorzugsweise ausgewählten oder auswählbaren Blutanteilsstoffs in der verbrauchten Dialysierflüssigkeit gemessene zumindest eine Signal ($S_{DO}$) einer physikalischen und/oder chemischen Größe der verbrauchten Dialysierflüssigkeit in seinem zeitlichen Verlauf zu erfassen;
- die extrakorporale Blutbehandlungsmaschine in einem Grund-Modus (M-0) zum kontinuierlichen Betrieb, in welchem die Dialysierflüssigkeitsströmung durch den Dialysator (2) zugelassen ist, zu betreiben,
- das der in dem Grund-Modus (M-0) verbrauchten Dialysierflüssigkeit zugehörige Signal als ein Grund-Signal ($S_{DO, pre, L}$, $S_{DO, pre, K}$) zu messen;
- die extrakorporale Blutbehandlungsmaschine von dem Grund-Modus (M-0) in einen Interims-Modus (M-I) zum Interims-Betrieb, in welchem eine Dialysierflüssigkeitsmenge auf der Dialysierflüssigkeits-Membranseite (8D) im Dialysator (2) eingesperrt wird, während der Blutstrom im extrakorporalen Blutkreis mit einer festgelegten Blutflussrate betrieben wird, wobei das Blut auf der Blut-Membranseite (8B) weiter strömt, für die jeweilige Dauer eines vorbestimmten und/oder vorbestimmbaren Interims-Zeitintervalls (L, K) zu schalten, insbesondere in einen Bypass-Modus als den Interims-Modus (M-I) zu schalten; nachfolgend
- in den Grund-Modus (M-0) zu wechseln, um die zuvor eingesperrte Dialysierflüssigkeitsmenge als einen jeweiligen Dialysierflüssigkeitsbolus der zumindest einen Sensorvorrichtung (31, 32) zuzuführen, um eine dem Dialysierflüssigkeitsbolus zugehörige Signaländerung gegenüber dem Grund-Signal ($S_{DO, pre, L}$, $S_{DO, pre, K}$) als ein zugehöriges Bolus-Signal ($S_{DO, ext, L}$, $S_{DO, ext, K}$; A; $A_{part}$) zu messen;
- den Interims-Modus (M-I) für ein erstes Interims-Zeitintervall (L) zur Gleichgewichtseinstellung des Stofftransports zu schalten, wobei eine erste Dialysierflüssigkeitsmenge im Dialysator (2) zumindest solange eingesperrt wird, bis sich auf der Dialysierflüssigkeits-Membranseite (8D) und der Blut-Membranseite (8B) des Dialysators (2) ein sich nicht mehr oder nur noch unwesentlich veränderndes Konzentrationsgleichgewicht des Blutanteilsstoffs einstellt,

**dadurch gekennzeichnet, dass**

die Steuer- und Recheneinheit (100) ferner zur Ausbildung eines Messzyklus dafür vorgesehen und ausgebildet ist:

- den Interims-Modus (M-I) für ein, gegenüber dem ersten Interims-Zeitintervall (L) kürzeres, zweites Interims-Zeitintervall (K) zu schalten, wobei eine zweite Dialysierflüssigkeitsmenge im Dialysator (2) nur solange eingesperrt wird, dass sich das Konzentrationsgleichgewicht des Blutanteilsstoffs noch nicht einstellt,
- aus einer Abweichung eines zweiten Bolus-Signals ($S_{DO, ext, K}$; $A_K$; $A_{part, K}$) in Folge des zweiten Interims-Zeitintervalls (K) gegenüber einem ersten Bolus-Signal ($S_{DO, ext, L}$; $A_L$; $A_{part, L}$) in Folge des ersten Interims-Zeitintervalls (L) das Vorhandensein der Rezirkulation zu ermitteln.

2. Extrakorporale Blutbehandlungsmaschine (50) nach Anspruch 1, **dadurch gekennzeichnet, dass**

- das erste Bolus-Signal ($S_{DO, ext, L}$; $A_L$; $A_{part, L}$) und zweite Bolus-Signal ($S_{DO, ext, K}$; $A_K$; $A_{part, K}$) jeweils einem maximalen Peak ($S_{DO, ext, L}$, $S_{DO, ext, K}$) im Signal der zumindest einen Sensorvorrichtung (31, 32) unmittelbar nach der Freigabe des jeweiligen ersten bzw. zweiten Dialysierflüssigkeitsbolus aus dem Dialysator (2) entspricht;
- die Steuer- und Recheneinheit (100) ferner dafür vorgesehen und ausgebildet ist:

- einen Delta-Quotienten ($V_D$) aus einer zweiten Differenz ($D_K$), berechnet aus dem maximalen Peak des zweiten Bolus-Signals ($S_{DO, ext, K}$) und dem Grund-Signal ($S_{DO, pre}$), bezogen auf eine erste Differenz ($D_L$), berechnet aus dem maximalen Peak des ersten Bolus-Signals ($S_{DO, ext, L}$) und dem Grund-Signal ($S_{DO, pre}$), zu ermitteln; und
- auf Grundlage des ermittelten Delta-Quotienten ($V_D$) sowie eines vorbekannten Berechnungsmodells einen aktuellen Rezirkulationswert (R) zu bestimmen.

3. Extrakorporale Blutbehandlungsmaschine (50) nach Anspruch 1 oder 2, **dadurch gekennzeichnet, dass**

- das erste Bolus-Signal ($S_{DO, ext, L}$; $A_L$; $A_{part, L}$) und zweite Bolus-Signal ($S_{DO, ext, K}$; $A_K$; $A_{part, K}$), ggf. zusätzlich, jeweils einem, um das Grund-Signal ($S_{DO, pre}$) bereinigten, gesamten Flächenintegral ($A_L$, $A_K$) im Signal der zumindest einen Sensorvorrichtung (31, 32) unmittelbar nach der Freigabe des jeweiligen ersten bzw. zweiten Dialysierflüssigkeitsbolus aus dem Dialysator (2) entspricht;
- die Steuer- und Recheneinheit (100) ferner dafür vorgesehen und ausgebildet ist:

- einen Gesamtflächenintegral-Quotienten ($V_A$) aus dem zweiten gesamten Flächenintegral ($A_K$) bezogen auf das erste gesamte Flächenintegral ($A_L$) zu ermitteln; und
- auf Grundlage des Gesamtflächenintegral-Quotienten ($V_A$) sowie eines vorbekannten Berechnungsmodells einen aktuellen Rezirkulationswert (R) zu bestimmen.

4. Extrakorporale Blutbehandlungsmaschine (50) nach einem der vorstehenden Ansprüche, **dadurch gekennzeichnet, dass**

- das erste Bolus-Signal ($S_{DO, ext, L}$; $A_L$; $A_{part, L}$) und zweite Bolus-Signal ($S_{DO, ext, K}$; $A_K$; $A_{part, K}$), ggf. zusätzlich, jeweils einem, um das Grund-Signal ($S_{DO, pre}$) bereinigten, teilweisen Flächenintegral ($A_{part, L}$, $A_{part, K}$) im Signal der zumindest einen Sensorvorrichtung (31, 32) unmittelbar nach der Freigabe des jeweiligen ersten bzw. zweiten Dialysierflüssigkeitsbolus aus dem Dialysator (2) entspricht;
- die Steuer- und Recheneinheit (100) ferner dafür vorgesehen und ausgebildet ist:

- einen Teilflächenintegral-Quotienten ($V_{A, part}$) aus dem zweiten teilweisen Flächenintegral ($A_{part, K}$) bezogen auf das erste teilweise Flächenintegral ($A_{part, L}$) zu ermitteln; und
- auf Grundlage des Teilflächenintegral-Quotienten ($V_{A, part}$) sowie eines vorbekannten Berechnungsmodells einen aktuellen Rezirkulationswert (R) zu bestimmen.

5. Extrakorporale Blutbehandlungsmaschine (50) nach einem der Ansprüche 2 bis 4, **dadurch gekennzeichnet, dass** das Berechnungsmodell zur Berechnung des aktuellen Rezirkulationswerts (R) eine Dialysierflüssigkeitsflussrate ($Q_D$) des Dialysierflüssigkeitskreises (30), eine Blutflussrate ($Q_B$) im extrakorporalen Blutschlauchsystem des Blutkreises (20) und eine theoretische Clearance des Dialysators (2) berücksichtigt, insbesondere weiter noch zumindest einen aus einer Anzahl weiterer Maschinen-, Dialysator- und/oder Einstellungsparameter der Blutbehandlungsmaschine (50) als Einflußgröße berücksichtigt.

6. Extrakorporale Blutbehandlungsmaschine (50) nach einem der vorstehenden Ansprüche, **dadurch gekennzeichnet, dass** die auf einer Dialysierflüssigkeitsseite (30) dem Dialysator (2) nachgeschaltete zumindest eine erste Sensorvorrichtung (31) und/oder gegebenenfalls eine weitere dem Dialysator (2) nachgeschaltete, auf eine andere physikalische und/oder chemische Größe gerichtete, zweite Sensorvorrichtung (32):

- eingerichtet ist, als das zumindest eine Signal ($S_{DO}$) insbesondere eine Leitfähigkeit der verbrauchten Dialysierflüssigkeit in seinem zeitlichen Verlauf zu erfassen, weiter bevorzugt als eine temperaturkompensierende Leitfähigkeitssonde ausgebildet ist; und/oder
- eingerichtet ist, als das zumindest eine Signal ($S_{DO}$) eine Absorptionseigenschaft, insbesondere die Absorbanz und/oder den Transmissionsgrad, der verbrauchten Dialysierflüssigkeit in seinem zeitlichen Verlauf zu erfassen, weiter bevorzugt in einem Wellenlängenbereich zwischen 200 nm und 320 nm; und/oder
- in der Dialysierflüssigkeitsablaufleitung (16) direkt an dem Dialysierflüssigkeitsablauf (6) angeordnet ist.

7. Extrakorporale Blutbehandlungsmaschine (50) nach dem direkt vorstehenden Anspruch, **dadurch gekennzeichnet, dass** die Steuer- und Recheneinheit (100) ferner dafür vorgesehen und ausgebildet ist:

- einen ersten aktuellen Rezirkulationswert (R) auf Basis des ersten Signals ($S_{DO}$) der ersten Sensorvorrichtung (31) und einen zweiten aktuellen Rezirkulationswert (R) auf Basis des zweiten Signals ($S_{DO}$) der zweiten Sensorvorrichtung (32) zu bestimmen; und daraufhin auf Grundlage des Vergleichs der ersten und zweiten aktuellen Rezirkulationswerte (R):
- vorzugsweise daraus einen mittleren aktuellen Rezirkulationswert (R) zu mitteln; oder
- im Falle einer, insbesondere statistisch signifikanten, Abweichung, insb. größer 10 Prozentpunkten, voneinander, das Ergebnis zu verwerfen und einen erneuten Messzyklus automatisch zu initiieren; oder
- auf Grundlage einer Vorentscheidung, ob der entsprechende erste bzw. zweite aktuelle Rezirkulationswert (R) in einen vorbestimmten Relevanzbereich fallen, das Ergebnis nicht verwerfen.

8. Extrakorporale Blutbehandlungsmaschine (50) nach einem der vorstehenden Ansprüche, **dadurch gekennzeich-**

**net, dass** weiterhin zumindest eine auf einer Dialysierflüssigkeitsseite (30) dem Dialysator (2) vorgeschaltete dritte Sensorvorrichtung (33) vorgesehen ist, welche:

- eingerichtet ist, als das zumindest eine Signal ($S_{DO}$) insbesondere eine Leitfähigkeit der unverbrauchten Dialysierflüssigkeit in seinem zeitlichen Verlauf zu erfassen, weiter bevorzugt als eine temperaturkompensierende Leitfähigkeitssonde ausgebildet ist; und/oder
- eingerichtet ist, als das zumindest eine Signal ($S_{DO}$) eine Absorptionseigenschaft, insbesondere die Absorbanz und/oder den Transmissionsgrad, der unverbrauchten Dialysierflüssigkeit in seinem zeitlichen Verlauf zu erfassen, weiter bevorzugt in einem Wellenlängenbereich zwischen 200 nm und 320 nm; und/oder
- in der Dialysierflüssigkeitszulaufleitung (14) direkt an dem Dialysierflüssigkeitszulauf (4) angeordnet ist; und/oder
- stromauf der Bypassleitung (22) angeordnet ist.

9. Extrakorporale Blutbehandlungsmaschine (50) nach dem direkt vorstehenden Anspruch 8, **dadurch gekennzeichnet, dass**

- die dem Dialysator (2) vorgeschaltete dritte Sensorvorrichtung (33) und die dem Dialysator (2) nachgeschaltete erste Sensorvorrichtung (31) auf Basis eines gleichen Messprinzips eingerichtet sind, um eine gleiche physikalische und/oder chemische Größe, insbesondere eine Leitfähigkeit, als das jeweilig zugehörige dritte Signal ($S_{DO}$) der dritten Sensorvorrichtung (33) sowie das erste Signal ($S_{DO}$) der ersten Sensorvorrichtung (31) zu erfassen; und
- die dem Dialysator (2) nachgeschaltete zweite Sensorvorrichtung (32) auf Basis eines demgegenüber anderen Messprinzip eingerichtet ist, um eine demgegenüber andere physikalische und/oder chemische Größe als das zugehörige zweite Signal ($S_{DO}$) der zweiten Sensorvorrichtung (32) zu erfassen;

die Steuer- und Recheneinheit (100) ferner dafür vorgesehen und ausgebildet ist:

- im Falle einer, für den Grund-Modus (M-0) im Wesentlichen bestimmbaren, Übereinstimmung des dritten Signals ($S_{DO}$) und des ersten Signals ($S_{DO}$) das zweite Signal ($S_{DO}$) der zweiten Sensorvorrichtung (32) priorisierend für den Messzyklus heranzuziehen, um das Vorhandenseins einer Rezirkulation zu ermitteln, insbesondere den aktuellen Rezirkulationswert (R) zu bestimmen.

10. Extrakorporale Blutbehandlungsmaschine (50) nach einem der vorstehenden Ansprüche, **dadurch gekennzeichnet, dass**

- der Dialysator (2) einen Dialysierflüssigkeitszulauf (4) für frische Dialysierflüssigkeit, einen Dialysierflüssigkeitsablauf (6) für verbrauchte Dialysierflüssigkeit und eine Filtermembran (8) aufweist, die eine Dialysierflüssigkeits-Membranseite (8D), auf der der Dialysator (2) über eine Dialysierflüssigkeitszulaufleitung (14) und eine Dialysierflüssigkeitsablaufleitung (16) an einen Dialysierflüssigkeitskreis (30) angeschlossen ist, von einer Blut-Membranseite (8B) trennt, auf der der Dialysator (2) an einen extrakorporaler Blutkreis (20) angeschlossen oder anschließbar ist;
- eine Bypassleitung (22) vorgesehen ist, mittels derer die Dialysierflüssigkeits-Membranseite (8D) wahlweise in einem Bypass-Modus als dem Interims-Modus (M-I) zum Interims-Betrieb für das erste Interims-Zeitintervall (L) sowie für das zweite Interims-Zeitintervall (K) überbrückbar ist, um im Dialysator (2) befindliche Dialysierflüssigkeit temporär einzusperren, wofür an der Dialysierflüssigkeitszulaufleitung (14) und der Dialysierflüssigkeitsablaufleitung (16) wenigstens jeweils ein mit der Steuer- und Recheneinheit (100) elektrisch verbundenes Sperrventil (9, 10) zwischen der Bypassleitung (22) und dem Dialysator (2) angeordnet ist;
- die Steuer- und Recheneinheit (100) zur Steuerung der extrakorporalen Blutbehandlungsmaschine (50) mit einer Speichereinheit (110) ausgebildet oder ausrüstbar ist und/oder über eine Schnittstelle mit einer hinsichtlich der Steuer- und Recheneinheit (100) externen Speichereinheit elektrisch verbunden oder verbindbar ist,

wobei die extrakorporale Blutbehandlungsmaschine (50) weiter aufweist:

- einen auf der Speichereinheit (110) abgelegten oder ablegbaren Datensatz, der eine Anzahl von für unterschiedliche Parameter der Blutbehandlungsmaschine (50) geeignete Blutflussraten ($Q_B$) im extrakorporalen Blutkreis (20) und zugehörige, vorzugsweise analytisch vorab bestimmte und/oder pauschal gesetzte, erste Interims-Zeitintervalle (L) angibt, innerhalb welcher im Dialysator (2) bei entsprechend eingestellter Blutflussrate ($Q_B$) unter der Annahme eines maximal möglichen Rezirkulationswerts (R) ein Konzentrationsausgleich des zumindest einen ausgewählten oder auswählbaren Blutanteilsstoffs zwischen Blut im extrakorporalen Blutkreis (20) und im Dialysator (2) eingesperrter Dialysierflüssigkeit ausschließlich infolge von Diffusion beendet ist;

so dass die Steuer- und Recheneinheit (100) zur Bestimmung des ersten Bolus-Signals ($S_{DO,\,ext,\,L}$; $A_L$; $A_{part,\,L}$) bzw. des zweiten Bolus-Signals ($S_{DO,\,ext,\,K}$; $A_K$; $A_{part,\,K}$) die extrakorporale Blutbehandlungsmaschine (50) jeweils für die Dauer des im Datensatz angegebenen ersten Interims-Zeitintervalls (L) bzw. des, gegenüber dem ersten Interims-Zeitintervall (L) kürzeren, zweiten Interims-Zeitintervalls (K) in den Bypass-Modus schaltet und den extrakorporalen Blutkreis (20), vorzugsweise gleichzeitig, bei der angegebenen Blutflussrate ($Q_B$) betreibt und unmittelbar nach jeweiliger Beendigung des Bypass-Modus für den jeweilig durch den Bypass-Modus entstandenen ersten bzw. zweiten Dialysierflüssigkeitsbolus in der aus dem Dialysator (2) ablaufenden, verbrauchten Dialysierflüssigkeit mithilfe der zumindest einen Sensorvorrichtung (31, 32) die dem jeweiligen ersten bzw. zweiten Dialysierflüssigkeitsbolus zugehörige Signaländerung gegenüber dem Grund-Signal ($S_{DO,\,pre}$) als ein zugehöriges erstes Bolus-Signal ($S_{DO,\,ext,\,L}$; $A_L$; $A_{part,\,L}$) bzw. zweites Bolus-Signal ($S_{DO,\,ext,\,K}$; $A_K$; $A_{part,\,K}$) messtechnisch erfasst,

- vorzugsweise das auf der Speichereinheit (110) abgelegte Berechnungsmodell, anhand welchem die Steuer- und Recheneinheit (100) unter Berücksichtigung des ersten Bolus-Signals ($S_{DO,\,ext,\,L}$; $A_L$; $A_{part,\,L}$), des zweiten Bolus-Signals ($S_{DO,\,ext,\,K}$; $A_K$; $A_{part,\,K}$) sowie des Grund-Signals ($S_{DO,\,pre}$), vorzugsweise vor dem Start oder zu Beginn eines Behandlungszyklus mittels der extrakorporalen Blutbehandlungsmaschine (50), optional einen aktuellen Rezirkulationswert (R) berechnet.

11. Extrakorporale Blutbehandlungsmaschine (50) nach einem der vorstehenden Ansprüche, **dadurch gekennzeichnet, dass** die Speichereinheit (32) in der extrakorporalen Blutbehandlungsmaschine (1) fest integriert ist.

12. Extrakorporale Blutbehandlungsmaschine (50) nach einem der vorstehenden Ansprüche, **dadurch gekennzeichnet, dass** die extrakorporale Blutbehandlungsmaschine (1) weiter eine, kabellos oder kabelgebunden verbundene, Anzeigevorrichtung aufweist, welche zur Anzeige des ermittelten Vorhandenseins einer Rezirkulation, insbesondere des bestimmten aktuellen Rezirkulationswertes (R), eingerichtet ist.

13. Extrakorporale Blutbehandlungsmaschine (50) nach einem der vorstehenden Ansprüche, **dadurch gekennzeichnet, dass** die zumindest eine Sensorvorrichtung (31, 32) und die Steuer- und Recheneinheit (100) in bzw. an einer separaten, zu der extrakorporalen Blutbehandlungsmaschine (50) nachrüstbaren und/oder nachgerüsteten, Modulvorrichtung (200) zur Rezirkulationsbestimmung ausgelagert vorgesehen sind, wobei die Modulvorrichtung (200) aufweist:

- eine Sensoreinheit mit:

  - der zumindest einen Sensorvorrichtung (31, 32),
  - einem Anschlussabschnitt oder -teil zum vorzugsweise adaptiven Anschließen der Sensoreinheit an den Fluidkreis der extrakorporalen Blutbehandlungsmaschine (50), derart, dass die zumindest eine Sensorvorrichtung (31, 32) auf der Dialysierflüssigkeitsseite (30) dem Dialysator (2) nachgeschaltet und zum Erfassen des zumindest einen Signals ($S_{DO}$) einer physikalischen und/oder chemischen Größe der verbrauchten Dialysierflüssigkeit in seinem zeitlichen Verlauf eingerichtet ist; und

  - eine als die Steuer- und Recheneinheit (100) ausgebildete, unabhängig von der extrakorporalen Blutbehandlungsmaschine (50) arbeitende Elektronik- und Auswerteeinheit, die das Vorhandensein der Rezirkulation ermittelt, insbesondere den aktuellen Rezirkulationswert (R) bestimmt; ferner optional:
  - eine, vorzugsweise kombinierte, Eingabe-/Ausgabeschnittstelle, die dafür ausgebildet ist, einem Benutzer die von der Elektronik- und Auswerteeinheit ermittelte Rezirkulation, insbesondere den bestimmten aktuellen Rezirkulationswert (R), auszugeben, und/oder
  - eine unabhängig von der extrakorporalen Blutbehandlungsmaschine (50) ausgebildete Energieversorgung.

14. Modulvorrichtung (200) für eine Bestimmung einer Rezirkulation zur Nachrüstung einer extrakorporalen Blutbehandlungsmaschine (50) mit einem Dialysator (2), aufweisend:

- eine Steuer- und Recheneinheit (100), die als eine unabhängig von der extrakorporalen Blutbehandlungsmaschine (50) arbeitende Elektronik- und Auswerteeinheit ausgebildet ist; und
- eine mit der Steuer- und Recheneinheit (100) elektrisch verbundene Sensoreinheit mit:

- zumindest einer Sensorvorrichtung (31, 32),
- einem Anschlussabschnitt oder -teil zum vorzugsweise adaptiven Anschließen der Sensoreinheit an den Fluidkreis der extrakorporalen Blutbehandlungsmaschine (50), derart, dass die zumindest eine Sensorvorrichtung (31, 32) auf einer Dialysierflüssigkeitsseite (30) dem Dialysator (2) nachgeschaltet und eingerichtet ist, um zur quantitativen Bestimmung zumindest eines vorzugsweise ausgewählten oder auswählbaren Blutanteilsstoffs in der verbrauchten Dialysierflüssigkeit zumindest ein Signal ($S_{DO}$) einer physikalischen und/oder chemischen Größe der verbrauchten Dialysierflüssigkeit zu messen;

- optional eine Eingabe-/Ausgabeschnittstelle zu einem Benutzer, und/oder
- optional eine unabhängig von der extrakorporalen Blutbehandlungsmaschine (50) ausgebildete Energieversorgung; und/oder
- optional ein Bypass-Sperrventil (11) in der Bypass-Leitung (22) zur Fluidverbindung einer Dialysierflüssigkeitsquelle (3) mit einer Dialysierflüssigkeitssenke (7) und zumindest das dem Dialysator (2) vorgeschaltete Sperrventil (9) in der Dialysierflüssigkeitszulaufleitung (14), insb. beide Sperrventile (9, 10) vor und nach dem Dialysator (2), zur Schaltung in einen Interims-Betrieb des Fluidkreises;
wobei die Steuer- und Recheneinheit (100) zur Bestimmung eines Vorhandenseins einer Rezirkulation ohne blutseitige Bolusgabe dafür vorgesehen und ausgebildet ist:

- das von der zumindest einen Sensorvorrichtung (31, 32) gemessene zumindest eine Signal ($S_{DO}$) in seinem zeitlichen Verlauf zu erfassen;
- die extrakorporale Blutbehandlung in einem Grund-Modus (M-0) zum kontinuierlichen Betrieb, in welchem die Dialysierflüssigkeitsströmung durch den Dialysator (2) zugelassen ist, zu betreiben,
- das der in dem Grund-Modus (M-0) verbrauchten Dialysierflüssigkeit zugehörige Signal als ein Grund-Signal ($S_{DO, pre, L}$, $S_{DO, pre, K}$) zu messen;
- die extrakorporale Blutbehandlung von dem Grund-Modus (M-0) in einen Interims-Modus (M-I) zum Interims-Betrieb, in welchem eine Dialysierflüssigkeitsmenge auf der Dialysierflüssigkeits-Membranseite (8D) im Dialysator (2) eingesperrt wird, während der Blutstrom im extrakorporalen Blutkreis mit einer festgelegten Blutflussrate betrieben wird, wobei das Blut auf der Blut-Membranseite (8B) weiter strömt, für die jeweilige Dauer eines vorbestimmten und/oder vorbestimmbaren Interims-Zeitintervalls (L, K) zu schalten, insbesondere in einen Bypass-Modus als den Interims-Modus (M-I) zu schalten; nachfolgend
- in den Grund-Modus (M-0) zu wechseln, um die zuvor eingesperrte Dialysierflüssigkeitsmenge als einen jeweiligen Dialysierflüssigkeitsbolus der zumindest einen Sensorvorrichtung (31, 32) zuzuführen, um eine dem Dialysierflüssigkeitsbolus zugehörige Signaländerung gegenüber dem Grund-Signal ($S_{DO, pre, L}$, $S_{DO, pre, K}$) als ein zugehöriges Bolus-Signal ($S_{DO, ext, L}$, $S_{DO, ext, K}$; A; $A_{part}$) zu messen;
- den Interims-Modus (M-I) für ein erstes Interims-Zeitintervall (L) zur Gleichgewichtseinstellung des Stofftransports zu schalten, wobei eine erste Dialysierflüssigkeitsmenge im Dialysator (2) zumindest solange eingesperrt wird, bis sich auf der Dialysierflüssigkeits-Membranseite (8D) und der Blut-Membranseite (8B) des Dialysators (2) ein sich nicht mehr oder nur noch unwesentlich veränderndes Konzentrationsgleichgewicht des Blutanteilsstoffs einstellt,

**dadurch gekennzeichnet, dass**
die Steuer- und Recheneinheit (100) ferner zur Ausbildung eines Messzyklus dafür vorgesehen und ausgebildet ist:

- den Interims-Modus (M-I) für ein, gegenüber dem ersten Interims-Zeitintervall (L) kürzeres, zweites Interims-Zeitintervall (K) zu schalten, wobei eine zweite Dialysierflüssigkeitsmenge im Dialysator (2) nur solange eingesperrt wird, dass sich das Konzentrationsgleichgewicht des Blutanteilsstoffs noch nicht einstellt,
- aus einer Abweichung eines zweiten Bolus-Signals ($S_{DO, ext, K}$; $A_K$; $A_{part, K}$) in Folge des zweiten Interims-Zeitintervalls (K) gegenüber einem ersten Bolus-Signal ($S_{DO, ext, L}$; $A_L$; $A_{part, L}$) in Folge des ersten Interims-Zeitintervalls (L) das Vorhandensein der Rezirkulation zu ermitteln.

15. Speichereinheit (110), auf welcher ein Verfahren zur Überwachung einer Rezirkulation bei einer extrakorporalen Blutbehandlung unter Verwendung einer extrakorporalen Blutbehandlungsmaschine (50) nach einem der vorstehenden Ansprüche 1 bis 13 und/oder unter Verwendung einer Modulvorrichtung (200) zur Rezirkulationsbestimmung nach dem direkt vorstehenden Anspruch 14 mit den folgenden Überwachungsschritten maschinenlesbar gespeichert ist:

- optionales Identifizieren des Dialysators (2);

- Vorbestimmen des ersten Interims-Zeitintervalls (L), insb. in Abhängigkeit einer eingestellten Blutflussrate ($Q_B$) oder pauschal, innerhalb welchem sich ein Konzentrationsgleichgewicht eines vorzugsweise zuvor ausgewählten Blutanteilsstoffs, vorzugsweise Harnstoff, einstellt;

- Vorbestimmen des, gegenüber dem ersten Interims-Zeitintervall (L) kürzeren, zweiten Interims-Zeitintervalls (K), innerhalb welchem sich das Konzentrationsgleichgewicht des Blutanteilsstoffs noch nicht einstellt;

- Ermitteln eines ersten Bolus-Signals ($S_{DO, ext, L}$; $A_L$; $A_{part, L}$) in Folge des ersten Interims-Zeitintervalls (L) mit folgenden Unterschritten:

  - Betreiben der extrakorporalen Blutbehandlungsmaschine in dem Grund-Modus (M-0) unter Messen des der verbrauchten Dialysierflüssigkeit zugehörigen Signals als einem Grund-Signal ($S_{DO, pre}$);
  - Umschalten von dem Grund-Modus (M-0) in den Interims-Modus (M-I), insbesondere in den Bypass-Modus, für die Dauer des ersten Interims-Zeitintervalls (L); danach
  - Zurückwechseln in den Grund-Modus (M-0), um die zuvor eingesperrte Dialysierflüssigkeitsmenge als den ersten Dialysierflüssigkeitsbolus der zumindest einen Sensorvorrichtung (31, 32) zuzuführen, und darauffolgendes Messen des ersten Bolus-Signals ($S_{DO, ext, L}$; $A_{part, L}$) als einer dem ersten Dialysierflüssigkeitsbolus zugehörigen Signaländerung gegenüber dem Grund-Signal ($S_{DO, pre}$);

- Ermitteln eines zweiten Bolus-Signals ($S_{DO, ext, K}$; $A_K$; $A_{part, K}$) in Folge des zweiten Interims-Zeitintervalls (K), vorher oder nachher zum Ermitteln des ersten Bolus-Signals ($S_{DO, ext, L}$; $A_{part, L}$), mit folgenden Unterschritten:

  - Betreiben der extrakorporalen Blutbehandlungsmaschine in dem Grund-Modus (M-0);
  - Messen des der in dem Grund-Modus (M-0) verbrauchten Dialysierflüssigkeit zugehörigen Signals als einem Grund-Signal ($S_{DO, pre}$);
  - Umschalten von dem Grund-Modus (M-0) in den Interims-Modus (M-I), insbesondere in den Bypass-Modus, für die Dauer des zweiten Interims-Zeitintervalls (K); danach
  - Zurückwechseln in den Grund-Modus (M-0), um die zuvor eingesperrte Dialysierflüssigkeitsmenge als den zweiten Dialysierflüssigkeitsbolus der zumindest einen Sensorvorrichtung (31, 32) zuzuführen, und darauffolgendes Messen des zweiten Bolus-Signals ($S_{DO, ext, K}$; $A_K$; $A_{part, K}$) als einer dem zweiten Dialysierflüssigkeitsbolus zugehörigen Signaländerung gegenüber dem Grund-Signal ($S_{DO, pre}$); und

- Ermitteln des Vorhandenseins der Rezirkulation, insbesondere Berechnen des aktuellen Rezirkulationswertes (R) anhand des Berechnungsmodells, aus einer Abweichung, insbesondere gemäß einem Quotienten, des zweiten Bolus-Signals ($S_{DO, ext, K}$; $A_K$; $A_{part, K}$) gegenüber dem ersten Bolus-Signal ($S_{DO, ext, L}$; $A_{part, L}$);

- optionales Anzeigen der Rezirkulation bzw. des aktuellen Rezirkulationswertes (R).

## Claims

1. An extracorporeal blood treatment machine (50), in particular dialysis machine, with a dialyzer (2) and at least one sensor device (31, 32), which is connected downstream of the dialyzer (2) on a dialysis fluid side (30) and is electrically connected to a control and calculation unit (100),

   wherein the control and calculation unit (100) for determining a presence of recirculation without blood-side bolus delivery is provided for:

   - detecting, in its chronological course, the at least one signal ($S_{DO}$) of a physical and/or chemical quantity of the spent dialysis fluid measured by the at least one sensor device (31, 32) for quantitative determination of at least one preferably selected or selectable blood component substance in the spent dialysis fluid;
   - operating the extracorporeal blood treatment machine in a base mode (M-0) for continuous operation in which the dialysis fluid flow through the dialyzer (2) is permitted,
   - measuring the signal associated with the dialysis fluid consumed in the base mode (M-0) as a base signal ($S_{DO, pre, L}$, $S_{DO, pre, K}$);
   - switching the extracorporeal blood treatment machine from the base mode (M-0) to an interim mode (M-I) for interim operation, in which a dialysis fluid quantity is confined on the dialysis fluid membrane side (8D) in the dialyzer (2), while the blood flow in the extracorporeal blood circuit is operated at a predetermined blood flow rate, wherein the blood continues to flow on the blood membrane side (8B), for the respective duration of a predetermined and/or predeterminable interim time interval (L, K), in particular to switch to a bypass mode as the interim mode (M-I); subsequently

- to change to base mode (M-0) to supply the previously confined dialysis fluid quantity as a respective dialysis fluid bolus to the at least one sensor device (31, 32) to measure a signal change associated with the dialysis fluid bolus relative to the base signal ($S_{DO, pre, L}$, $S_{DO, pre, K}$) as an associated bolus signal ($SDO_{, ext, L}$, $S_{DO, ext, K}$; A; $A_{part}$);

- the interim mode (M-I) for a first interim time interval (L) for setting the equilibrium of the substance transport, wherein a first dialysis fluid quantity is confined in the dialyzer (2) at least until a concentration balance of the blood component substance no longer or only insignificantly changes on the dialysis fluid membrane side (8D) and the blood membrane side (8B) of the dialyzer (2),

**characterized in that**

the control and calculation unit (100) is further provided and adapted to form a measuring cycle for:

- switching to the interim mode (M-I) for a second interim time interval (K) which is shorter than the first interim time interval (L), wherein a second dialysis fluid quantity is confined in the dialyzer (2) only until the concentration balance of the blood component substance has been established,

- determining the presence of recirculation from a deviation of a second bolus signal ($S_{DO, ext, K}$; $A_K$; $A_{part, K}$) in succession of the second interim time interval (K) relative to a first bolus signal ($S_{DO, ext, L}$; $A_L$; $A_{part, L}$) in succession of the first interim time interval (L).

2. The extracorporeal blood treatment machine (50) according to claim 1, **characterized in that**

- the first bolus signal ($S_{DO, ext, L}$; $A_L$; $A_{part, L}$) and second bolus signal ($S_{DO, ext, K}$; $A_K$; $A_{part, K}$) each corresponds to a maximum peak ($S_{DO, ext, L}$, $S_{DO, ext, K}$) in the signal of the at least one sensor device (31, 32) immediately after the release of the respective first or second dialysis fluid bolus from the dialyzer (2);
- the control and calculation unit (100) is further provided and configured for:

- determining a delta quotient ($V_D$) from a second difference ($D_K$) calculated from the maximum peak of the second bolus signal ($S_{DO, ext, K}$) and the base signal ($S_{DO, pre}$) relative to a first difference ($D_L$) calculated from the maximum peak of the first bolus signal ($S_{DO, ext, L}$) and the base signal ($S_{DO, pre}$); and
- determining a current recirculation value (R) based on the determined delta quotient ($V_D$) and a previously known computational model.

3. The extracorporeal blood treatment machine (50) according to claim 1 or 2, **characterized in that**

- the first bolus signal ($S_{DO, ext, L}$; $A_L$; $A_{part, L}$) and second bolus signal ($S_{DO, ext, K}$; $A_K$; $A_{part, K}$), if applicable additionally, corresponds in each case to a total surface integral ($A_L$, $A_K$), adjusted for the base signal ($S_{DO, pre}$), in the signal of the at least one sensor device (31, 32) immediately after the release of the respective first or second dialysis fluid bolus from the dialyzer (2);
- the control and calculation unit (100) is further provided and configured for:

- determining a total surface integral quotient ($V_A$) from the second total surface integral ($A_K$) with respect to the first total surface integral ($A_L$); and
- determining an actual recirculation value (R) based on the total area integral quotient ($V_A$) and a previously known computational model.

4. The extracorporeal blood treatment machine (50) according to one of the preceding claims, **characterized in that**

- the first bolus signal ($S_{DO, ext, L}$; $A_L$; $A_{part, L}$) and second bolus signal ($S_{DO, ext, K}$; $A_K$; $A_{part, K}$), if applicable additionally, corresponds in each case to a partial surface integral ($A_{part, L}$, $A_{part, K}$), adjusted for the base signal ($S_{DO, pre}$), in the signal of the at least one sensor device (31, 32) immediately after the release of the respective first or second dialysis fluid bolus from the dialyzer (2);
- the control and calculation unit (100) is further provided and configured for:

- determining a partial surface integral quotient ($V_{A, part}$) from the second partial surface integral ($A_{part, K}$) with respect to the first partial surface integral ($A_{part, L}$); and
- determining an actual recirculation value (R) on the basis of the partial area integral quotient ($V_{A, part}$) as well as a previously known computational model.

5. The extracorporeal blood treatment machine (50) according to one of claims 2 to 4, **characterized in that** the computational model for calculating the current recirculation value (R) takes into account a dialysis fluid flow rate ($Q_D$) of the dialysis fluid circuit (30), a blood flow rate ($Q_B$) in the extracorporeal blood tubing system of the blood circuit (20), and a theoretical clearance of the dialyzer (2), in particular also taking into account at least one of a number of further machine, dialyzer and/or setting parameters of the blood treatment machine (50) as an influencing variable.

6. The extracorporeal blood treatment machine (50) according to one of the preceding claims, **characterized in that** the at least one first sensor device (31) connected on a dialysis fluid side (30) downstream of the dialyzer (2) and/or, if applicable, a further second sensor device (32) connected downstream of the dialyzer (2) and directed at another physical and/or chemical variable:

   - is adapted to detect as the at least one signal ($S_{DO}$) in particular a conductivity of the consumed dialysis fluid in its chronological course, further preferably configured as a temperature-compensating conductivity probe; and/or
   - is adapted to detect as the at least one signal ($S_{DO}$) an absorption property, in particular the absorbance and/or the transmittance, of the spent dialysis fluid in its chronological course, further preferably in a wavelength range between 200 nm and 320 nm; and/or
   - in the dialysis fluid outlet line (16) is arranged directly at the dialysis fluid outlet (6).

7. The extracorporeal blood treatment machine (50) according to the directly preceding claim, **characterized in that** the control and calculation unit (100) is further provided and configured for:

   - determining a first current recirculation value (R) based on the first signal ($S_{DO}$) from the first sensor device (31) and a second current recirculation value (R) based on the second signal ($S_{DO}$) from the second sensor device (32); and thereafter based on the comparison of the first and second current recirculation values (R):
   - preferably averaging therefrom a mean current recirculation value (R); or
   - in the event of a deviation, in particular a statistically significant deviation of more than 10 percentage points, rejecting the result and automatically initiating a new measuring cycle;
   or
   - based on a preliminary decision as to whether the corresponding first or second current recirculation value (R) falls within a predetermined relevance range, not rejecting the result.

8. The extracorporeal blood treatment machine (50) according to one of the preceding claims, **characterized in that** furthermore at least one third sensor device (33) is provided on a dialysis fluid side (30) upstream of the dialyzer (2), which:

   - is adapted to detect as the at least one signal ($S_{DO}$) in particular a conductivity of the unconsumed dialysis fluid in its chronological course, further preferably configured as a temperature compensating conductivity probe; and/or
   - is adapted to detect as the at least one signal ($S_{DO}$) an absorption property, in particular the absorbance and/or the transmittance, of the unconsumed dialysis fluid in its chronological course, further preferably in a wavelength range between 200 nm and 320 nm; and/or
   - is arranged in the dialysis fluid inlet line (14) directly at the dialysis fluid inlet (4); and/or
   - is arranged upstream on the bypass line (22).

9. The extracorporeal blood treatment machine (50) according to the immediately preceding claim 8, **characterized in that**

   - the third sensor device (33) connected upstream of the dialyzer (2) and the first sensor device (31) connected downstream of the dialyzer (2) are set up on the basis of an identical measuring principle in order to detect an identical physical and/or chemical variable, in particular a conductivity, as the respectively associated third signal ($S_{DO}$) of the third sensor device (33) and the first signal ($S_{DO}$) of the first sensor device (31); and
   - the second sensor device (32) connected downstream of the dialyzer (2) is set up on the basis of a measuring principle different from the first one in order to detect a physical and/or chemical variable different from the second signal ($S_{DO}$) of the second sensor device (32);

   the control and calculation unit (100) is further provided and configured for:

- in the event of a substantially determinable match of the third signal ($S_{DO}$) and the first signal ($S_{DO}$) for the base mode (M-0), the second signal ($S_{DO}$) of the second sensor device (32) is used as a prioritizing signal for the measuring cycle in order to determine the presence of a recirculation, in particular to determine the current recirculation value (R).

**10.** The extracorporeal blood treatment machine (50) according to one of the preceding claims, **characterized in that**

- the dialyzer (2) has a dialysis fluid inlet (4) for fresh dialysis fluid, a dialysis fluid outlet (6) for used dialysis fluid and a filter membrane (8), which is a dialysis fluid membrane side (8D), on which the dialyzer (2) is connected to a dialysis fluid circuit (30) via a dialysis fluid inlet line (14) and a dialysis fluid outlet line (16), from a blood membrane side (8B) on which the dialyzer (2) is connected or connectable to an extracorporeal blood circuit (20);
- a bypass line (22) is provided via which the dialysis fluid membrane side (8D) can be selectively bridged in a bypass mode as the interim mode (M-I) for interim operation for the first interim time interval (L) as well as for the second interim time interval (K), in order to temporarily confine dialysis fluid located in the dialyzer (2), for which purpose at least one check valve (9, 10) electrically connected to the control and calculation unit (100) is arranged between the bypass line (22) and the dialyzer (2) at the dialysis fluid inlet line (14) and the dialysis fluid outlet line (16);
- the control and calculation unit (100) for controlling the extracorporeal blood treatment machine (50) is configured or can be equipped with a storage unit (110) and/or is electrically connected or can be connected via an interface to a storage unit external with respect to the control and calculation unit (100),

wherein the extracorporeal blood treatment machine (50) further comprises:

- a data set which is stored or can be stored on the storage unit (110) and which specifies a number of blood flow rates ($Q_B$) in the extracorporeal blood circuit (20) which are suitable for different parameters of the blood treatment machine (50) and associated first interim time intervals (L) which are preferably determined analytically in advance and/or set as a lump sum, within which, in the dialyzer (2), with a correspondingly set blood flow rate ($Q_B$) and assuming a maximum possible recirculation value (R), a concentration balance of the at least one selected or selectable blood component substance between blood in the extracorporeal blood circuit (20) and dialysis fluid confined in the dialyzer (2) is completed exclusively as a result of diffusion;

so that the control and calculation unit (100), in order to determine the first bolus signal ($S_{DO, ext, L}$; $A_L$; $A_{part, L}$) or the second bolus signal ($S_{DO, ext, K}$; $A_K$; $A_{part, K}$), respectively, switches the extracorporeal blood treatment machine (50) into the bypass mode for the duration of the first interim time interval (L) specified in the data set, or, respectively, of the second interim time interval (K), which is shorter than the first interim time interval (L), and operates the extracorporeal blood circuit (20), preferably simultaneously, at the specified blood flow rate ($Q_B$) and, immediately after termination of the bypass mode, for the first or, respectively, second dialysis fluid bolus created by the bypass mode, the signal change associated with the respective first or second dialysis fluid bolus is detected metrologically with respect to the base signal ($S_{DO, pre}$) as an associated first bolus signal ($S_{DO, ext, L}$; $A_L$; $A_{part, L}$) or second bolus signal ($S_{DO, ext, K}$; $A_K$; $A_{part, K}$), in the used dialysis fluid flowing out of the dialyzer (2) by means of the at least one sensor device (31, 32),

- preferably the computational model stored on the storage unit (110), by means of which the control and calculation unit (100) optionally calculates a current recirculation value (R) taking into account the first bolus signal ($S_{DO, ext, L}$; $A_L$; $A_{part, L}$), the second bolus signal ($SDO, ext, K$; $A_K$; $A_{part, K}$) and the base signal ($SDO, pre$), preferably before the start or at the start of a treatment cycle via the extracorporeal blood treatment machine (50).

**11.** The extracorporeal blood treatment machine (50) according to one of the preceding claims, **characterized in that** the storage unit (32) is permanently integrated in the extracorporeal blood treatment machine (1).

**12.** The extracorporeal blood treatment machine (50) according to one of the preceding claims, **characterized in that** the extracorporeal blood treatment machine (1) further comprises a display device, connected wirelessly or by cable, which is set up to display the determined presence of a recirculation, in particular the determined current recirculation value (R).

**13.** The extracorporeal blood treatment machine (50) according to one of the preceding claims, **characterized in that** the at least one sensor device (31, 32) and the control and calculation unit (100) are provided in or respectively on a separate module device (200), which can be retrofitted and/or is retrofitted to the extracorporeal blood treatment machine (50), for recirculation determination, wherein the module device (200) comprises:

- a sensor unit with:

- the at least one sensor device (31, 32),
- a terminal portion or part for preferably adaptively connecting the sensor unit to the fluid circuit of the extracorporeal blood treatment machine (50), such that the at least one sensor device (31, 32) on the dialysis fluid side (30) is connected downstream of the dialyzer (2) and is arranged to detect the at least one signal ($S_{DO}$) of a physical and/or chemical quantity of the consumed dialysis fluid in its chronological course; and

- an electronic and evaluation unit configured as the control and calculation unit (100), operating independently of the extracorporeal blood treatment machine (50), which determines the presence of the recirculation, in particular determines the current recirculation value (R); further optionally:
- a, preferably combined, input/output interface adapted to output to a user the recirculation determined by the electronic and evaluation unit, in particular the determined current recirculation value (R), and/or
- a power supply formed independently of the extracorporeal blood treatment machine (50).

14. A module device (200) for determining recirculation for retrofitting an extracorporeal blood treatment machine (50) with a dialyzer (2), comprising:

- a control and calculation unit (100) configured as an electronic and evaluation unit operating independently of the extracorporeal blood treatment machine (50); and
- a sensor unit electrically connected to the control and calculation unit (100) with:

- at least one sensor device (31, 32),
- a terminal portion or part for preferably adaptively connecting the sensor unit to the fluid circuit of the extracorporeal blood treatment machine (50), such that the at least one sensor device (31, 32) on a dialysis fluid side (30) is connected downstream of the dialyzer (2) and is arranged to measure at least one signal ($S_{DO}$) of a physical and/or chemical quantity of the spent dialysis fluid for quantitative determination of at least one preferably selected or selectable blood component substance in the spent dialysis fluid;

- optionally an input/output interface to a user, and/or
- optionally a power supply independent of the extracorporeal blood treatment machine (50); and/or
- optionally a bypass check valve (11) in the bypass line (22) for fluid connection of a dialysis fluid source (3) with a dialysis fluid sink (7) and at least the check valve (9) connected upstream of the dialyzer (2) in the dialysis fluid inlet line (14), in particular both check valves (9, 10) upstream and downstream of the dialyzer (2), for switching into an interim operation of the fluid circuit;
wherein the control and calculation unit (100) for determining a presence of recirculation without blood side bolus delivery is provided for:

- detecting the at least one signal ($S_{DO}$) measured by the at least one sensor device (31, 32) in its chronological course;
- operate the extracorporeal blood treatment in a base mode (M-0) for continuous operation, in which the dialysis fluid flow through the dialyzer (2) is permitted,
- measure the signal associated with the dialysis fluid consumed in the base mode (M-0) as a base signal ($S_{DO, pre, L}$, $S_{DO, pre, K}$);
- the extracorporeal blood treatment from the base mode (M-0) into an interim mode (M-I) for interim operation, in which a dialysis fluid quantity is confined on the dialysis fluid membrane side (8D) in the dialyzer (2), while the blood flow in the extracorporeal blood circuit is operated at a predetermined blood flow rate, wherein the blood continues to flow on the blood membrane side (8B), for the respective duration of a predetermined and/or predeterminable interim time interval (L, K), in particular to switch to a bypass mode as the interim mode (M-I); hereinafter
- to change to base mode (M-0) to supply the previously confined dialysis fluid quantity as a respective dialysis fluid bolus to the at least one sensor device (31, 32) to measure a signal change associated with the dialysis fluid bolus relative to the base signal ($S_{DO, pre, L}$, $S_{DO, pre, K}$) as an associated bolus signal ($S_{DO, ext, L}$, $S_{DO, ext, K}$; A; $A_{part}$);
- the interim mode (M-I) for a first interim time interval (L) for setting the equilibrium of the substance transport, wherein a first dialysis fluid quantity is confined in the dialyzer (2) at least until a concentration balance of the blood component substance no longer or only insignificantly changes on the dialysis fluid membrane side (8D) and the blood membrane side (8B) of the dialyzer (2),

**characterized in that**
the control and calculation unit (100) is further provided and adapted to form a measuring cycle for:

- the interim mode (M-I) for a second interim time interval (K) which is shorter than the first interim time interval (L), wherein a second dialysis fluid quantity is confined in the dialyzer (2) only until the concentration balance of the blood component substance has been established,

determining the presence of recirculation from a deviation of a second bolus signal ($S_{DO, ext, K}$; $A_K$ ; $A_{part, K}$) in succession of the second interim time interval (K) relative to a first bolus signal ($S_{DO, ext, L}$; $A_L$ ; $A_{part, L}$) in succession of the first interim time interval (L).

15. Storage unit (110) on which a method for monitoring a recirculation in an extracorporeal blood treatment using an extracorporeal blood treatment machine (50) according to one of the preceding claims 1 to 13 and/or using a module device (200) for recirculation determination according to the directly preceding claim 14 with the following monitoring steps is machine-readably stored:

- optional identification of the dialyzer (2);
- predetermining the first interim time interval (L), in particular as a function of a set blood flow rate ($Q_B$) or in general, within which a concentration balance of a preferably previously selected blood component substance, preferably urea, is established;
- pre-determination of the second interim time interval (K), which is shorter than the first interim time interval (L) and within which the concentration balance of the blood component substance has not yet been established;
- determ inng a first bolus signal ($S_{DO, ext, L}$; $A_L$ ; $A_{part, L}$) in sequence of the first interim time interval (L) with the following sub steps:

- operating the extracorporeal blood treatment machine in the base mode (M-0) while measuring the signal associated with the consumed dialysis fluid as a base signal ($S_{DO, pre}$);
- switching from the base mode (M-0) to the interim mode (M-I), in particular to bypass mode, for the duration of the first interim time interval (L); then
- switching back to the base mode (M-0) to supply the previously confined dialysis fluid quantity as the first dialysis fluid bolus to the at least one sensor device (31, 32), and subsequently measuring the first bolus signal ($S_{DO, ext, L}$; $A_L$ ; $A_{part, L}$) as a signal change associated with the first dialysis fluid bolus relative to the base signal ($S_{DO, pre}$);

- detecting a second bolus signal ($S_{DO, ext, K}$; $A_K$ ; $A_{part, K}$) in sequence of the second interim time interval (K), before or after detecting the first bolus signal ($S_{DO, ext, L}$; $A_L$ ; $A_{part, L}$), with the following sub steps:

- operating of the extracorporeal blood treatment machine in the base mode (M-0);
- measuring the signal associated with the dialysis fluid consumed in the base mode (M-0) as a base signal ($S_{DO, pre}$);
- switching from the base mode (M-0) to the interim mode (M-I), in particular to the bypass mode, for the duration of the second interim time interval (K); then
- switching back to the base mode (M-0) to supply the previously confined dialysis fluid quantity as the second dialysis fluid bolus to the at least one sensor device (31, 32), and thereafter measuring the second bolus signal ($S_{DO, ext, K}$; $A_K$ ; $A_{part, K}$) as a signal change associated with the second dialysis fluid bolus from the base signal ($S_{DO, pre}$); and

- determining the presence of the recirculation, in particular calculating the current recirculation value (R) using the computational model, from a deviation, in particular according to a quotient, of the second bolus signal ($S_{DO, ext, K}$; $A_K$ ; $A_{part, K}$) with respect to the first bolus signal ($S_{DO, ext, L}$; $A_L$ ; $A_{part, L}$);
- optional display of the recirculation or the current recirculation value (R).

**Revendications**

1. Machine extracorporelle de traitement du sang (50), en particulier machine de dialyse, comprenant un dialyseur (2) ainsi qu'au moins un dispositif de détection (31, 32) qui est monté en aval du dialyseur (2) sur un côté de liquide de dialyse (30) et qui est relié électriquement à une unité de commande et de calcul (100), dans laquelle l'unité de commande et de calcul (100) est prévue et réalisée à cet effet pour déterminer la présence d'une recirculation sans délivrance de bolus côté sang :

   - détecter dans son évolution temporelle l'au moins un signal ($S_{DO}$) d'une grandeur physique et/ou chimique du liquide de dialyse consommé, mesuré par l'au moins un dispositif de détection (31, 32) pour la détermination quantitative d'au moins un composant sanguin de préférence sélectionné ou sélectionnable dans le liquide de dialyse utilisé ;
   - faire fonctionner la machine extracorporelle de traitement du sang dans un mode de base (M-0) pour un fonctionnement continu, dans lequel l'écoulement de liquide de dialyse à travers le dialyseur (2) est autorisé,
   - mesurer le signal associé au liquide de dialyse utilisé dans le mode de base (M-0) comme un signal de base ($S_{DO, \, pre, \, L}$, $S_{DO, \, pre, \, K}$) ;
   - la machine extracorporelle de traitement du sang du mode de base (M-0) à un mode intermédiaire (M-I) pour un fonctionnement intermédiaire dans lequel une quantité de liquide de dialyse est enfermée sur le côté membrane de liquide de dialyse (8D) dans le dialyseur (2), tandis que le débit sanguin dans le circuit extracorporel de sang fonctionne à un débit sanguin fixe, dans laquelle le sang continue à s'écouler sur le côté membrane de sang (8B), pendant la durée respective d'un intervalle de temps intermédiaire (L, K) prédéterminé et/ou pouvant être prédéterminé, en particulier pour passer à un mode de dérivation autre que le mode intermédiaire (M-I) ; puis
   - passer au mode de base (M-0) pour fournir la quantité de liquide de dialyse précédemment bloquée comme un bolus de liquide de dialyse respectif à l'au moins un dispositif de détection (31, 32) pour mesurer une variation de signal associée au bolus de liquide de dialyse par rapport au signal de base ($S_{DO, \, pre, \, L}$, $S_{DO, \, pre, \, K}$) comme un signal de bolus associé ($S_{DO, \, ext, \, L}$, $S_{DO, \, ext, \, K}$; A; $A_{part}$) ;
   - commuter le mode intermédiaire (M-I) pour un premier intervalle de temps intermédiaire (L) pour l'ajustement de l'équilibre du transport de substance, dans laquelle une première quantité de liquide de dialyse est enfermée dans le dialyseur (2) au moins jusqu'à ce qu'un équilibre de concentration de substance constitutive du sang qui ne se modifie plus ou seulement de manière insignifiante s'établisse sur le côté membrane de liquide de dialyse (8D) et le côté membrane de sang (8B) du dialyseur (2),

   **caractérisé en ce que**
   l'unité de commande et de calcul (100) est en outre prévue et réalisée pour réaliser un cycle de mesure à cet effet :

   - le mode intermédiaire (M-I) pour un second intervalle de temps intermédiaire (K) plus court que le premier intervalle de temps intermédiaire (L), dans laquelle une seconde quantité de liquide de dialyse n'est enfermée dans le dialyseur (2) que tant que l'équilibre de concentration de la substance constitutive du sang ne s'est pas encore établi,
   - déterminer la présence de la recirculation à partir d'un écart entre un deuxième signal de bolus ($S_{DO, \, ext, \, K}$ ; $A_K$ ; $A_{part, \, K}$) à la suite du second intervalle de temps intermédiaire (K) et un premier signal de bolus ($S_{DO, \, ext, \, L}$ ; $A_L$ ; $A_{part, \, L}$) à la suite du premier intervalle de temps intermédiaire (L).

2. Machine extracorporelle de traitement du sang (50) selon la revendication 1, **caractérisée en ce que**

   - le premier signal de bolus ($S_{DO, \, ext, \, L}$ ; $A_L$ ; $A_{part, \, L}$) et le deuxième signal de bolus ($S_{DO, \, ext, \, K}$ ; ; $A_{part, \, K}$) correspondent respectivement à un pic maximal ($S_{DO, \, ext, \, L}$, $S_{DO, \, ext, \, K}$) dans le signal du au moins un dispositif de détection (31, 32) immédiatement après la libération du premier ou du second bolus de liquide de dialyse respectif hors du dialyseur (2) ;
   - l'unité de commande et de calcul (100) est en outre prévue et réalisée pour :

     - déterminer un quotient delta ($V_D$) à partir d'une seconde différence ($D_K$), calculée à partir du pic maximal du deuxième signal de bolus ($S_{DO, \, ext, \, K}$) et du signal de base ($S_{DO, \, pre}$), par rapport à une première différence ($D_L$), calculée à partir du pic maximal du premier signal de bolus ($S_{DO, \, ext, \, L}$) et du signal de base ($S_{DO, \, pre}$) ; et
     - déterminer une valeur de recirculation actuelle (R) sur la base du quotient delta ($V_D$) déterminé ainsi que d'un modèle de calcul connu au préalable.

**3.** Machine extracorporelle de traitement du sang (50) selon la revendication 1 ou 2, **caractérisée en ce que**

- le premier signal de bolus ($S_{DO, \, ext, \, L}$ ; $A_L$ ; $A_{part, \, L}$) et le deuxième signal de bolus ($S_{DO, \, ext, \, K}$ ; $A_K$ ; $A_{part, \, K}$) correspondent, le cas échéant de manière supplémentaire, respectivement à une intégrale de surface totale ($A_L$, $A_K$), corrigée du signal de base ($S_{DO, \, pre}$) dans le signal du au moins un dispositif de détection (31, 32) immédiatement après la libération du premier ou du second bolus de liquide de dialyse respectif hors du dialyseur (2) ;
- l'unité de commande et de calcul (100) est en outre prévue et réalisée pour :

   - déterminer un quotient d'intégrale de surface totale ($V_A$) à partir de la seconde intégrale de surface totale ($A_K$) par rapport à la première intégrale de surface totale ($A_L$) ; et
   - déterminer une valeur de recirculation actuelle (R) sur la base du quotient d'intégrale de surface totale ($V_A$) ainsi que d'un modèle de calcul connu au préalable.

**4.** Machine extracorporelle de traitement du sang (50) selon l'une quelconque des revendications précédentes, **caractérisée en ce que**

- le premier signal de bolus ($S_{DO, \, ext, \, L}$ ; $A_L$ ; $A_{part, \, L}$) et le deuxième signal de bolus ($S_{DO, \, ext, \, K}$ ; $A_K$ ; $A_{part, \, K}$) correspondent, le cas échéant de manière supplémentaire, respectivement à une intégrale de surface partielle ($A_{part, \, L}$, $A_{part, \, K}$), corrigée du signal de base ($S_{DO, \, pre}$) dans le signal du au moins un dispositif de détection (31, 32) immédiatement après la libération du premier ou du second bolus de liquide de dialyse respectif hors du dialyseur (2) ;
- l'unité de commande et de calcul (100) est en outre prévue et réalisée pour :

   - déterminer un quotient d'intégrale de surface partielle ($V_{A, \, part}$) à partir de la seconde intégrale de surface partielle ($A_{part, \, K}$) par rapport à la première intégrale de surface partielle ($A_{part, \, L}$) ; et
   - déterminer une valeur de recirculation actuelle (R) sur la base du quotient d'intégrale de surface partielle ($V_{A, \, part}$) ainsi que d'un modèle de calcul connu au préalable.

**5.** Machine extracorporelle de traitement du sang (50) selon l'une quelconque des revendications 2 à 4, **caractérisée en ce que** le modèle de calcul pour calculer la valeur de recirculation actuelle (R) tient compte d'un débit de liquide de dialyse ($Q_D$) du circuit de liquide de dialyse (30), d'un débit sanguin ($Q_B$) dans le système extracorporel de tuyauterie sanguine du circuit du sang (20) et d'une clairance théorique du dialyseur (2), en particulier en tenant compte en outre d'au moins un paramètre parmi un certain nombre d'autres paramètres de la machine, du dialyseur et/ou des paramètres de réglage de la machine de traitement du sang (50) en tant que grandeur d'influence.

**6.** Machine extracorporelle de traitement du sang (50) selon l'une quelconque des revendications précédentes, **caractérisée en ce que** le au moins un premier dispositif de détection (31) monté en aval du dialyseur (2) sur un côté du liquide de dialyse (30) et/ou le cas échéant un autre deuxième dispositif de détection (32) monté en aval du dialyseur (2) et orienté sur une autre grandeur physique et/ou chimique :

- est conçu pour détecter, en tant qu'au moins un signal ($S_{DO}$), en particulier une conductivité du liquide de dialyse utilisé dans son évolution dans le temps, plus préférentiellement est réalisé comme une sonde de conductivité compensant la température ; et/ou
- est conçu pour détecter, en tant qu'au moins un signal ($S_{DO}$), une propriété d'absorption, en particulier l'absorbance et/ou le degré de transmission, du liquide de dialyse utilisé dans son évolution dans le temps, plus préférentiellement dans une plage de longueurs d'onde comprise entre 200 nm et 320 nm ; et/ou
- est agencé dans la conduite d'évacuation de liquide de dialyse (16) directement sur l'évacuation de liquide de dialyse (6).

**7.** Machine extracorporelle de traitement du sang (50) selon la revendication qui précède directement, **caractérisée en ce que**

l'unité de commande et de calcul (100) est en outre prévue et réalisée pour :

   - déterminer une première valeur de recirculation actuelle (R) sur la base du premier signal ($S_{DO}$) du premier dispositif de détection (31) et une seconde valeur de recirculation actuelle (R) sur la base du deuxième signal ($S_{DO}$) du deuxième dispositif de détection (32) ; et ensuite, sur la base de la comparaison des première et seconde valeurs de recirculation actuelles (R) :

de préférence en déduire une valeur moyenne de recirculation actuelle (R) ; ou

- dans le cas d'un écart, en particulier statistiquement significatif, supérieur à 10 points de pourcentage, rejeter le résultat et lancer automatiquement un nouveau cycle de mesure ; ou
- sur la base d'une décision préalable indiquant si la première ou la seconde valeur de recirculation actuelle (R) correspondante se situe dans une plage de pertinence prédéterminée, ne pas rejeter le résultat.

8. Machine extracorporelle de traitement du sang (50) selon l'une quelconque des revendications précédentes, **caractérisée en ce qu'il** est en outre prévu au moins un troisième dispositif de détection (33) monté en amont du dialyseur (2) sur un côté de liquide de dialyse (30), lequel :

- est conçu pour détecter, en tant qu'au moins un signal ($S_{DO}$), en particulier une conductivité du liquide de dialyse non utilisé dans son évolution dans le temps, plus préférentiellement est réalisé sous la forme d'une sonde de conductivité compensant la température ; et/ou
- est conçu pour détecter, en tant qu'au moins un signal ($S_{DO}$), une propriété d'absorption, en particulier l'absorbance et/ou le degré de transmission, du liquide de dialyse non utilisé dans son évolution dans le temps, plus préférentiellement dans une plage de longueurs d'onde comprise entre 200 nm et 320 nm ; et/ou
- est agencé dans la conduite d'amenée de liquide de dialyse (14) directement sur l'amenée de liquide de dialyse (4) ; et/ou
- en amont de la conduite de dérivation (22).

9. Machine extracorporelle de traitement du sang (50) selon la revendication 8 qui précède directement, **caractérisée en ce que**

- le troisième dispositif de détection (33) monté en amont du dialyseur (2) et le premier dispositif de détection (31) monté en aval du dialyseur (2) sont conçus sur la base d'un même principe de mesure pour détecter une même grandeur physique et/ou chimique, en particulier une conductivité, en tant que troisième signal ($S_{DO}$) respectivement associé du troisième dispositif de détection (33) ainsi que premier signal ($S_{DO}$) du premier dispositif de détection (31) ; et
- le deuxième dispositif de détection (32) monté en aval du dialyseur (2) est conçu sur la base d'un principe de mesure différent pour détecter une grandeur physique et/ou chimique différente du deuxième signal correspondant ($S_{DO}$) du deuxième dispositif de détection (32) ;
- l'unité de commande et de calcul (100) est en outre prévue et réalisée pour :
- dans le cas d'une concordance, pouvant être déterminée pour l'essentiel pour le mode de base (M-0), du troisième signal ($S_{DO}$) et du premier signal ($S_{DO}$), utiliser le deuxième signal ($S_{DO}$) du deuxième dispositif de détection (32) en priorité pour le cycle de mesure, afin de déterminer la présence d'une recirculation, en particulier pour déterminer la valeur de recirculation actuelle (R).

10. Machine extracorporelle de traitement du sang (50) selon l'une quelconque des revendications précédentes, **caractérisée en ce que**

- le dialyseur (2) présente une amenée de liquide de dialyse (4) pour le liquide de dialyse frais, une évacuation de liquide de dialyse (6) pour le liquide de dialyse utilisé et une membrane filtrante (8), qui présente un côté de membrane de liquide de dialyse (8D), sur lequel le dialyseur (2) est raccordé à un circuit de liquide de dialyse (30) par l'intermédiaire d'une conduite d'amenée de liquide de dialyse (14) et d'une conduite d'évacuation de liquide de dialyse (16), sépare d'un côté de membrane de sang (8B) sur lequel le dialyseur (2) est raccordé ou peut être raccordé à un circuit sanguin extracorporel (20) ;
- une conduite de dérivation (22) est prévue, au moyen de laquelle le côté membrane de liquide de dialyse (8D) peut être dérivé au choix dans un mode de dérivation comme le mode intermédiaire (M-I) pour le fonctionnement intermédiaire pour le premier intervalle de temps intermédiaire (L) ainsi que pour le second intervalle de temps intermédiaire (K), pour enfermer temporairement le liquide de dialyse se trouvant dans le dialyseur (2), pour lequel au moins une soupape d'arrêt (9, 10) reliée électriquement à l'unité de commande et de calcul (100) est agencée entre la conduite de dérivation (22) et le dialyseur (2) sur la conduite d'amenée de liquide de dialyse (14) et la conduite d'évacuation de liquide de dialyse (16) ;
- l'unité de commande et de calcul (100) pour la commande de la machine extracorporelle de traitement du sang (50) est réalisée ou peut être équipée d'une unité de mémoire (110) et/ou est reliée ou peut être reliée électriquement par une interface à une unité de mémoire externe par rapport à l'unité de commande et de calcul (100),

dans lequel la machine extracorporelle de traitement du sang (50) présente en outre :

- un jeu de données stocké ou pouvant être stocké sur l'unité de mémoire (110), qui indique un nombre de débits sanguins ($Q_B$) appropriés pour différents paramètres de la machine de traitement du sang (50) dans le circuit sanguin extracorporel (20) et des premiers intervalles de temps intermédiaires (L) associés, de préférence déterminés préalablement de manière analytique et/ou fixés de manière globalisée, dans lequel, dans le dialyseur (2), pour un débit sanguin ($Q_B$) réglé de manière correspondante et en supposant une valeur de recirculation (R) maximale possible, un équilibrage de la concentration d'au moins une substance sanguine sélectionnée ou pouvant être sélectionnée entre le sang dans le circuit sanguin extracorporel (20) et le liquide de dialyse enfermé dans le dialyseur (2) est terminé exclusivement par suite de la diffusion ;
- de sorte que l'unité de commande et de calcul (100), pour déterminer le premier signal de bolus ($S_{DO, ext, L}$ ; $A_L$ ; $A_{part, L}$) ou le deuxième signal de bolus ($S_{DO, ext, K}$ ; ; $A_{part, K}$), actionne la machine extracorporelle de traitement du sang (50) respectivement en mode de dérivation pour la durée du premier intervalle de temps intermédiaire (L) indiqué dans le jeu de données ou du second intervalle de temps intermédiaire (K), plus court par rapport au premier intervalle de temps intermédiaire (L), et fait fonctionner le circuit sanguin extracorporel (20), de préférence simultanément, au débit sanguin indiqué ($Q_B$) et, immédiatement après la fin respective du mode de dérivation pour le premier ou le deuxième bolus de liquide de dialyse respectif généré par le mode de dérivation, dans le liquide de dialyse utilisé s'écoulant à partir du dialyseur (2), à l'aide de l'au moins un dispositif de détection (31, 32), par une technique de mesure, détecte la modification de signal associée au premier ou au second bolus de liquide de dialyse respectif par rapport au signal de base ($S_{DO, pre}$) en tant que premier signal de bolus ($S_{DO, ext, L}$ ; $A_L$ ; $A_{part, L}$) ou deuxième signal de bolus ($S_{DO, ext, K}$ ; ; $A_{part, K}$) associé,
- de préférence le modèle de calcul enregistré sur l'unité de mémoire (110), à l'aide duquel l'unité de commande et de calcul (100) calcule en option une valeur de recirculation actuelle (R) en tenant compte du premier signal de bolus ($S_{DO, ext, L}$ ; $A_L$ ; $A_{part, L}$), du deuxième signal de bolus ($S_{DO, ext, K}$ ; ; $A_{part, K}$) ainsi que du signal de base ($S_{DO, pre}$), de préférence avant le démarrage ou au début d'un cycle de traitement au moyen de la machine extracorporelle de traitement du sang (50).

11. Machine extracorporelle de traitement du sang (50) selon l'une quelconque des revendications précédentes, **caractérisée en ce que** l'unité de mémoire (32) est intégrée de manière fixe dans la machine extracorporelle de traitement du sang (1).

12. Machine extracorporelle de traitement du sang (50) selon l'une quelconque des revendications précédentes, **caractérisée en ce que** la machine extracorporelle de traitement du sang (1) présente en outre un dispositif d'affichage, sans fil ou filaire, qui est conçu pour afficher la présence déterminée d'une recirculation, en particulier la valeur de recirculation actuelle déterminée (R).

13. Machine extracorporelle de traitement du sang (50) selon l'une quelconque des revendications, **caractérisée en ce que** l'au moins un dispositif de détection (31, 32) et l'unité de commande et de calcul (100) sont prévus dans ou sur un dispositif modulaire (200) séparé, pouvant être équipé ultérieurement et/ou mis à niveau pour la machine extracorporelle de traitement du sang (50), pour la détermination de la recirculation, le dispositif modulaire (200) présentant :

- une unité de détection avec :

 - au moins un dispositif de détection (31, 32),
 - une section ou partie de raccordement pour le raccordement, de préférence adaptatif, de l'unité de capteur au circuit de fluide de la machine extracorporelle de traitement du sang (50), de sorte que le au moins un dispositif de capteur (31, 32) est monté en aval du dialyseur (2) du côté du liquide de dialyse (30) et est conçu pour détecter le au moins un signal ($S_{DO}$) d'une grandeur physique et/ou chimique du liquide de dialyse utilisé dans son évolution dans le temps ; et

- une unité électronique et d'évaluation réalisée en tant qu'unité de commande et de calcul (100), fonctionnant indépendamment de la machine extracorporelle de traitement du sang (50), qui détermine la présence de la recirculation, en particulier qui détermine la valeur de recirculation actuelle (R) ; en outre, facultativement :
- une interface d'entrée-sortie, de préférence combinée, qui est réalisée pour délivrer à un utilisateur la recirculation déterminée par l'unité électronique et d'évaluation, en particulier la valeur de recirculation actuelle déterminée (R), et/ou

- une alimentation en énergie réalisée indépendamment de la machine extracorporelle de traitement du sang (50).

14. Dispositif modulaire (200) pour la détermination d'une recirculation pour l'équipement ultérieur d'une machine extracorporelle de traitement du sang (50) avec un dialyseur (2), présentant :

- une unité de commande et de calcul (100) qui est réalisée en tant qu'unité électronique et d'évaluation fonctionnant indépendamment de la machine extracorporelle de traitement du sang (50) ; et
- une unité de détection reliée électriquement à l'unité de commande et de calcul (100) :

    - au moins un dispositif de détection (31, 32),
    - une section ou partie de raccordement pour le raccordement de préférence adaptatif de l'unité de capteur au circuit de fluide de la machine extracorporelle de traitement du sang (50), de sorte que l'au moins un dispositif de capteur (31, 32) est monté en aval du dialyseur (2) sur un côté de liquide de dialyse (30) et est conçu pour mesurer au moins un signal ($S_{DO}$) d'une grandeur physique et/ou chimique du liquide de dialyse utilisé pour la détermination quantitative d'au moins une substance constitutive du sang de préférence choisie ou pouvant être sélectionnée dans le liquide de dialyse utilisé ;

- facultativement, une interface d'entrée/sortie vers un utilisateur, et/ou
- facultativement, une alimentation en énergie réalisée indépendamment de la machine extracorporelle de traitement du sang (50) ; et/ou
- facultativement une soupape d'arrêt de dérivation (11) dans la conduite de dérivation (22) pour la liaison fluidique d'une source de liquide de dialyse (3) avec un puits de liquide de dialyse (7) et au moins la soupape d'arrêt (9) montée en amont du dialyseur (2) dans la conduite d'amenée de liquide de dialyse (14), en particulier les deux soupapes d'arrêt (9, 10) avant et après le dialyseur (2), pour la commutation dans un fonctionnement intermédiaire du circuit de fluide ;

dans lequel l'unité de commande et de calcul (100) est prévue et réalisée pour déterminer une présence d'une recirculation sans administration de bolus côté sang :

    - détecter l'au moins un signal ($S_{DO}$) mesuré par l'au moins un dispositif de détection (31, 32) dans son évolution dans le temps ;
    - faire fonctionner le traitement extracorporel du sang dans un mode de base (M-0) pour un fonctionnement continu, dans lequel l'écoulement de liquide de dialyse à travers le dialyseur (2) est autorisé,
    - mesurer le signal associé au liquide de dialyse utilisé dans le mode de base (M-0) comme un signal de base ($S_{DO, \, pre, \, L}$, $S_{DO, \, pre, \, K}$) ;
    - le traitement extracorporel du sang du mode de base (M-0) à un mode intermédiaire (M-I) pour un fonctionnement intermédiaire dans lequel une quantité de liquide de dialyse est enfermé sur le côté membrane de liquide de dialyse (8D) dans le dialyseur (2), tandis que le débit sanguin dans le circuit extracorporel de sang fonctionne à un débit sanguin fixe, dans lequel le sang continue à s'écouler sur le côté membrane de sang (8B), pendant la durée respective d'un intervalle de temps intermédiaire (L, K) prédéterminé et/ou pouvant être prédéterminé, en particulier pour passer à un mode de dérivation autre que le mode intermédiaire (M-I) ; puis
    - passer au mode de base (M-0) pour fournir la quantité de liquide de dialyse précédemment bloquée comme un bolus de liquide de dialyse respectif à l'au moins un dispositif de détection (31, 32) pour mesurer une variation de signal associée au bolus de liquide de dialyse par rapport au signal de base ($S_{DO, \, pre, \, L}$, $S_{DO, \, pre, \, K}$) comme un signal de bolus associé ($S_{DO, \, ext, \, L}$, $S_{DO, \, ext, \, K}$; A; $A_{part}$) ;
    - commuter le mode intermédiaire (M-I) pour un premier intervalle de temps intermédiaire (L) pour l'ajustement de l'équilibre du transport de substance, dans laquelle une première quantité de liquide de dialyse est enfermée dans le dialyseur (2) au moins jusqu'à ce qu'un équilibre de concentration de substance constitutive du sang qui ne se modifie plus ou seulement de manière insignifiante s'établisse sur le côté membrane de liquide de dialyse (8D) et le côté membrane de sang (8B) du dialyseur (2),

    **caractérisé en ce que**
    l'unité de commande et de calcul (100) est en outre prévue et réalisée pour réaliser un cycle de mesure à cet effet :

        - le mode intermédiaire (M-I) pour un second intervalle de temps intermédiaire (K) plus court que le premier intervalle de temps intermédiaire (L), dans laquelle une seconde quantité de liquide de dialyse n'est enfermée dans le dialyseur (2) que tant que l'équilibre de concentration de la substance constitutive du sang ne s'est pas encore établi,

- déterminer la présence de la recirculation à partir d'un écart entre un deuxième signal de bolus ($S_{DO, ext, K}$ ; $A_K$ ; $A_{part, K}$) à la suite du second intervalle de temps intermédiaire (K) et un premier signal de bolus ($S_{DO, ext, L}$ ; $A_L$ ; $A_{part, L}$) à la suite du premier intervalle de temps intermédiaire (L).

15. Unité de mémoire (110) sur laquelle est enregistré, de manière lisible par une machine, un procédé de surveillance d'une recirculation lors d'un traitement extracorporel du sang en utilisant une machine extracorporelle de traitement du sang (50) selon l'une quelconque des revendications 1 à 13 précédentes et/ou en utilisant un dispositif modulaire (200) pour déterminer la recirculation selon la revendication 14 qui précède directement, avec les étapes de surveillance suivantes :

- identification facultative du dialyseur (2) ;
- prédétermination du premier intervalle de temps intermédiaire (L), en particulier en fonction d'un débit sanguin réglé ($Q_B$) ou de manière globalisée, à l'intérieur duquel s'établit un équilibre de concentration d'une substance constitutive du sang de préférence sélectionnée au préalable, de préférence l'urée ;
- prédétermination du second intervalle de temps intermédiaire (K), plus court que le premier intervalle de temps intermédiaire (L), à l'intérieur duquel l'équilibre de concentration de la substance constitutive du sang ne s'établit pas encore ;
- détermination d'un premier signal de bolus ($S_{DO, ext, L}$ ; $A_L$ ; $A_{part, L}$) à la suite du premier intervalle de temps intermédiaire (L) avec les sous-étapes suivantes :

- actionnement de la machine extracorporelle de traitement du sang dans le mode de base (M-0) en mesurant le signal associé au liquide de dialyse utilisé en tant que signal de base ($S_{DO, pre}$) ;
- passage du mode de base (M-0) au mode intermédiaire (M-I), en particulier au mode de dérivation, pendant la durée du premier intervalle de temps intermédiaire (L) ; puis
- retour au mode de base (M-0) pour fournir la quantité de liquide de dialyse précédemment enfermée en tant que premier bolus de liquide de dialyse audit au moins un dispositif de détection (31, 32), et ensuite mesure du premier signal de bolus ($S_{DO, ext, L}$ ; $A_L$ ; $A_{part, L}$) en tant que changement de signal associé au premier bolus de liquide de dialyse par rapport au signal de base ($S_{DO, pre}$);

- détection d'un deuxième signal de bolus ($S_{DO, ext, K}$; $A_K$; $A_{part, K}$) à la suite du second intervalle de temps intermédiaire (K), avant ou après la détection du premier signal de bolus ($S_{DO, ext, L}$; $A_L$; $A_{part, L}$), avec les sous-étapes suivantes :

- actionnement de la machine extracorporelle de traitement du sang dans le mode de base (M-0) ;
- mesure du signal associé au liquide de dialyse utilisé dans le mode de base (M-0) en tant que signal de base ($S_{DO, pre}$) ;
- passage du mode de base (M-0) au mode intermédiaire (M-I), en particulier au mode de dérivation, pendant la durée du second intervalle de temps intermédiaire (K) ; puis
- retour au mode de base (M-0) pour fournir la quantité de liquide de dialyse précédemment enfermée en tant que second bolus de liquide de dialyse audit au moins un dispositif de détection (31, 32), et ensuite mesure du deuxième signal de bolus ($S_{DO, ext, K}$ ; $A_K$ ; $A_{part, K}$) en tant que changement de signal associé au second bolus de liquide de dialyse par rapport au signal de base ($S_{DO, pre}$) ; et

- détermination de la présence de la recirculation, en particulier calcul de la valeur de recirculation actuelle (R) à l'aide du modèle de calcul, à partir d'un écart, en particulier en fonction d'un quotient, du deuxième signal de bolus ($S_{DO, ext, K}$ ; $A_K$ ; $A_{part, K}$) par rapport au premier signal de bolus ($S_{DO, ext, L}$ ; $A_L$ ; $A_{part, L}$) ;
- affichage facultatif de la recirculation ou de la valeur de recirculation actuelle (R).

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19702441 C1
- EP 2783715 A1
- US 5588959 A
- WO 9608305 A1
- DE 102013103221 A1
- WO 2020212332 A1
- EP 3431118 A1